# EUROPEAN PATENT APPLICATION

(11) **EP 4 759 824 A1**
(43) Date of publication of application: **17.06.2026**
(21) Application number: 24866619.0
(22) Date of filing: 22.03.2024
(51) Int. Cl.: C07K 7/06, C07C 311/42, C07C 303/40, A61P 35/00, A61K 31/198, C07K 1/13

(54) **DANSYLAMIDE-MODIFIED COMPOUNDS TARGETING PSMA, PREPARATION METHOD THEREFOR, AND USE THEREOF**

(30) Priority: 20.09.2023 CN 202311212336
(71) Applicant: Yantai Lannacheng Biotechnology Co., Ltd., Yantai, Shandong 264117 (CN)
(72) Inventor: CHEN, Xiaoyuan, Yantai, Shandong 264117 (CN); YANG, Hongzhang, Yantai, Shandong 264117 (CN); ZHOU, Zijian, Yantai, Shandong 264117 (CN); SHU, Linlin, Yantai, Shandong 264117 (CN); WEN, Xuejun, Yantai, Shandong 264117 (CN); WU, Xiaoming, Yantai, Shandong 264117 (CN); ZHANG, Jingjing, Yantai, Shandong 264117 (CN)
(74) Representative: Wang, Bo
(86) International application number: PCT/CN2024/083379
(87) International publication number: WO 2025/060375

(57) **Abstract**

The present invention provides a dansylamide-modified PSMA-targeting compound having a structure thereof as represented by formula A or formula I, where R₁ is of an amino acid analog structure; R₂ is of a prostate-specific membrane antigen-targeting compound structure; R₃ is a nuclide chelating group; and L is -(X)ₙ-(CH₂)ₘ-(Y)_{q}-, where X and Y are independently selected from lysine, glutamic acid, or a derivative structure containing lysine or glutamic acid, n is an integer from 0 to 10, m is an integer from 0 to 20, and q is an integer from 0 to 6, and where each CH₂ may be individually replaced with -OCH₂-, -NH(CO)- or -(CO)-NH-. The present invention further provides a radionuclide-labeled compound based on the structure of the compound. The compound and the radionuclide-labeled compound exhibit relatively high tumor uptake and retention time, making them suitable for use in nuclide therapy and imaging of PSMA-highly expressing tumors.

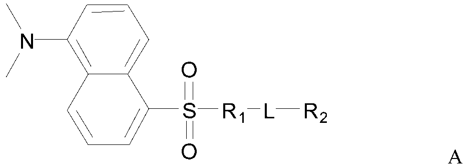

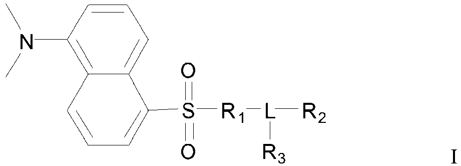

## Description

### TECHNICAL FIELD

The present invention belongs to the technical field of diagnosis and treatment of prostate cancer, including but not limited thereto, and specifically relates to a prostate-specific membrane antigen (PSMA)-targeting compound with high tumor uptake and long retention, and a radionuclide-labeled disease diagnosis and treatment probe thereof.

### BACKGROUND

Prostate-specific membrane antigen (PSMA) is only minimally expressed in normal tissues but is overexpressed in approximately 90% of prostate cancer lesions (100 to 1,000 times higher than reference values). Furthermore, the expression level of PSMA in lesions is closely related to tumor aggressiveness and malignancy. Therefore, accurately evaluating the expression of PSMA in lesions is of great significance for the diagnosis, staging, therapeutic efficacy evaluation, progression, and prognosis assessment of prostate cancer. Currently, PSMA imaging has become an indispensable imaging examination means for patients with prostate cancer. In addition, PSMA can also be highly expressed in other tumors, such as lung cancer, liver cancer, gastric cancer, breast cancer, colorectal cancer, renal cell carcinoma, bladder cancer, brain tumors, thyroid cancer, adenoid cystic carcinoma, and the like, exhibiting good application potential in the diagnosis and treatment of tumors beyond prostate cancer.

Radioligand therapy (RLT) targeting PSMA is one of the important treatment modalities for metastatic castration-resistant prostate cancer (mCRPC), and ¹⁷⁷Lu-PSMA-617 is the most commonly used nuclide therapy drug. In September 2021, Novartis announced the results of the phase III VISION clinical trial of ¹⁷⁷Lu-PSMA-617 in the treatment of patients with PSMA-positive mCRPC. The study showed that, compared with the best standards of care (SOC), ¹⁷⁷Lu-PSMA-617 combined with SOC significantly improved the median radiographic progression-free survival (8.7 months vs. 3.4 months) and median overall survival (15.3 months vs. 11.3 months) in mCRPC patients. On March 23, 2022, Pluvicto (lutetium Lu-177 vipivotide tetraxetan, ¹⁷⁷Lu-PSMA-617) was approved by the FDA for the treatment of adult patients with PSMA-positive mCRPC who have previously received androgen receptor inhibitors and taxane chemotherapy. However, it is noteworthy that although ¹⁷⁷Lu-PSMA-617 has made certain progress in the treatment of mCRPC patients, a considerable proportion of prostate cancer patients still exhibit poor therapeutic effects. Furthermore, in terms of treatment, small-molecule peptides such as PSMA-617 undergo rapid metabolism and clearance, resulting in a relatively low effective dose and excessively short retention time at the tumor site. Consequently, high doses or more frequent administration methods are required to meet therapeutic needs, which increases the likelihood of adverse reactions and imposes a heavy economic and social burden. Therefore, optimizing the pharmacokinetics of molecular probes, improving the utilization rate of radionuclides such as Lu-177, and reducing the dose of radionuclides used is one of the important directions for the development of nuclide theranostics. To this end, this study proposes to use a dansylamide compound as the core, which significantly enhances the uptake and retention of PSMA probes at the tumor site. This holds significant importance for the clinical application of PSMA-based theranostics.

### SUMMARY

Based on the above background, a primary objective of the present invention is to develop a novel prostate-specific membrane antigen-targeting compound with dansylamide as the core, which enhances tumor uptake and prolongs tumor retention. The present invention can overcome the defects of excessively rapid metabolism and excessively short retention time in target organs associated with small-molecule PSMA-targeting probes, which improves the efficacy of PSMA-targeting nuclide therapy and imaging, and holds potential for clinical application and promotion.

Another objective of the present invention is to provide a series of radiolabeled prostate-specific membrane antigen-targeting complexes with high tumor uptake and long retention.

Another objective of the present invention is to provide a method for preparing the radiolabeled prostate-specific membrane antigen-targeting complexes with high tumor uptake and long retention.

A further objective of the present invention is to provide use of the complexes in prostate-cancer-targeting radionuclide imaging and therapy.

Technical solutions for achieving the above primary objective of the present invention include the following two aspects: ligand synthesis and radiolabeling.

In a first aspect, the present invention provides a dansylamide-modified PSMA-targeting compound with high tumor uptake and long retention, having a molecular structure thereof formed by linking a dansylamide compound and a PSMA-targeting compound via a linking group L, and as represented by formula A below: 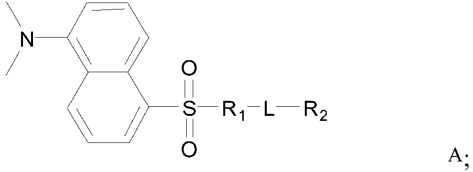 having a molecular structure thereof formed by linking a dansylamide compound, a PSMA-targeting compound, and a nuclide chelating group via a linking group L, and as represented by formula I below: 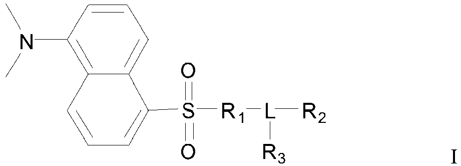 where:
L is -(X)ₙ-(CH₂)ₘ-(Y)_{q}-, where n is an integer from 0 to 10 (preferably an integer from 0-5), X and Y are independently selected from lysine, glutamic acid, or a derivative structure containing lysine or glutamic acid, m is an integer from 0 to 20 (preferably an integer from 0-10), and q is an integer from 0 to 6 (preferably an integer from 0-3), and where each CH₂ may be individually replaced with -OCH₂-, -NH(CO)-, or -(CO)-NH-
R₁ is of an amino acid analog structure, including an L-amino acid, a D-amino acid, and a derivative thereof 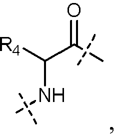 where R₄ includes but is not limited to: (CH₂)ᵣ-aryl, (CH₂)ᵣ-heteroaryl, (CH₂)ᵣ-COOH, (CH₂)ᵣ-NH₂, (CH₂)ᵣ-OH, (CH₂)ᵣ-SH, (CH₂)ᵣ-naphthylmethyl, (CH₂)ᵣ-indolyl, and (CH₂)ᵣ-benzothiazolyl, where r is an integer from 0 to 10 (for example, may be 0, 1, 2, 3, 4, 5, 6, 7, 8, 9, or 10). In a preferred embodiment, R₁ may specifically be of the following structure: 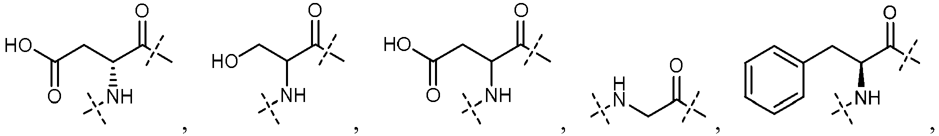 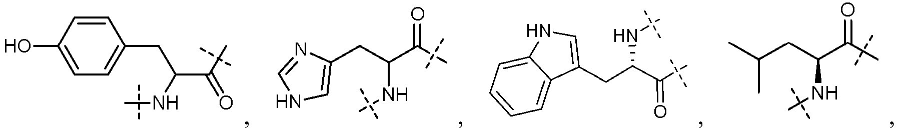 or 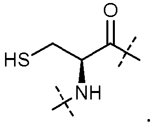
R₂ is derived from a prostate-specific membrane antigen-targeting compound, as shown in formula II: 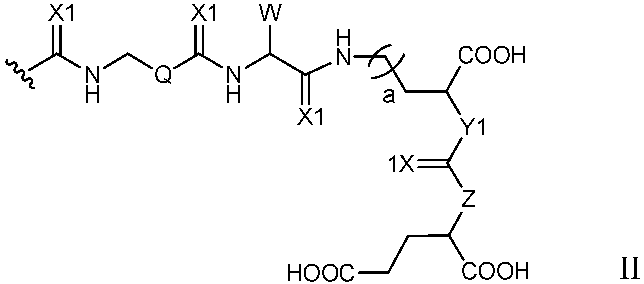 where:
   a is an integer from 0 to 20 (preferably an integer from 0-10)
   X1 structures at different positions are the same or different and are independently selected from O, N, S, or P;
   Y1 is O, S, or NH;
   Z is O, S, or NH;
   Q is selected from substituted or unsubstituted alkyl, or substituted or unsubstituted alkylaryl and cycloalkyl, preferably selected from substituted or unsubstituted C₅-C₁₄ aryl, substituted or unsubstituted C5-C₁₄ alkylaryl, or substituted or unsubstituted C₅-C₁₄ cycloalkyl;
   W is selected from -(CH₂)_{b}-aryl or -(CH₂)_{b}-heteroaryl, where b is an integer from 0-10 (specifically, may be 0, 1, 2, 3, 4, 5, 6, 7, 8, 9, or 10).
   R₂ is derived from a prostate-specific membrane antigen-targeting compound, preferably but not limited to the following structure: 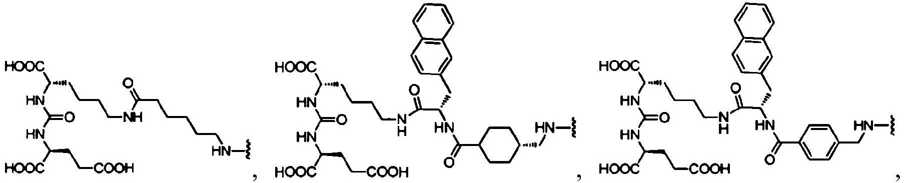 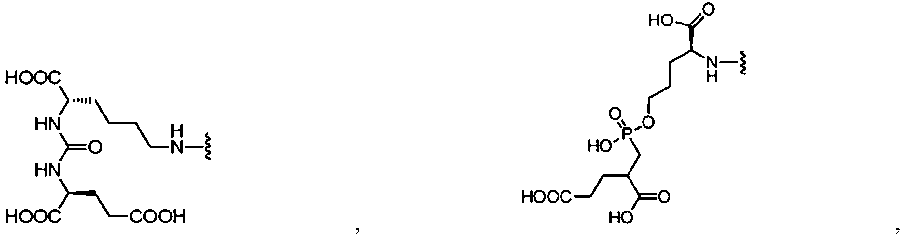 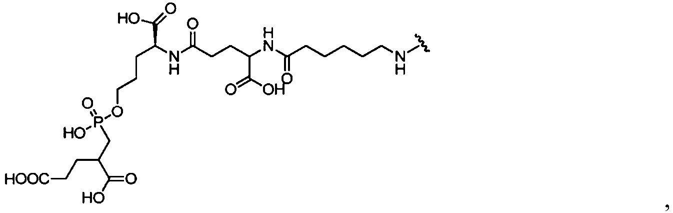 or 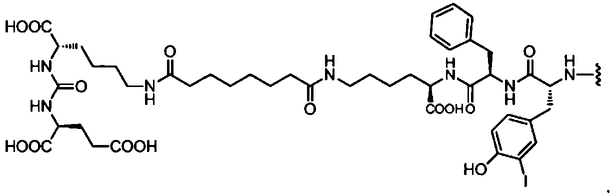
   R₃ is a nuclide chelating group, including but not limited to the following structure or derived from the following structure:

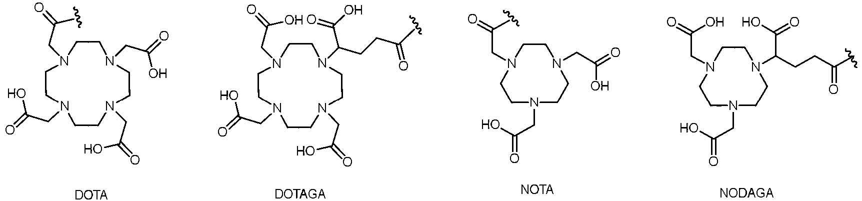

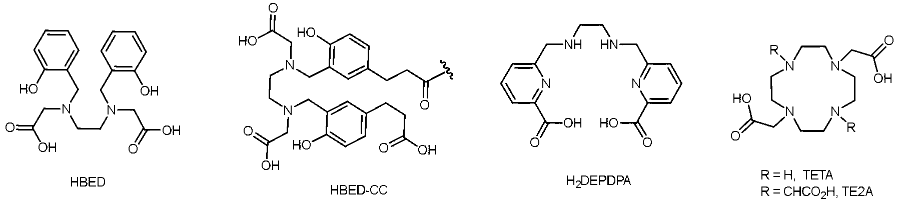

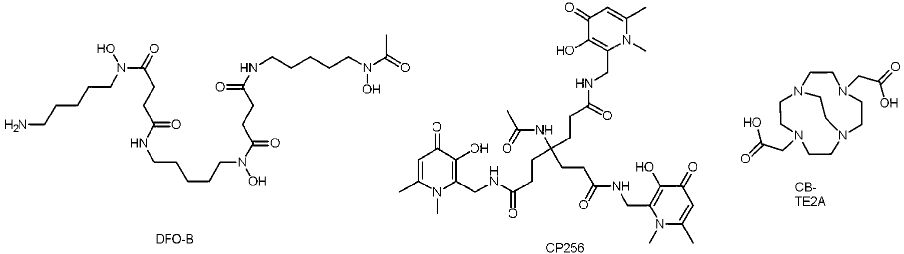

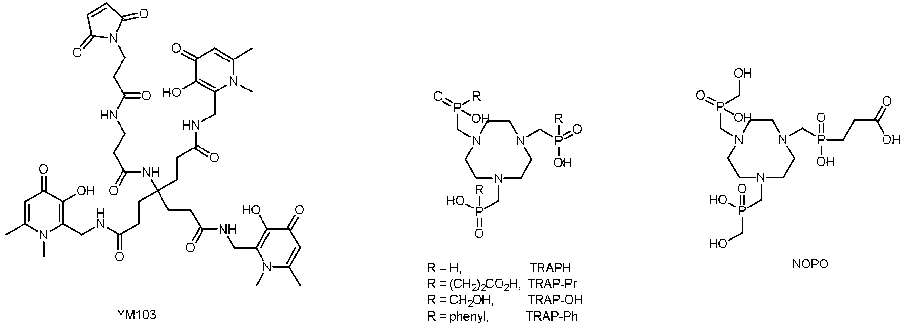

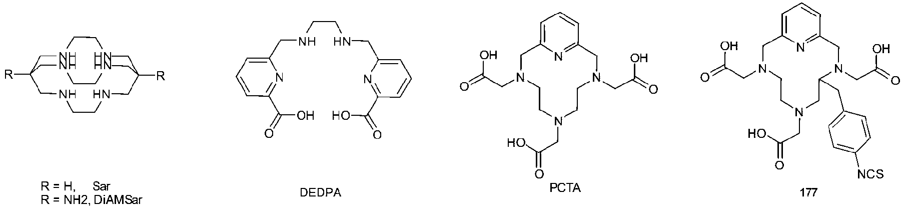

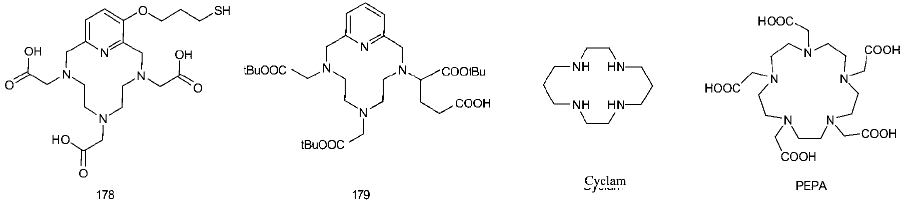

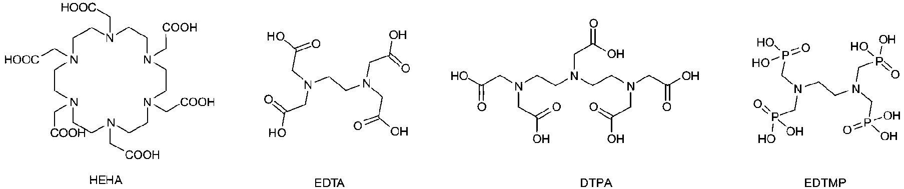

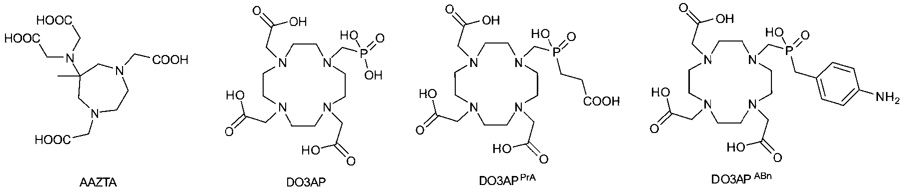

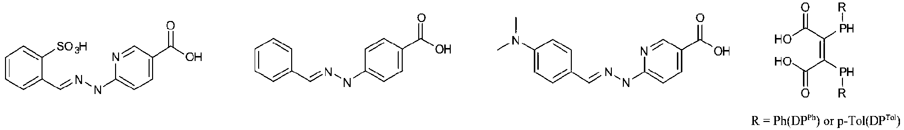

In a preferred embodiment of the present invention, R₂ is 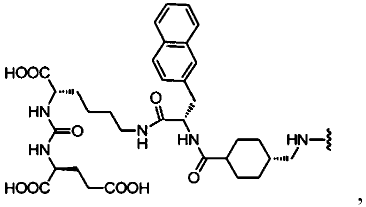 and R₃ is 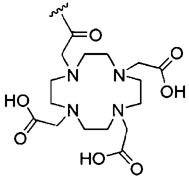 or 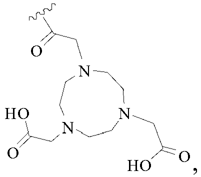
that is, a structure of a compound of formula A is as represented by formula B; and 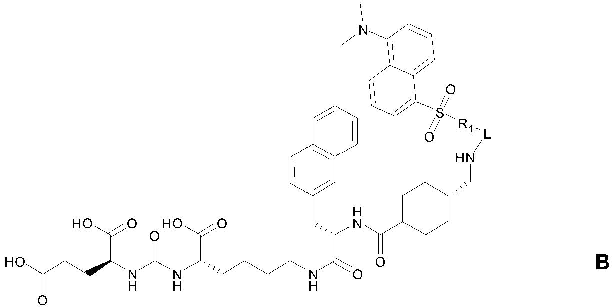
a structure of a compound of formula I is as represented by formula III or formula III-1 below: 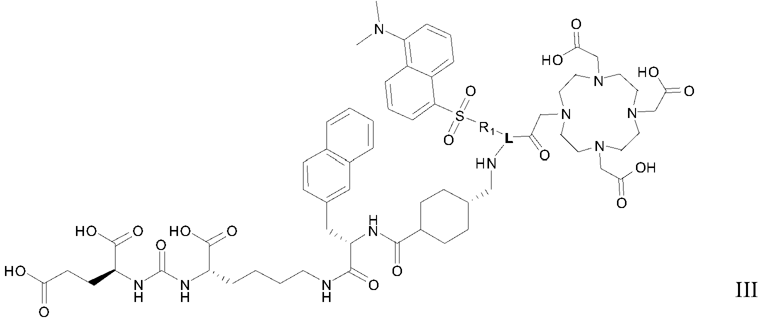 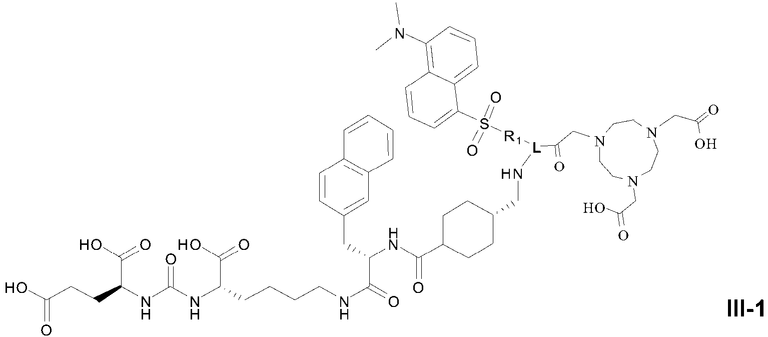
at this time, L is selected from:
   Lys, -Lys-(CO)-CH₂-(CO)-NH-CH₂-(CO), -Lys-(CO)-CH₂-(OCH₂CH₂)-O-CH₂(C O)-NH-CH₂-(CO), -Lys-(CO)-CH₂-(OCH₂CH₂)₃-O-CH₂(CO)-NH-CH₂-(CO), -(CO)-CH ₂-(CO)-Lys-, -(CO)-(OCH₂CH₂)-O-CH₂(CO)-Lys-, or -(CO)-CH₂-(OCH2CH2)₃-O-CH₂(CO)-Lys-.

In a more preferred embodiment of the present invention, R₂ is 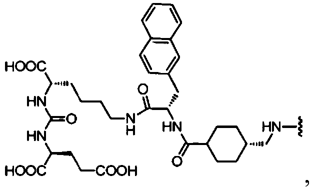 R₃ is 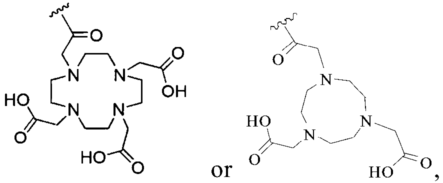 and L is Lys, that is, a structure of a compound of formula A is as represented by formula C: 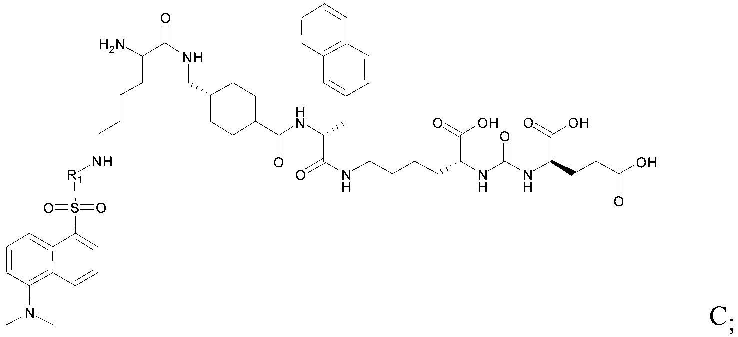
a structure of a compound of formula I is as represented by formula IV or formula IV' below: 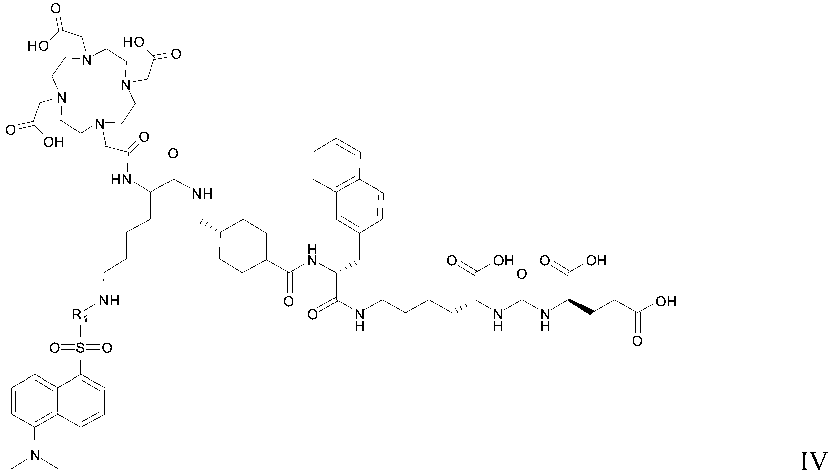 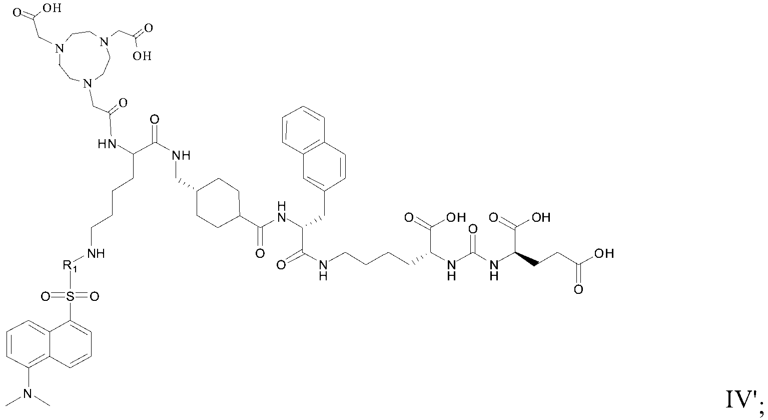
R₁ in formulas B, C, III, III-1, IV, and IV' above is of an amino acid analog structure, including an L-amino acid, a D-amino acid, and a derivative thereof 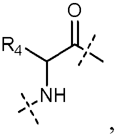 where R₄ includes but is not limited to: (CH₂)ᵣ-aryl, (CH₂)ᵣ-heteroaryl, (CH₂)ᵣ-COOH, (CH₂)ᵣ-NH₂, (CH₂)ᵣ-OH, (CH₂)ᵣ-SH, (CH₂)ᵣ-naphthylmethyl, (CH₂)ᵣ-indolyl, and (CH₂)ᵣ-benzothiazolyl, where r is an integer from 0 to 10 (for example, may be 0, 1, 2, 3, 4, 5, 6, 7, 8, 9, or 10). In a preferred embodiment, R₁ may specifically be of the following structure: 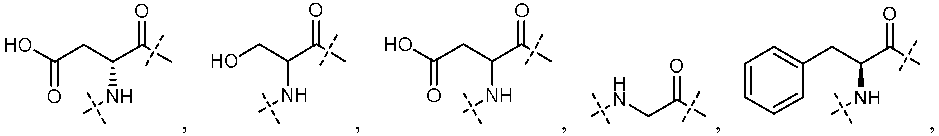 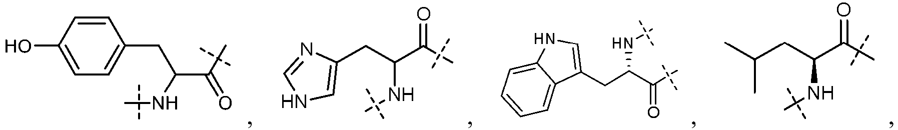 or

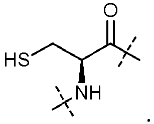

In a further preferred embodiment of the present invention, a structure of a compound of formula A may be any one of those represented by formulas A-1 to A-12 below:

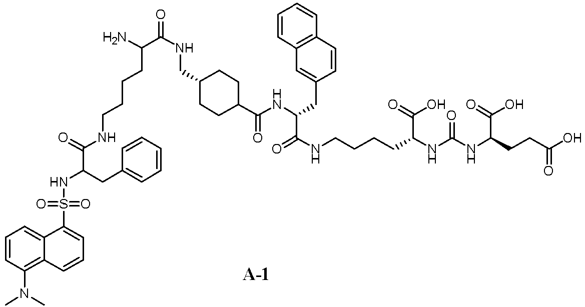

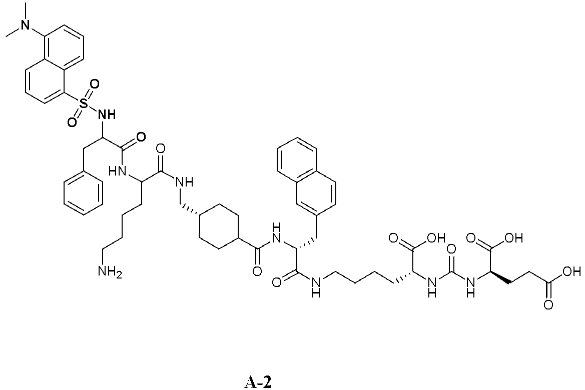

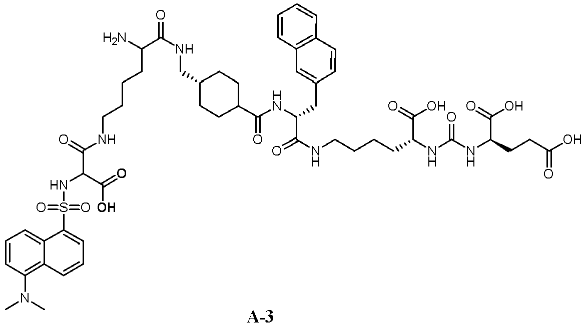

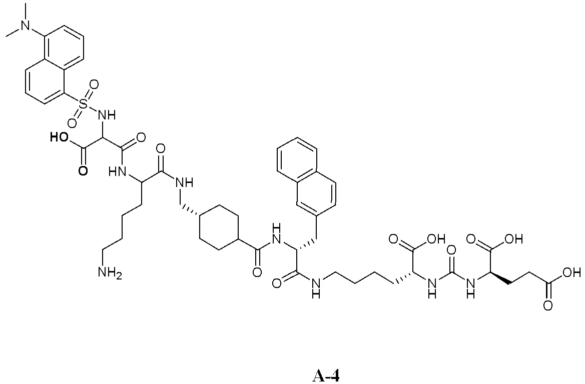

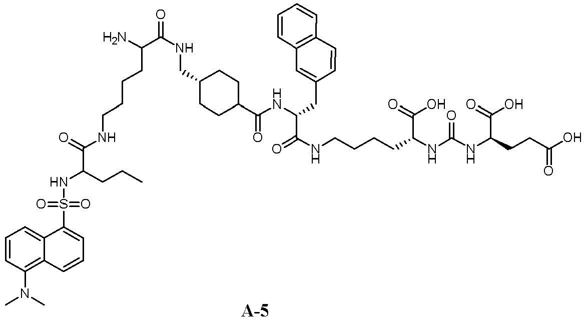

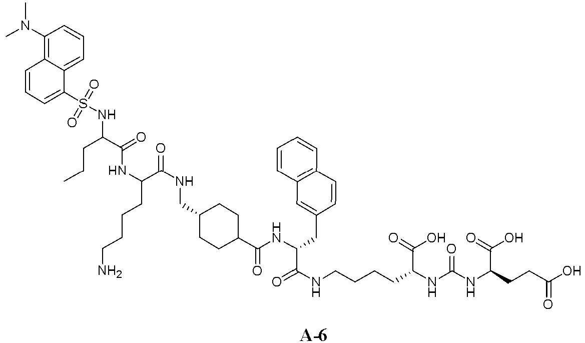

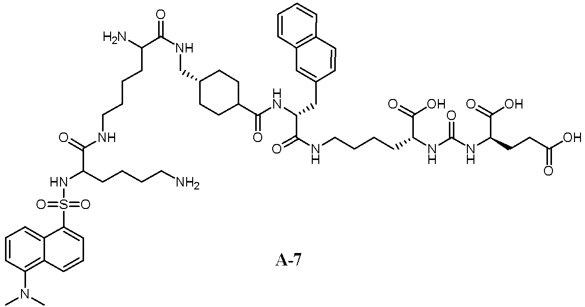

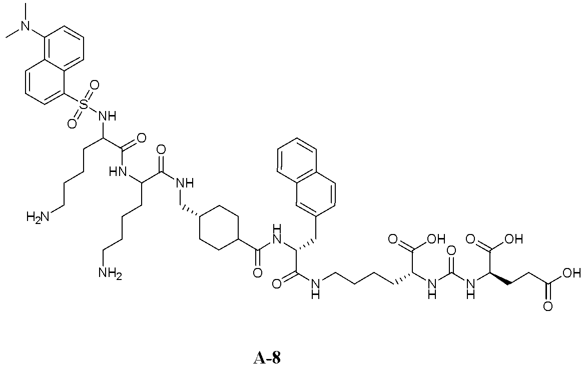

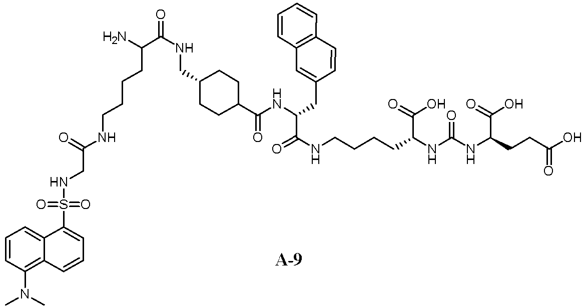

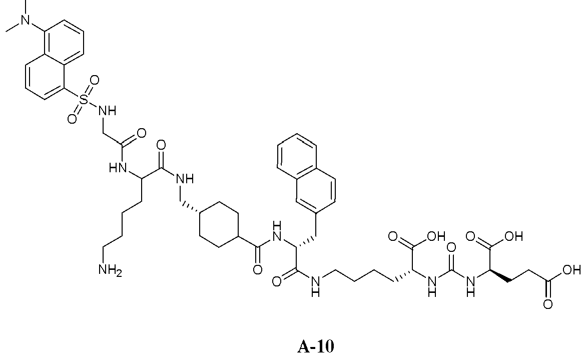

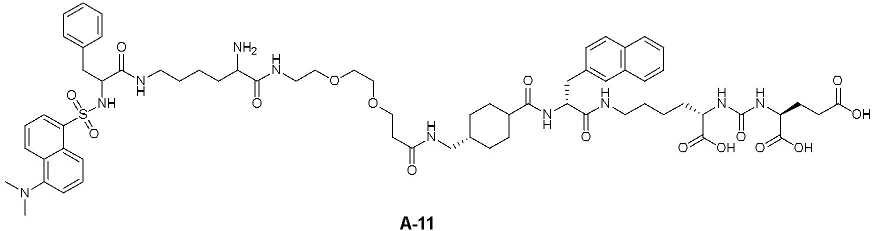

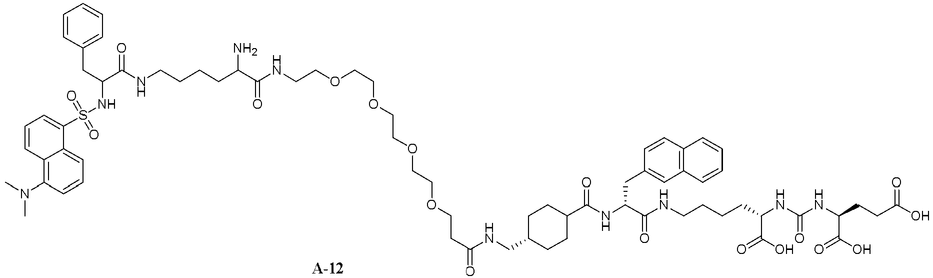

The present invention further provides a compound having any one of structures represented by formulas A'-1 to A'-12:

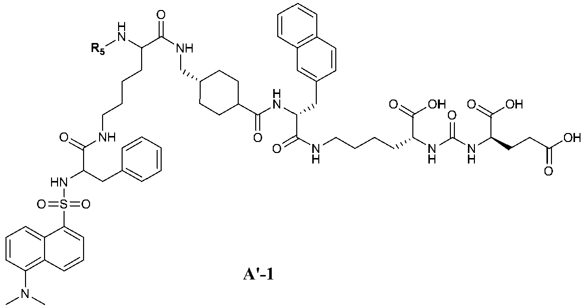

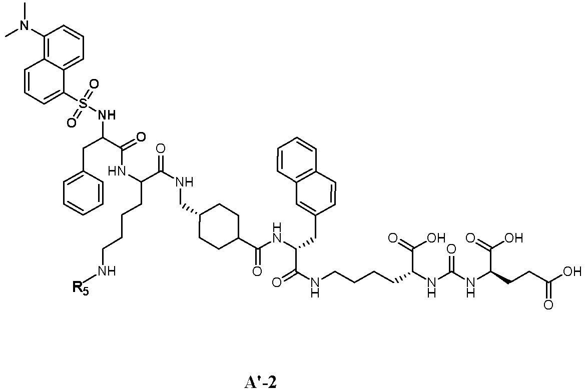

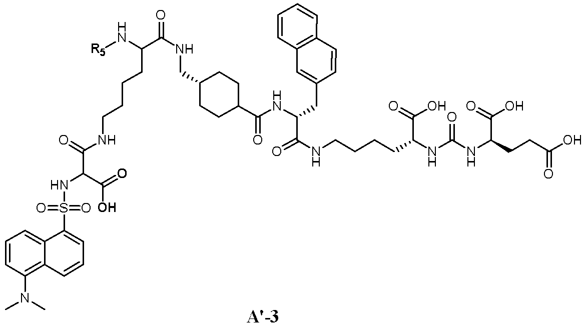

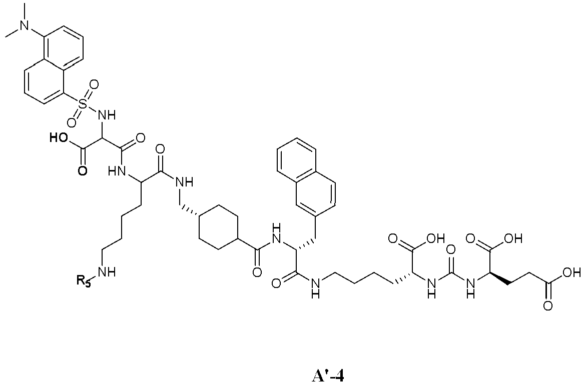

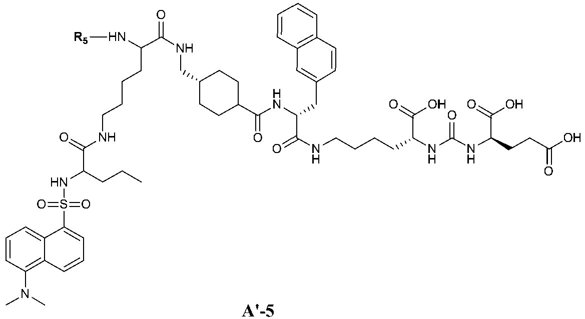

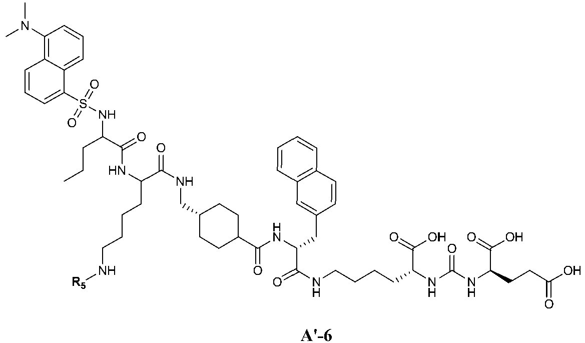

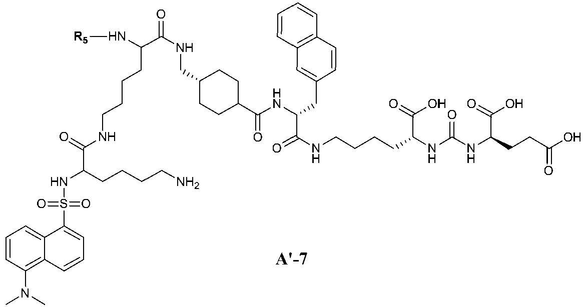

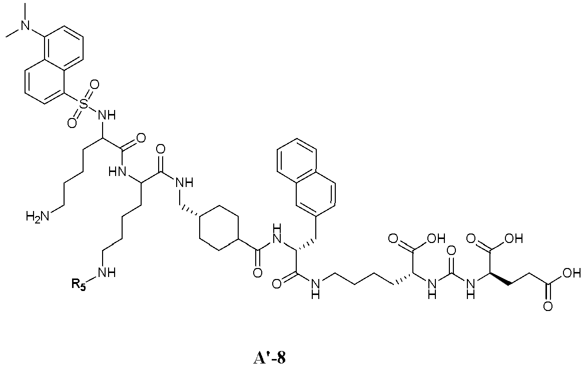

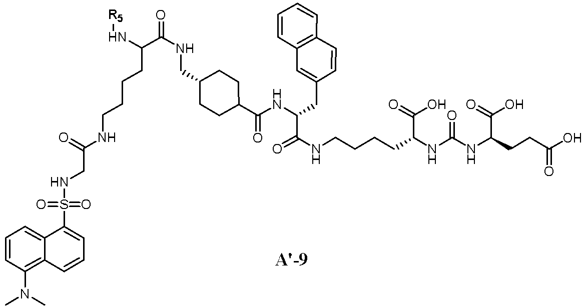

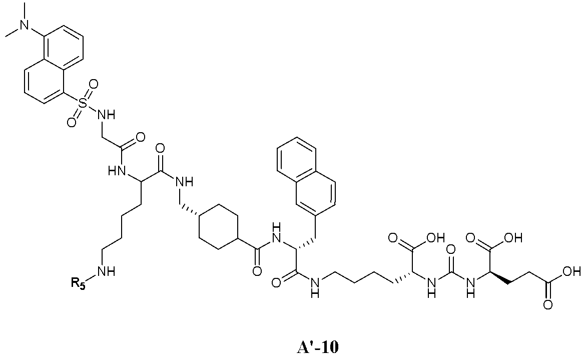

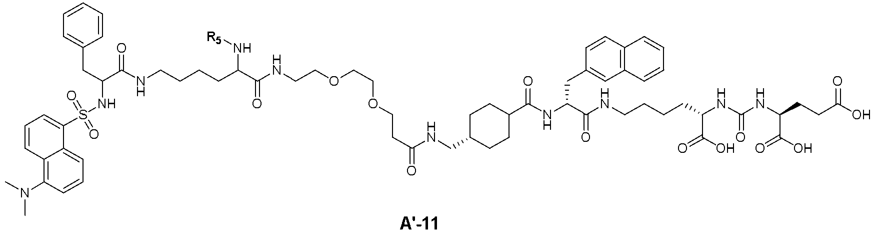

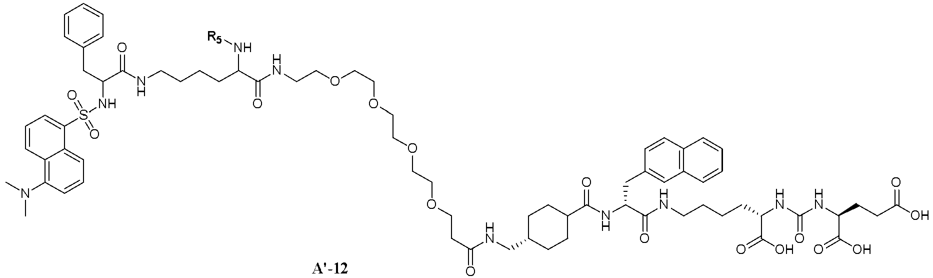

R₅ is a nuclide chelating group selected from any one of the following structures:

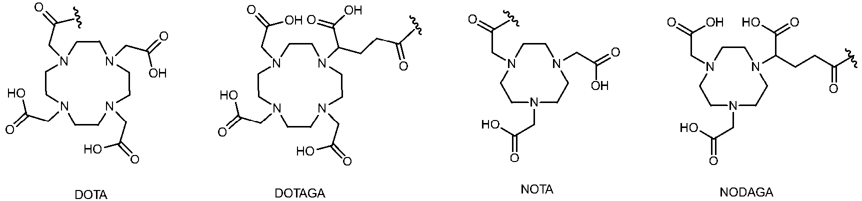

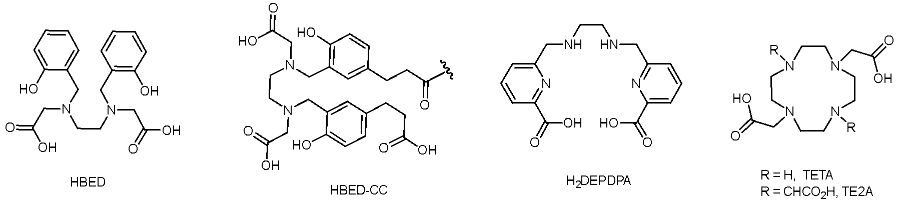

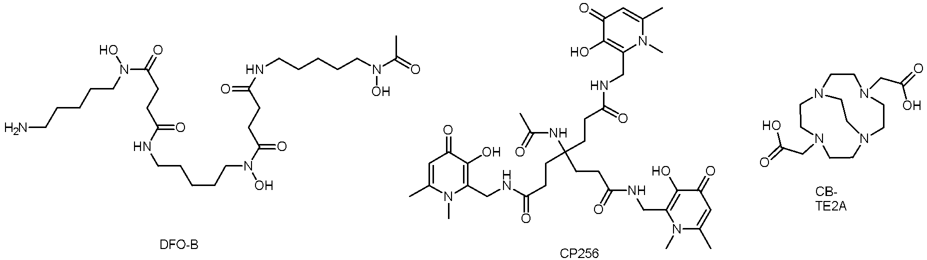

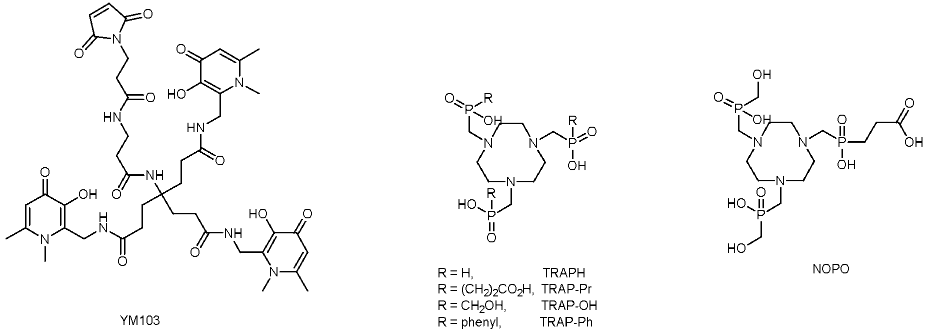

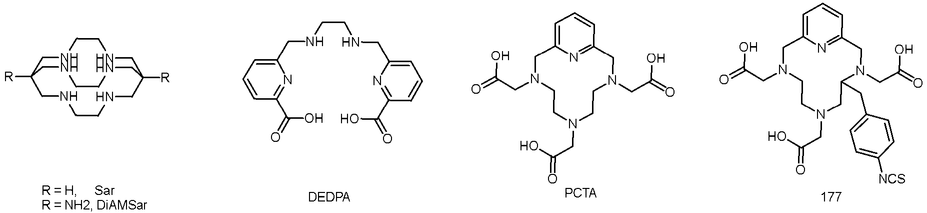

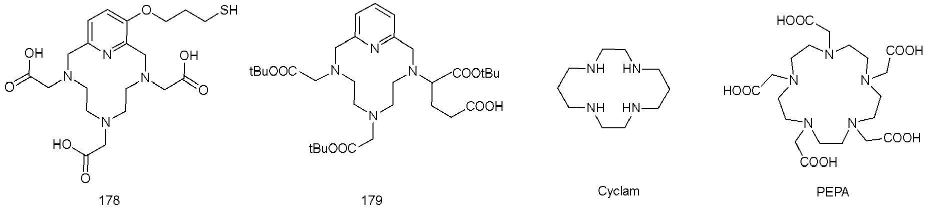

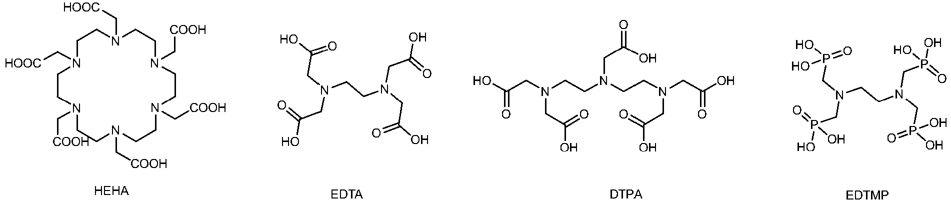

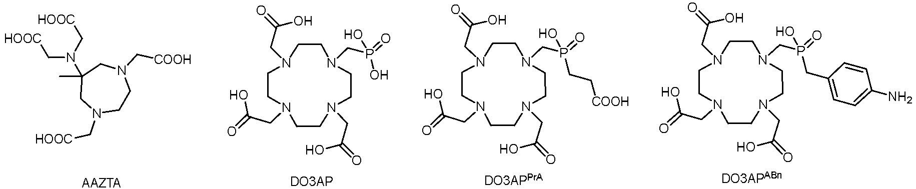

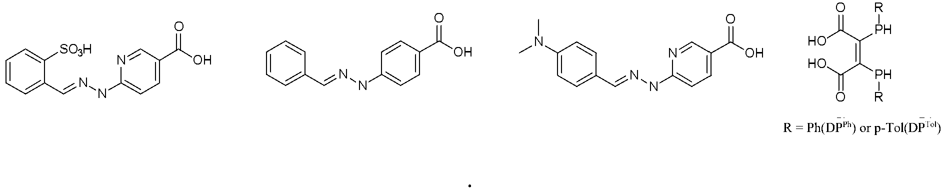

A compound of formula I is any one of those represented by formulas IV-1 to IV-24: 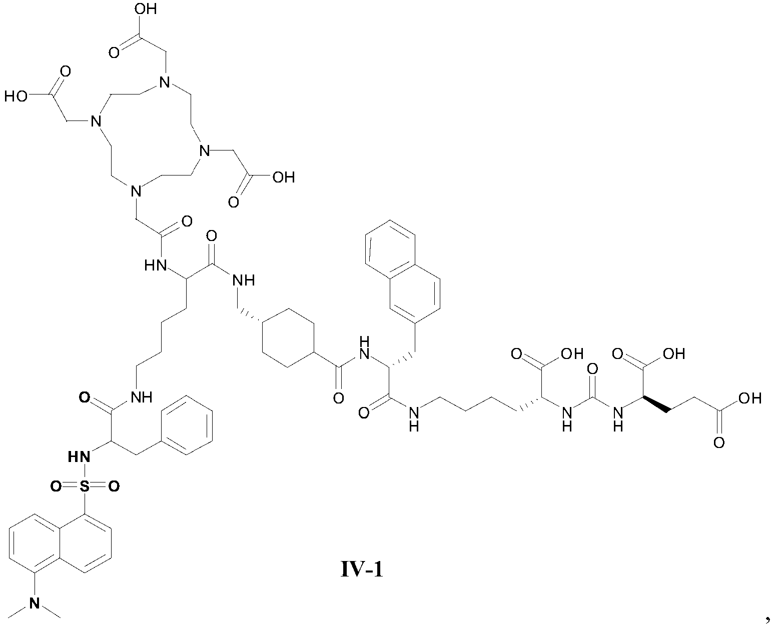 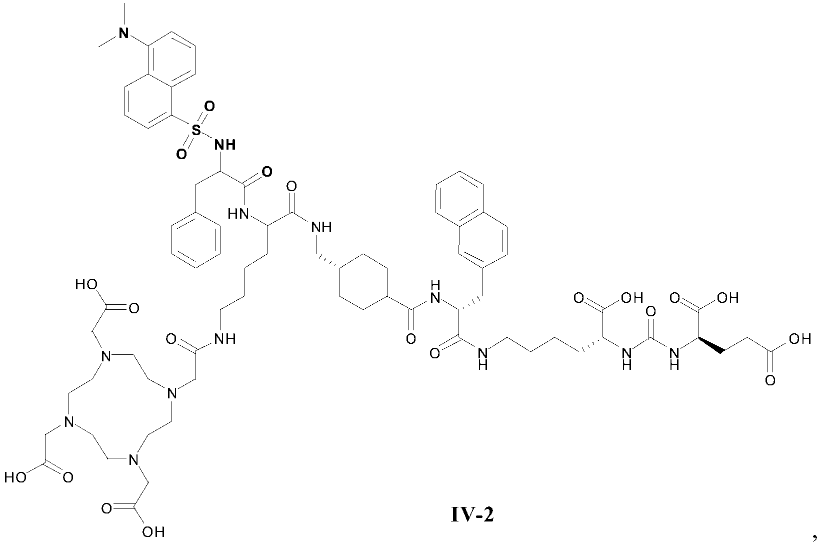 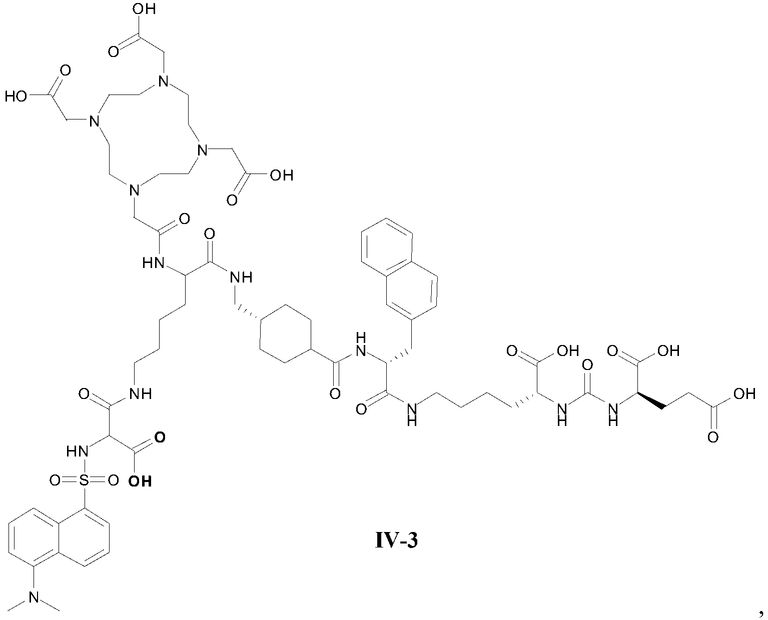 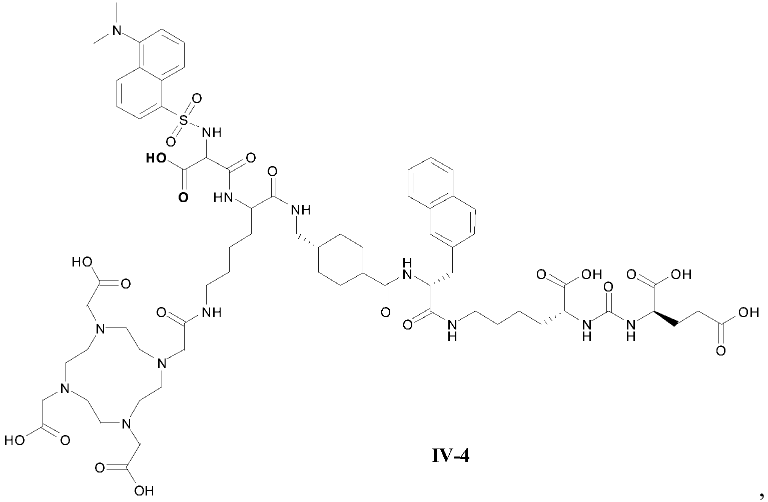 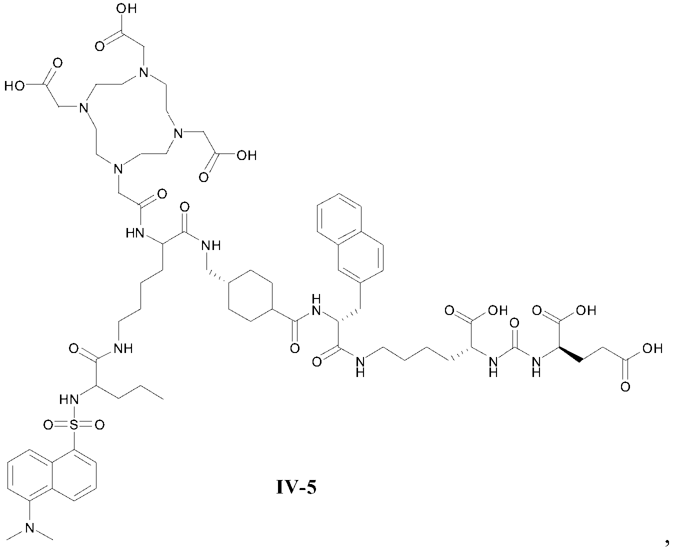 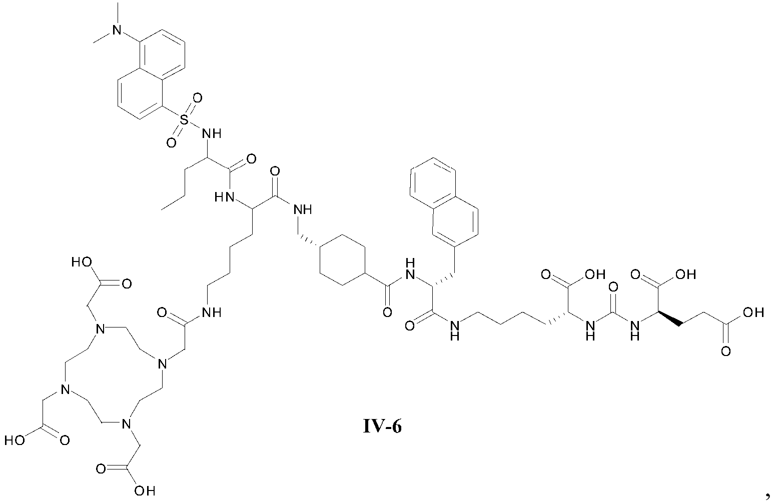 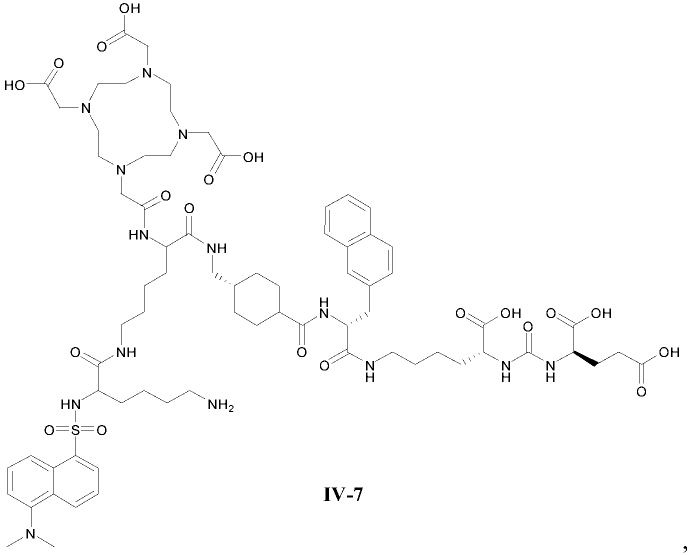 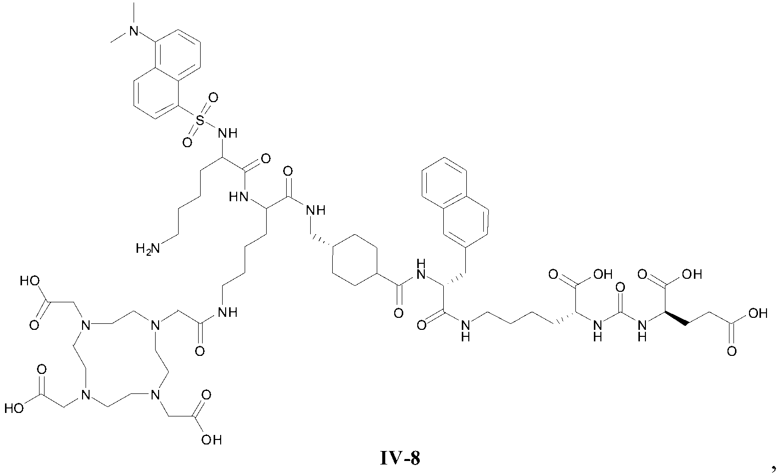 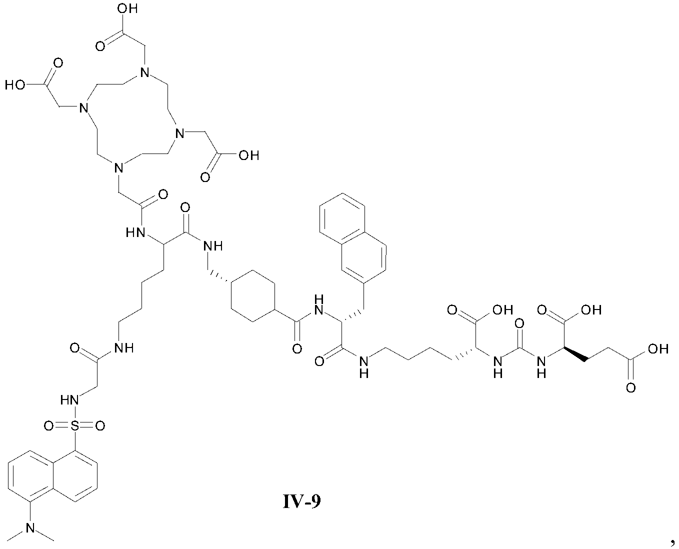 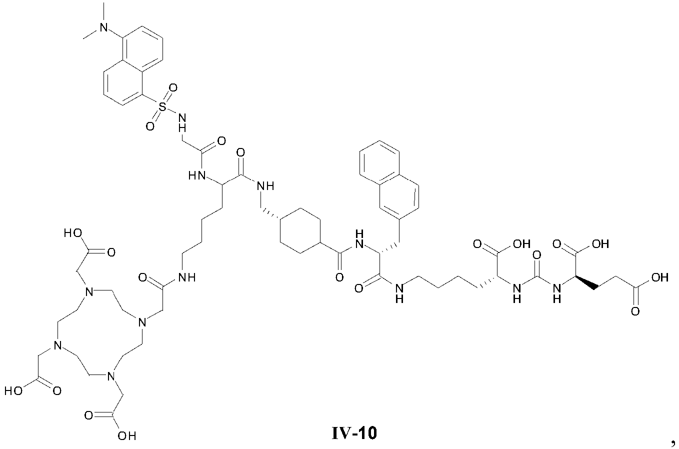 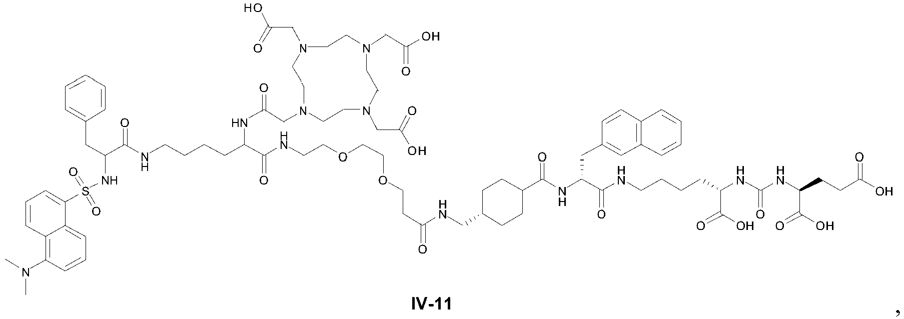 or

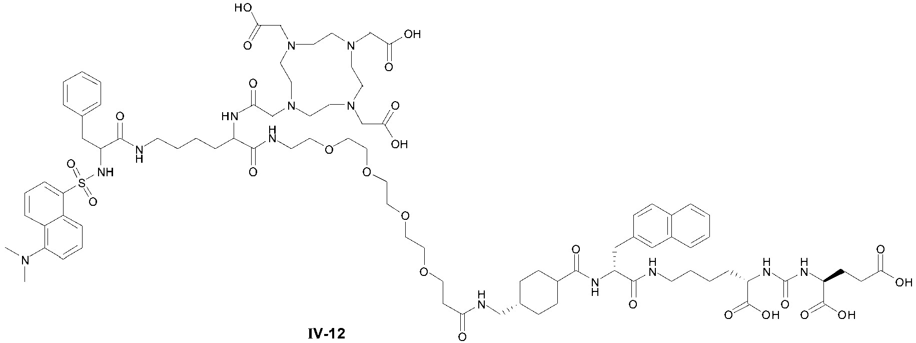

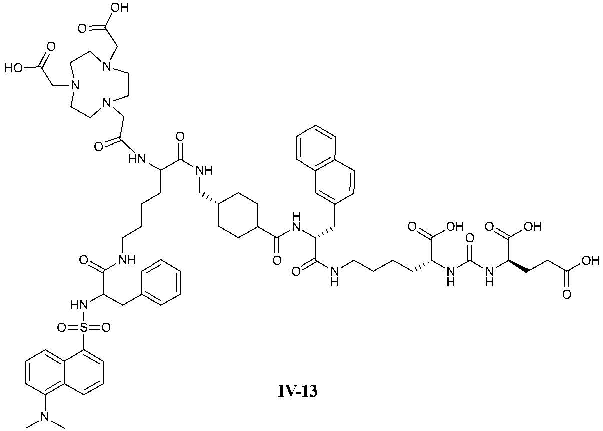

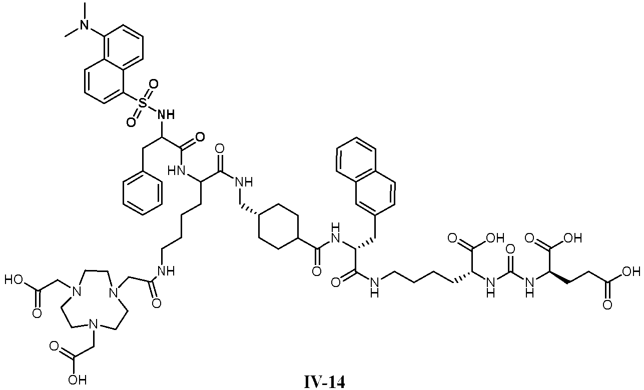

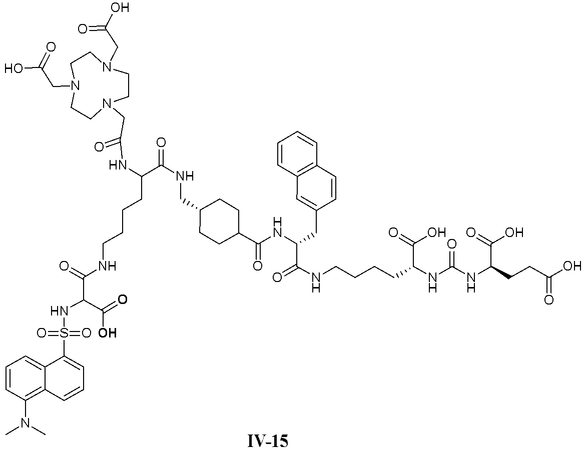

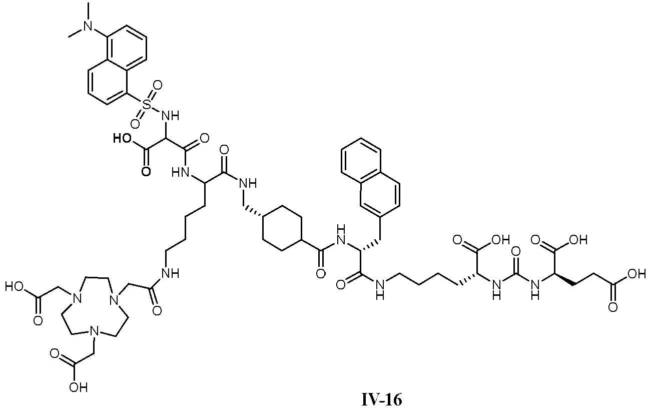

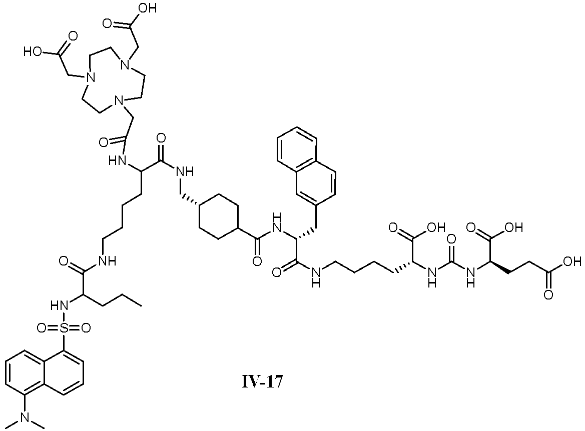

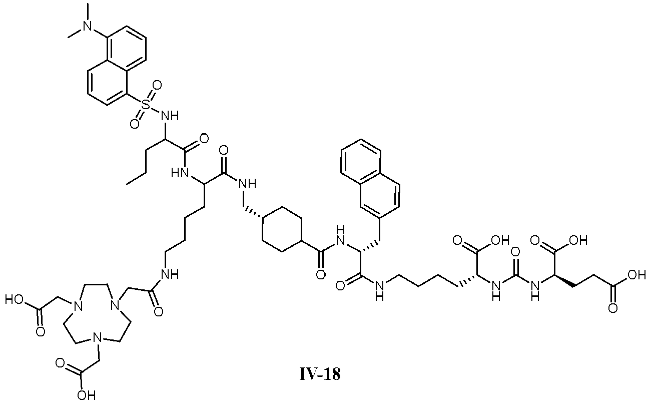

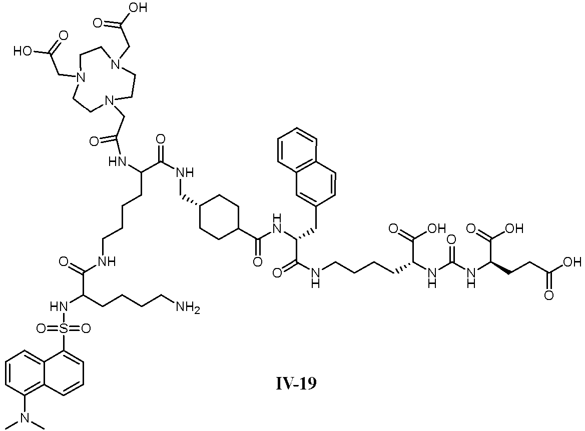

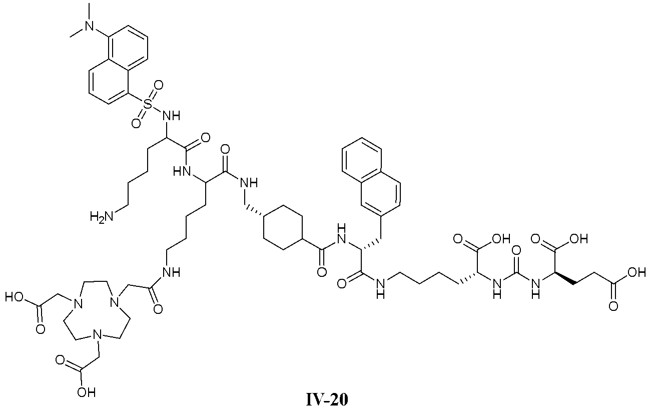

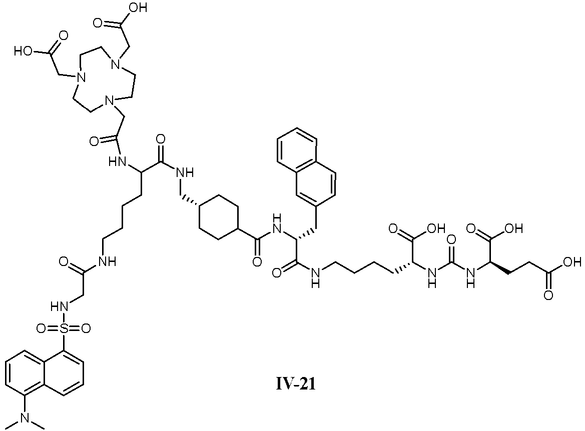

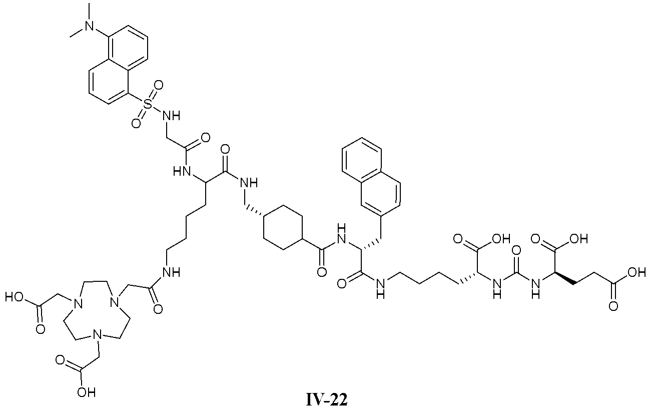

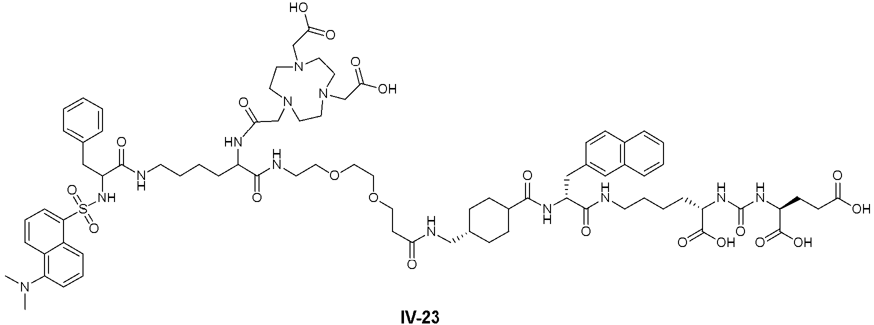

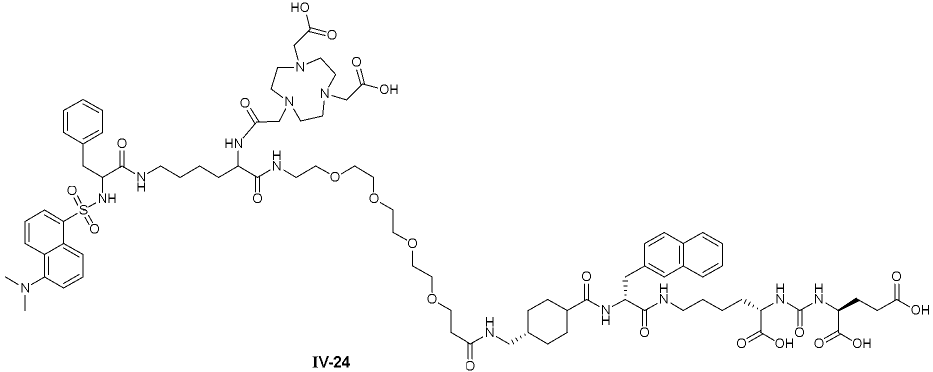

On this basis, the present invention further provides a method for preparing the compound represented by formula IV-1, including steps of:
reacting 5-(dimethylamino)naphthalene-1-sulfonyl chloride with an amino group of an amino acid to obtain a dansylamide derivative, subsequently activating an exposed carboxyl group of the amino acid via NHS (N-hydroxysuccinimide), and further performing an amide condensation reaction with Nε-Boc-L-lysine or (tert-butoxycarbonyl)-L-lysine; subsequently reacting with PSMA-617 with participation of 1-(3-dimethylaminopropyl)-3-ethylcarbodiimide hydrochloride and NHS, then removing a Boc protecting group under action of TFA, and subsequently reacting with PSMA-617 with participation of EDC and NHS; subsequently removing an Fmoc protection using piperazine; and finally reacting with DOTA-NHS (hydroxysuccinimide-tetraazacyclododecanetetraacetic acid) to obtain the compound having a structure as represented by formula IV-1 below.

In another aspect, the present invention further provides a radiolabeled complex, which is a complex obtained by labeling a radionuclide using the compound of formula A'-1 to A'-12 or the compound of formula I according to the present invention as a ligand. The radiolabeled complex can serve as a novel tumor radioactive theranostic probe, that is, it can serve as a radionuclide diagnostic probe or a radionuclide therapeutic probe. The radionuclide is preferably any one of ¹⁷⁷Lu, ¹⁸F , ⁴³Sc, ⁴⁴Sc, ⁴⁷Sc, ⁵⁵Co, ⁵⁷Co, ⁶⁸Ga, ⁶⁷Ga, ⁶²Cu, ⁶⁴Cu, ⁶⁷Cu, ⁸⁹Zr, ⁸⁶Y, ⁹⁰Y, ^{99m}Tc, ⁹⁹Tc, ⁹⁰ln, ¹¹¹ln, ⁸⁹Sr, ¹⁵²Gd, ¹⁵³Gd, ¹⁵³Sm, ¹⁴⁹Tb, ¹⁵¹Tb, ¹⁶¹Tb, ¹⁶⁶Ho, ¹⁶⁶Dy, ¹⁸⁶Re, ¹⁸⁸Re, ²¹²Pb, ²¹²Bi, ²¹³Bi, ²²³Ra, ²²⁵Ac, ²²⁶Th, ²²⁷Th, or ²¹¹At.

The present invention further provides methods for preparing other compounds, which can be specifically carried out by referring to the preparation of the compound represented by formula IV-1 above, with necessary structural substitutions.

In a preferred embodiment of the present invention, a structure of the radiolabeled complex is as represented by formula V below: 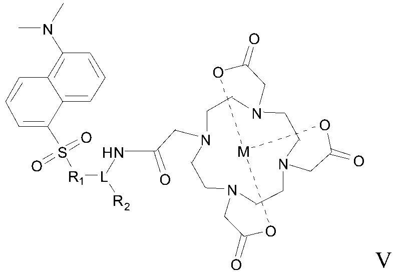
where L is -(X)ₙ-(CH₂)ₘ-(Y)_{q}-, where n is an integer from 0 to 10 (preferably an integer from 0-5), X and Y are independently selected from lysine, glutamic acid, or a derivative structure containing lysine and glutamic acid, m is an integer from 0 to 20 (preferably an integer from 0-10), q is an integer from 0 to 6 (preferably an integer from 0-3), and each CH₂ may be individually replaced with -OCH₂-, -NH(CO)-, or -(CO)-NH-;
R₁ is of an amino acid analog structure, including an L-amino acid, a D-amino acid, and a derivative thereof 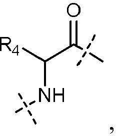 where R₄ includes but is not limited to: (CH₂)ᵣ-aryl, (CH₂)ᵣ-heteroaryl, (CH₂)ᵣ-COOH, (CH₂)ᵣ-NH₂, (CH₂)ᵣ-OH, (CH₂)ᵣ-SH, (CH₂)ᵣ-naphthylmethyl, (CH₂)ᵣ-indolyl, and (CH₂)ᵣ-benzothiazolyl, where r is an integer from 0 to 10 (for example, may be 0, 1, 2, 3, 4, 5, 6, 7, 8, 9, or 10). In a preferred embodiment, R₁ may specifically be of the following structure: 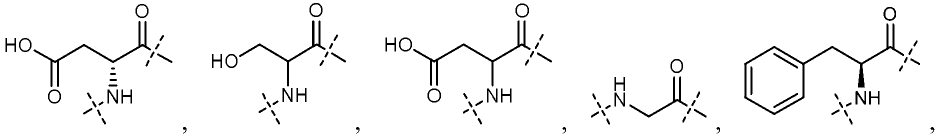 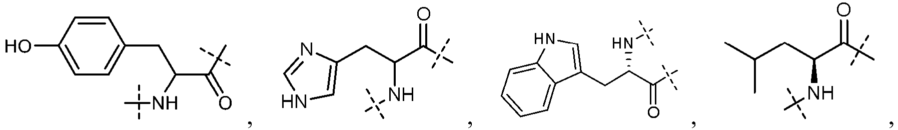 or 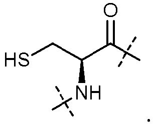
R₂ is derived from a prostate-specific membrane antigen-targeting compound, as shown in formula II: 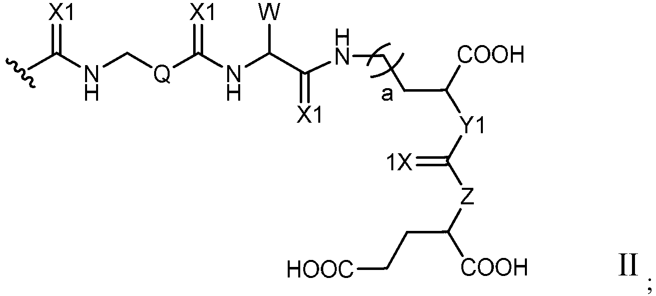 where:
   X1 structures at different positions are the same or different and are independently selected from O, N, S, or P;
   Y1 is O, S, or NH;
   Z is O, S, or NH;
   a is an integer from 0 to 20; Q is selected from substituted or unsubstituted alkyl, substituted or unsubstituted alkylaryl, or substituted or unsubstituted cycloalkyl; and
   W is selected from -(CH₂)_{b}-aryl or -(CH₂)_{b}-heteroaryl, where b is an integer from 0-10 (for example, may be 0, 1, 2, 3, 4, 5, 6, 7, 8, 9, or 10).

In a preferred embodiment, R₂ may be any one of the following structures: 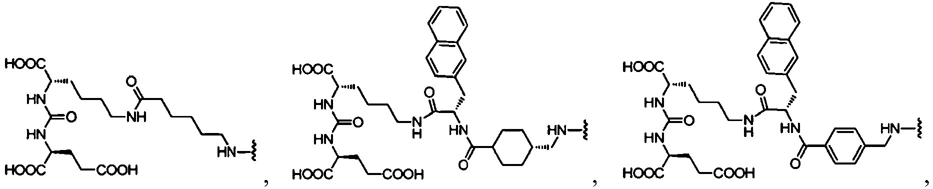 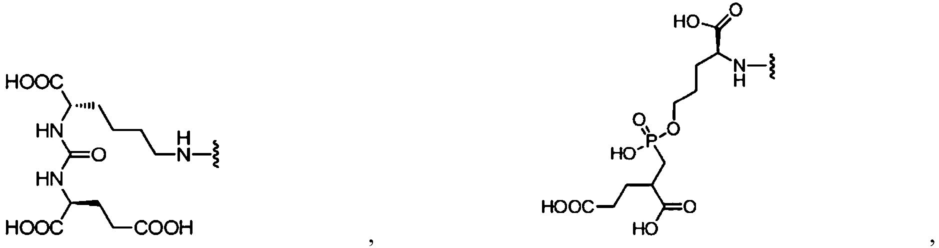 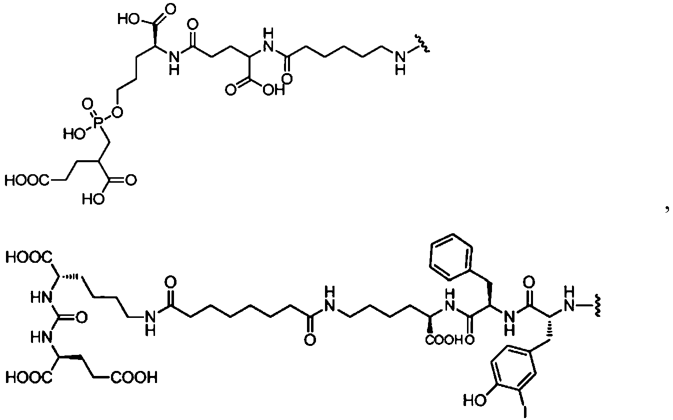 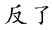 or
M is a radionuclide selected from any one of ¹⁷⁷Lu, ¹⁸F, ⁴³Sc, ⁴⁴Sc, ⁴⁷Sc, ⁵⁵Co, ⁵⁷Co, ⁶⁸Ga, ⁶⁷Ga, ⁶²Cu, ⁶⁴Cu, ⁶⁷Cu, ⁸⁹Zr, ⁸⁶Y, ⁹⁰Y, ^{99m}Tc, ⁹⁹Tc, ⁹⁰ln, ¹¹¹ln, ⁸⁹Sr, ¹⁵²Gd, ¹⁵³Gd, ¹⁵³Sm, ¹⁴⁹Tb, ¹⁵¹Tb, ¹⁶¹Tb, ¹⁶⁶Ho, ¹⁶⁶Dy, ¹⁸⁶Re, ¹⁸⁸Re, ²¹²Pb, ²¹²Bi, ²¹³Bi, ²²³Ra, ²²⁵Ac, ²²⁶Th, ²²⁷Th, or ²¹¹At.

The remaining limitations are as described above and will not be repeated herein.

In some preferred embodiments, the compound of formula V above may also be subjected to chelating agent replacement, such as replacing DOTA with NOTA. The remaining limitations are as described above and will not be repeated herein.

The radiolabeled complex according to the present invention can be prepared through a radionuclide-containing compound and the compound of formula I according to the present invention following various existing labeling methods. A preferred labeling method of the present invention is a wet method or a lyophilization method below:
Steps of the wet method are as follows: dissolving an appropriate amount of the compound of formula I according to the present invention in a buffer solution or deionized water, then adding a radionuclide-containing solution, reacting at room temperature to 100°C for 5-30 min, then diluting with physiological saline or water for injection, and filtering through a sterile filter membrane to generate a radionuclide-labeled complex injection.

Steps of the lyophilization method are as follows: dissolving an appropriate amount of the compound of formula I according to the present invention in a buffer solution or deionized water, dispensing into lyophilization containers, and sealing into lyophilized kits after lyophilization, where relevant excipients, antioxidants, or acid-base regulators may be added to the lyophilized kits as needed; dissolving by adding deionized water or a buffer solution into the lyophilized kits, then adding a radionuclide-containing solution, reacting at room temperature to 100°C for 5-30 min, diluting with physiological saline or water for injection, and filtering through a sterile filter membrane to generate a radionuclide-labeled complex injection.

The present invention further provides use of the radiolabeled complex represented by formula V or other radiolabeled complexes (e.g., substituting DOTA in formula V with NOTA or other chelators) in nuclide therapy and imaging of a PSMA-highly-expressing tumor in a mammal.

In the preferred use of the present invention, the complex is prepared as an injection and administered via intravenous injection for use in a human patient or a mammal with a PSMA-highly-expressing tumor.

The present invention provides a dansylamide-modified prostate-specific membrane antigen-targeting compound, and a radionuclide-labeled complex thereof, and provides methods for preparing and labeling such compounds. Biological test results indicate that it has a moderately prolonged blood circulation half-life, enhanced tumor uptake and enrichment, and retention time. This novel performance is not possessed by other current PSMA imaging agents, making it suitable for use in nuclide therapy and imaging of PSMA-highly expressing tumors.

### BRIEF DESCRIPTION OF DRAWINGS

FIG. 1 is a mass spectrometry identification spectrum of a compound of formula IV-1 according to the present invention.
FIG. 2 is a mass spectrometry identification spectrum of a compound of formula IV-5 according to the present invention.
FIG. 3 is a mass spectrometry identification spectrum of a compound of formula IV-9 according to the present invention.
FIG. 4 is a mass spectrometry identification spectrum of a compound of formula IV-13A according to the present invention.
FIG. 5 is a mass spectrometry identification spectrum of a compound of formula IV-13 according to the present invention.
FIG. 6 shows ⁶⁸Ga labeling identification results of a compound of formula IV-1 according to the present invention.
FIG. 7 shows stability of ⁶⁸Ga-formula IV-1 compound according to the present invention in physiological saline and serum.
FIG. 8 shows stability of ⁶⁸Ga-formula IV-3 compound according to the present invention in physiological saline and serum.
FIG. 9 shows stability of ⁶⁸Ga-formula IV-9 compound according to the present invention in physiological saline and serum.
FIG. 10 shows results of cellular uptake experiments and saturation binding experiments of ⁶⁸Ga-formula IV-1 compound and ⁶⁸Ga-PSMA617 according to the present invention.
FIG. 11 shows a MicroPET image and quantification of regions of interest thereof of ⁶⁸Ga-formula IV-1 compound according to the present invention in PC3-PIP model mice.
FIG. 12 shows a MicroPET image and quantification of regions of interest thereof of ⁶⁸Ga-formula IV-9 compound according to the present invention in PC3-PIP model mice.
FIG. 13 shows biodistribution of ¹⁷⁷Lu-formula IV-1 compound according to the present invention in normal mice.
FIG. 14 is a SPECT image of PC3-PIP model mice after administration of ¹⁷⁷Lu-formula IV-1 compound according to the present invention.
FIG. 15 shows tissue uptake results of PC3-PIP model mice after administration of ¹⁷⁷Lu-formula IV-1 compound according to the present invention.
FIG. 16 is a PET image of prostate cancer patients after administration of ⁶⁸Ga-formula IV-1 compound according to the present invention.
FIG. 17 shows quantification of regions of interest in prostate cancer patients after administration of ⁶⁸Ga-formula IV-1 compound according to the present invention.
FIG. 18 is a PET image of prostate cancer patients after administration of ⁶⁸Ga-formula IV-9 compound according to the present invention.
FIG. 19 shows quantification of regions of interest in prostate cancer patients after administration of ⁶⁸Ga-formula IV-9 compound according to the present invention.

### DETAILED DESCRIPTION OF EMBODIMENTS

The technical solution of the present invention is further illustrated and described below through specific embodiments in conjunction with the drawings.

### Example 1: Preparation of a compound of formula IV-1

The synthetic route is as follows:

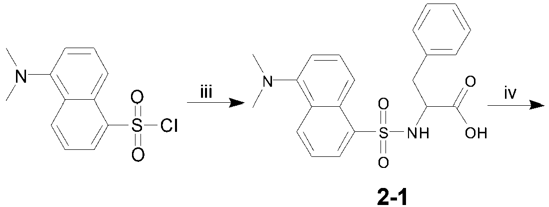

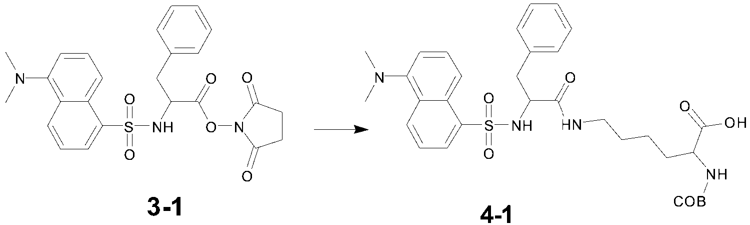

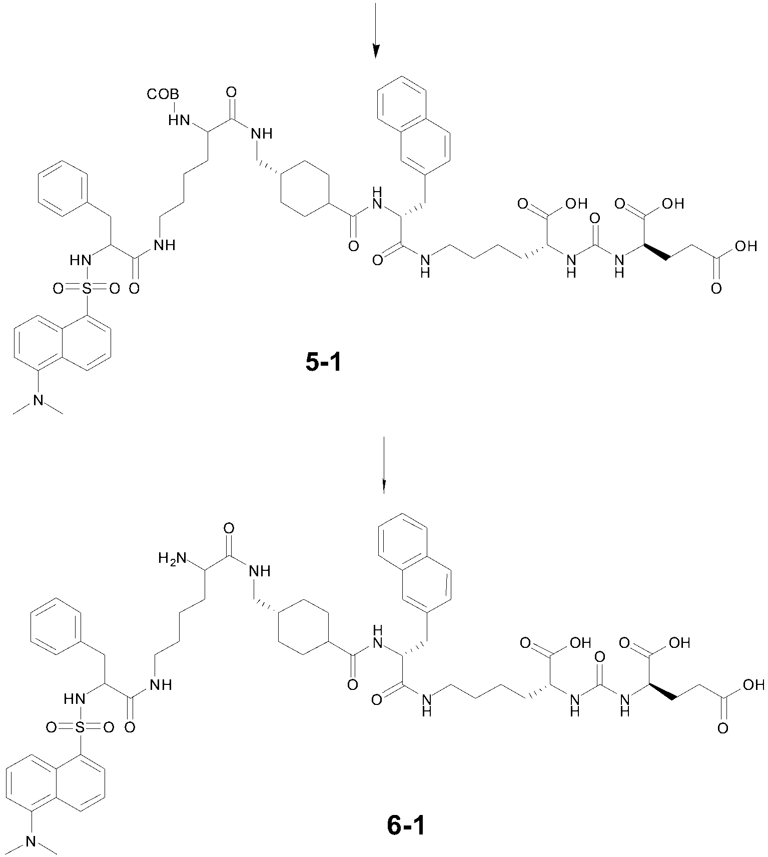

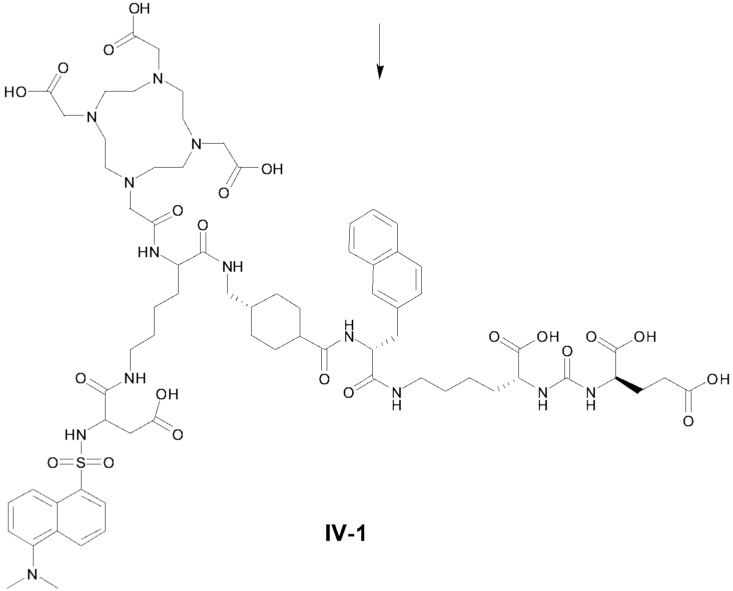

The specific steps are as follows:

### Synthesis of compound 2-1:

L-phenylalanine (1.69 g, 10 mmol) was dissolved in 20 mL of 10% sodium carbonate solution. Then, 5-(dimethylamino)naphthalene-1-sulfonyl chloride (2.69 g, 10 mmol) was dissolved in 20 mL of 1,4-dioxane and added dropwise to the L-phenylalanine solution under ice bath conditions. The mixture was reacted at room temperature for 4 h. After the reaction was completed, 1M dilute hydrochloric acid was added to adjust the pH to about 4, followed by extraction. The organic phase was taken, and the solvent was removed by distillation under reduced pressure to obtain a crude product, which was purified by a silica gel column to obtain compound 2-1 as a pale yellow solid with a yield of 81%.

### Synthesis of compound 3-1:

Compound **2-1** (400 mg, 1 mmol) and NHS (230 mg, 2 mmol) were dissolved in 10 mL of tetrahydrofuran. Dicyclohexylcarbodiimide (DCC) (412 mg, 2 mmol) was added dropwise under ice bath conditions. The mixture was reacted at room temperature overnight. After the reaction was completed, the mixture was filtered, then the filtrate was taken, the solvent was removed by distillation under reduced pressure to obtain a crude product, which was purified by a silica gel column to obtain compound **3-1** as a pale yellow solid with a yield of 34%.

### Synthesis of compound 4-1:

Compound **3-1** (495 mg, 1 mmol) and (tert-butoxycarbonyl)-L-lysine (246 mg, 1 mmol) were dissolved in 10 mL of dimethyl sulfoxide (DMSO). Then, triethylamine (505 mg, 5 mmol) was added. The mixture was reacted at room temperature overnight. After the reaction was completed, dilute hydrochloric acid was added for extraction three times. The organic phase filtrate was taken, and the solvent was removed by distillation under reduced pressure to obtain a crude product, which was purified by a silica gel column to obtain compound **4-1** as a pale yellow solid with a yield of 76%.

### Synthesis of compound 5-1:

Compound **4-1** (112 mg, 0.2 mmol), 1-(3-dimethylaminopropyl)-3-ethylcarbodiimide hydrochloride (40 mg, 0.2 mmol), N-hydroxysuccinimide (23 mg, 0.2 mmol), and 5 mL of N,N-dimethylformamide were charged to a 10 mL flask, respectively. After 4 h of reaction, N,N-diisopropylethylamine (60 mg, 0.5 mmol) and PSMA-617 (130 mg, 0.2 mmol) were added. The resulting mixture was stirred for reaction, and the completion of the reaction was monitored by HPLC. The solvent was removed by distillation under reduced pressure to obtain a crude product. The crude product was purified by a reversed-phase column and lyophilized to obtain pure compound **5-1** with a yield of 59%.

### Synthesis of compound 6-1:

120 mg of compound **5-1** was dissolved in 5 mL of 2 M hydrochloric acid-ethyl acetate solution and stirred at room temperature for 4 h. After the reaction was completed, the mixture was filtered to obtain an insoluble substance, which was washed by repeatedly adding ethyl acetate three times to obtain compound **6-1** with a yield of 92%.

### Synthesis of the compound of formula IV-1:

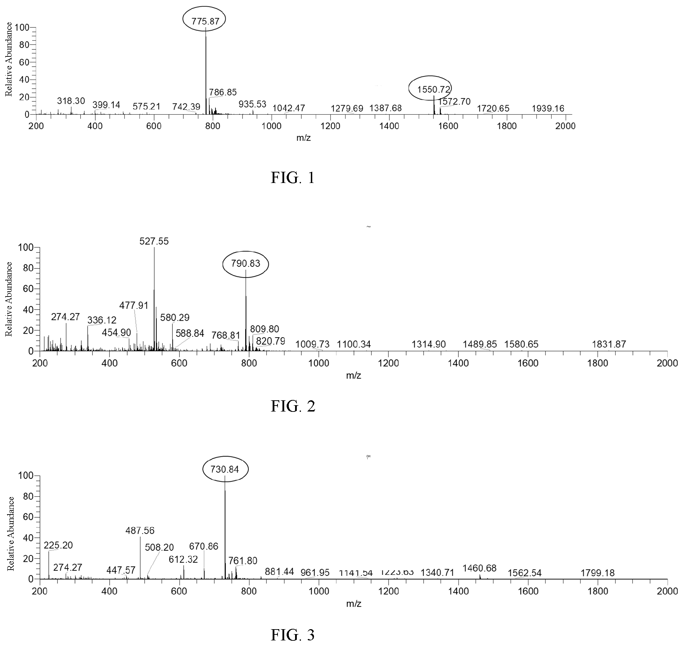

Compound **6-1** (5.5 mg, 5 µmol), hydroxysuccinimide-tetraazacyclododecanetetraacetic acid (DOTA-NHS) (8 mg, 2 µmol), and N,N-diisopropylethylamine (3.2 mg, 25 µmol) were sequentially placed into 1 mL of N,N-dimethylformamide. The reaction system was stirred for reaction at room temperature, and the reaction was monitored by HPLC until completion. The crude product was purified by HPLC and lyophilized to obtain the pure compound of formula IV-1 with a yield of 52%. Structural characterization is shown in FIG. 1.

### Example 2. Preparation of a compound of formula IV-3

The synthetic route of this example is as follows:

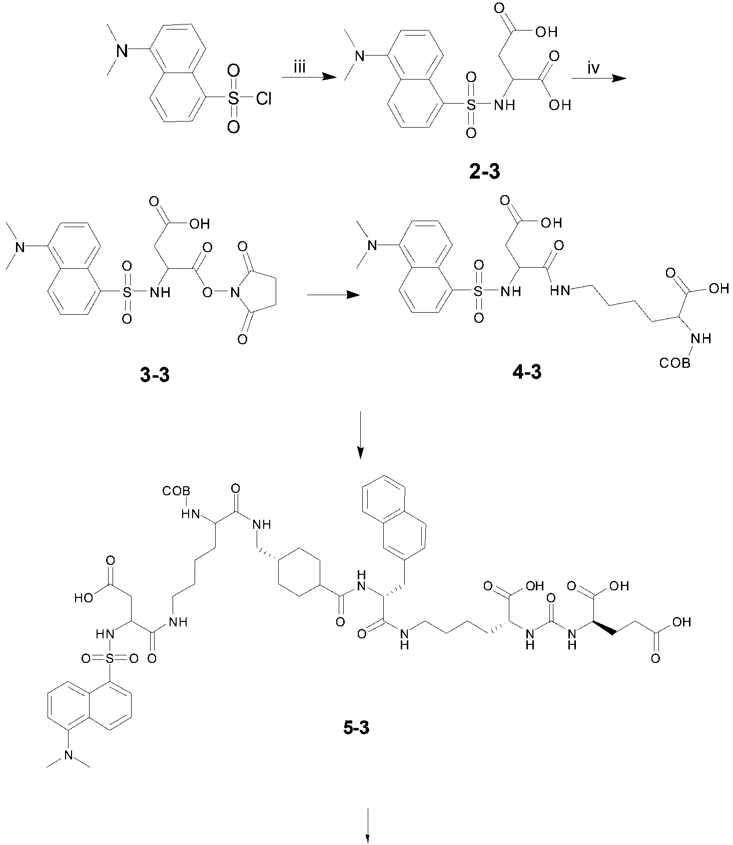

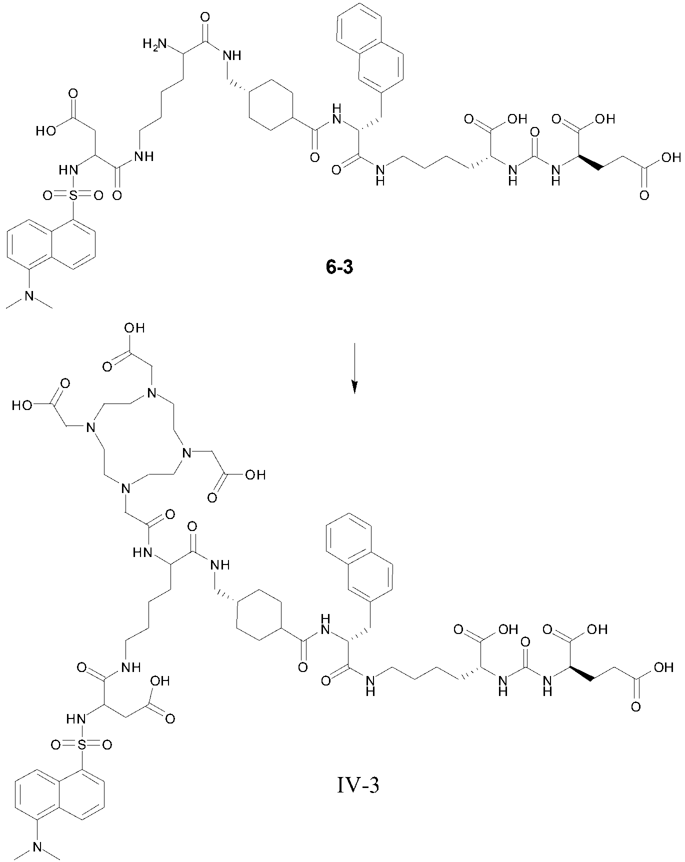

This example was carried out with reference to Example 1. L-phenylalanine, which was reacted with 5-(dimethylamino)naphthalene-1-sulfonyl chloride in Example 1, was replaced with L-aspartic acid to obtain compound 2-3. Then, with reference to the process for preparing compound 3-1 in Example 1, compound 2-1 in the above process was replaced with compound 2-3 to obtain compound 3-3. Further, with reference to the process from preparing compound 4-1 to compound IV-1 in Example 1, the compound of formula IV-3 was finally obtained.

### Example 3. Preparation of a compound of formula IV-5

The synthetic route of this example is as follows:

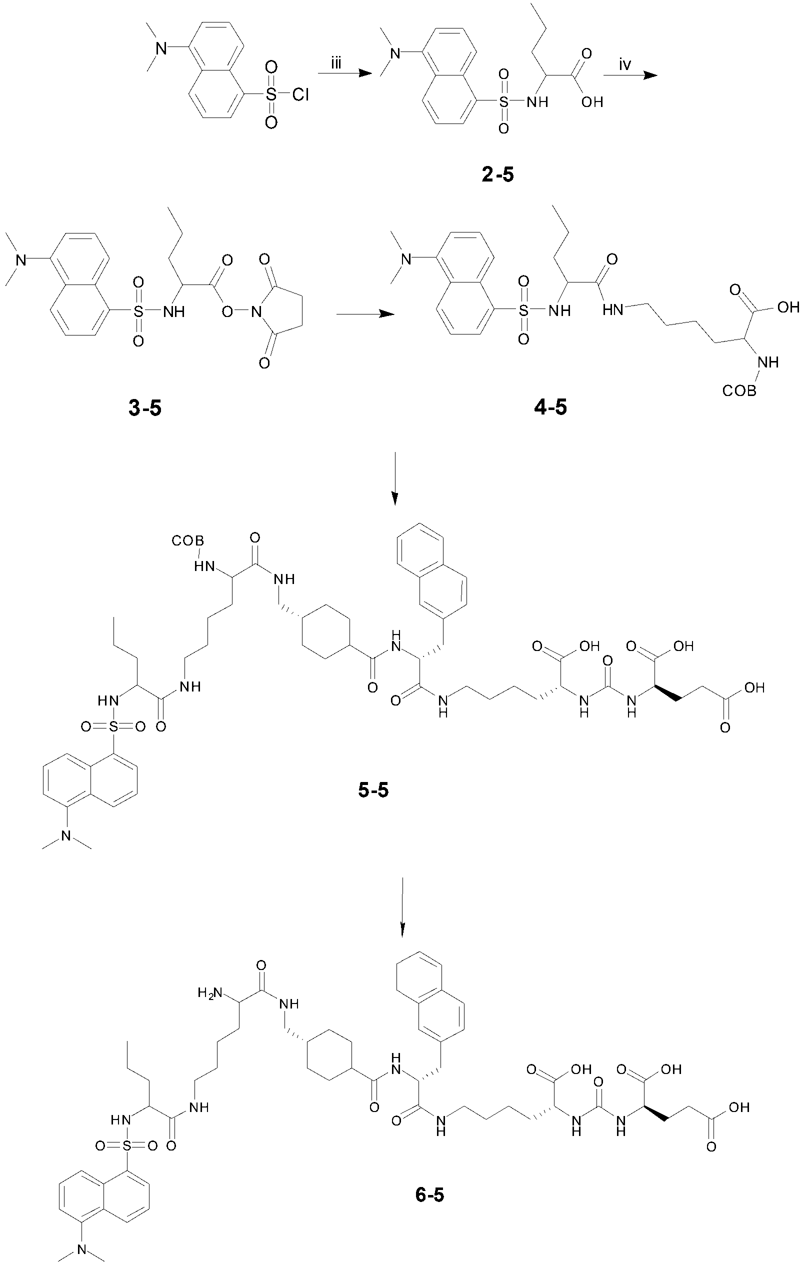

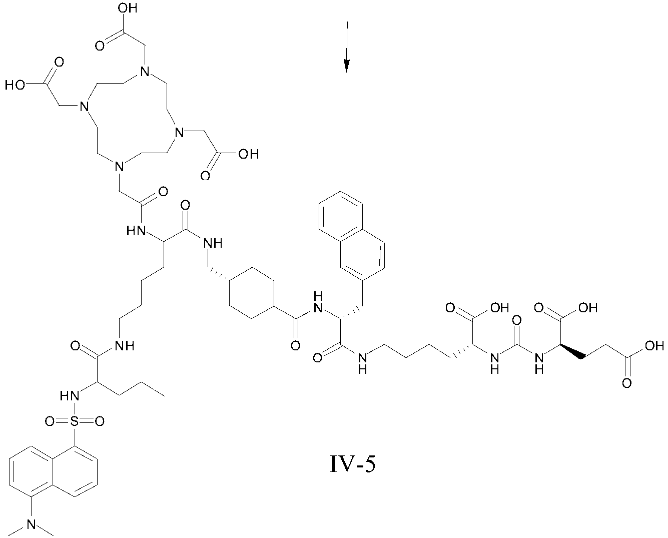

The above preparation steps of this example were carried out with reference to Example 1. L-phenylalanine, which was reacted with 5-(dimethylamino)naphthalene-1-sulfonyl chloride in Example 1, was replaced with L-norvaline to obtain compound 2-5. Then, with reference to the process for preparing compound 3-1 in Example 1, compound 2-1 in the above process was replaced with compound 2-5 to obtain compound 3-5. Further, with reference to the process from preparing compound 4-1 to compound IV-1 in Example 1, the compound of formula IV-5 was finally obtained. Its structural characterization is shown in FIG. 2.

### Example 4: Preparation of a compound of formula IV-7

The synthetic route of this example is as follows:

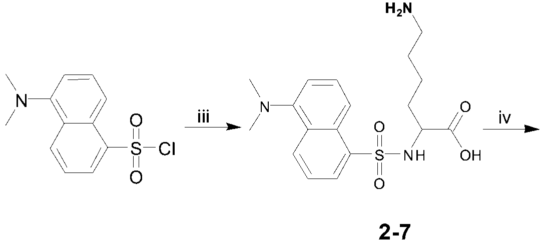

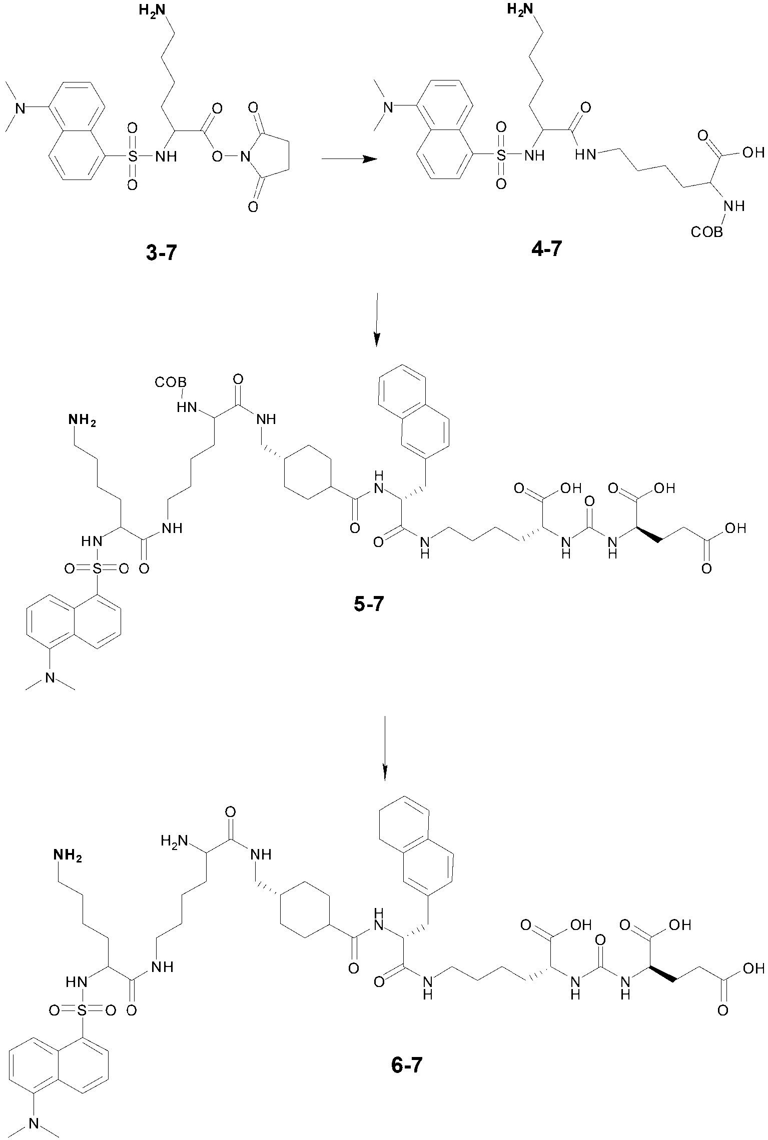

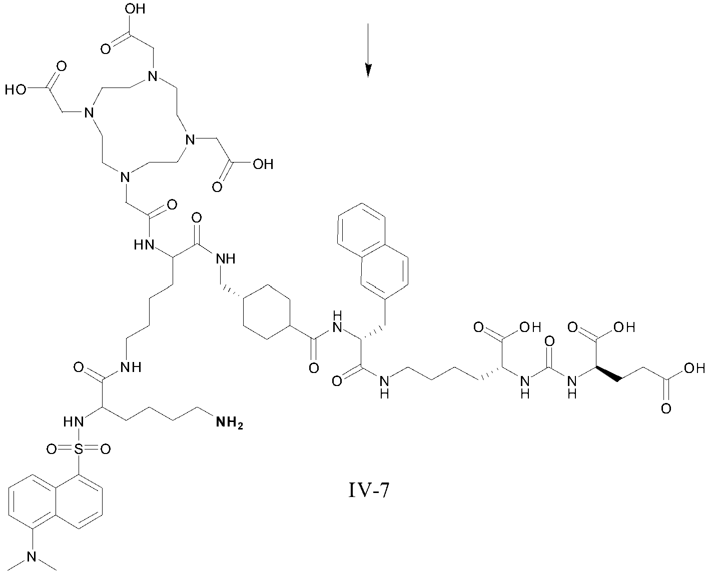

The above preparation steps of this example were carried out with reference to Example 1. L-phenylalanine, which was reacted with 5-(dimethylamino)naphthalene-1-sulfonyl chloride in Example 1, was replaced with L-lysine to obtain compound 2-7. Then, with reference to the process for preparing compound 3-1 in Example 1, compound 2-1 in the above process was replaced with compound 2-7 to obtain compound 3-7. Further, with reference to the process from preparing compound 4-1 to compound IV-1 in Example 1, the compound of formula IV-7 was finally obtained.

### Example 5. Preparation of a compound of formula IV-9

The synthetic route of this example is as follows:

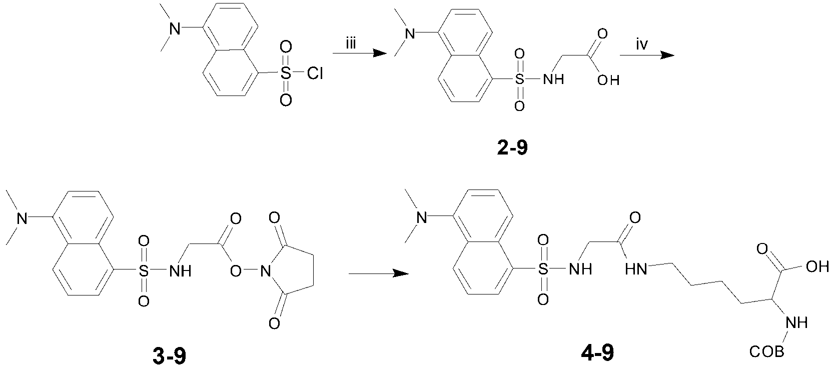

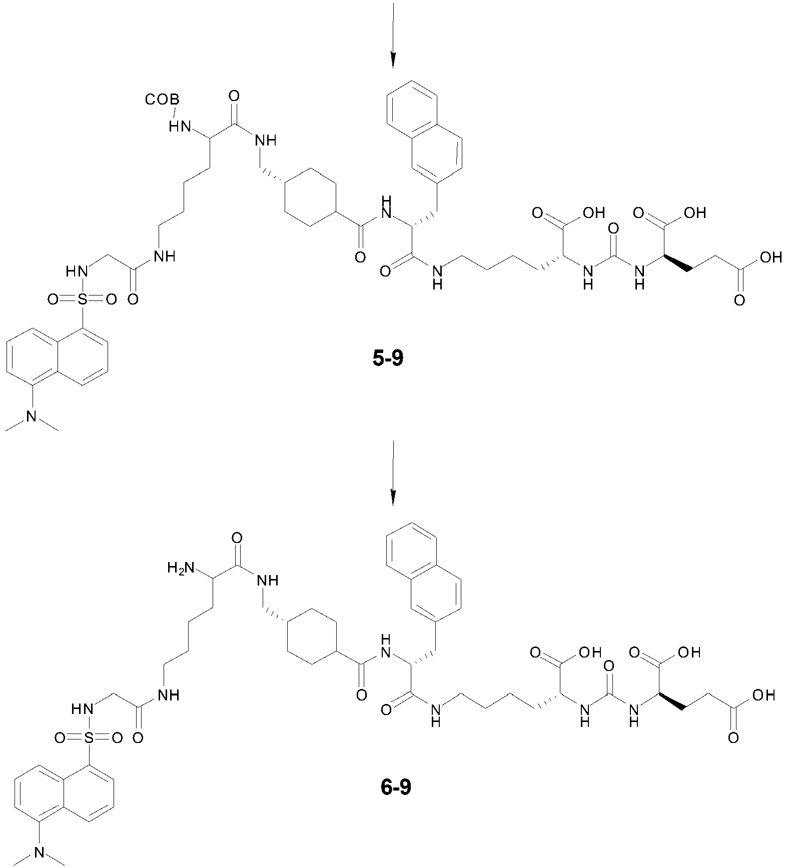

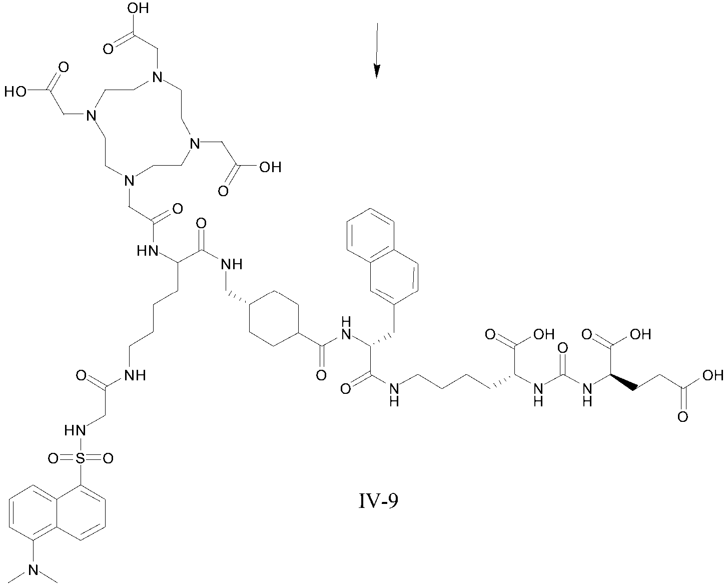

The above preparation steps of this example were carried out with reference to Example 1. L-phenylalanine, which was reacted with 5-(dimethylamino)naphthalene-1-sulfonyl chloride in Example 1, was replaced with L-glycine to obtain compound 2-9. Then, with reference to the process for preparing compound 3-1 in Example 1, compound 2-1 in the above process was replaced with compound 2-9 to obtain compound 3-9. Further, with reference to the process from preparing compound 4-1 to compound IV-1 in Example 1, the compound of formula IV-9 was finally obtained. Its structural characterization is shown in FIG. 3.

### Example 6. Preparation of a compound of formula IV-2

With reference to Example 1, (tert-butoxycarbonyl)-L-lysine, which was reacted with compound 3-1 in Example 1, was replaced with Nε-Boc-L-lysine to sequentially prepare compound 4-2, compound 5-2, and compound 6-2, and the compound of formula IV-2 was finally obtained.

The reaction route is as follows:

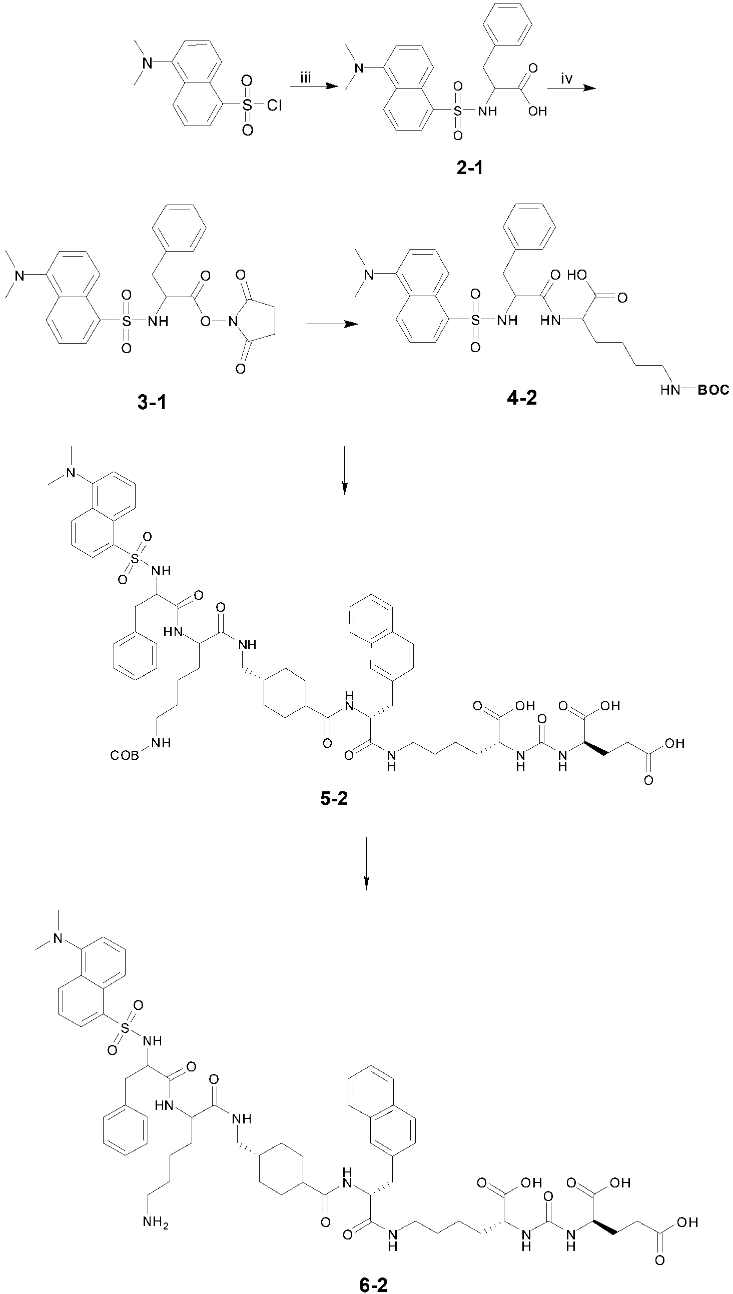

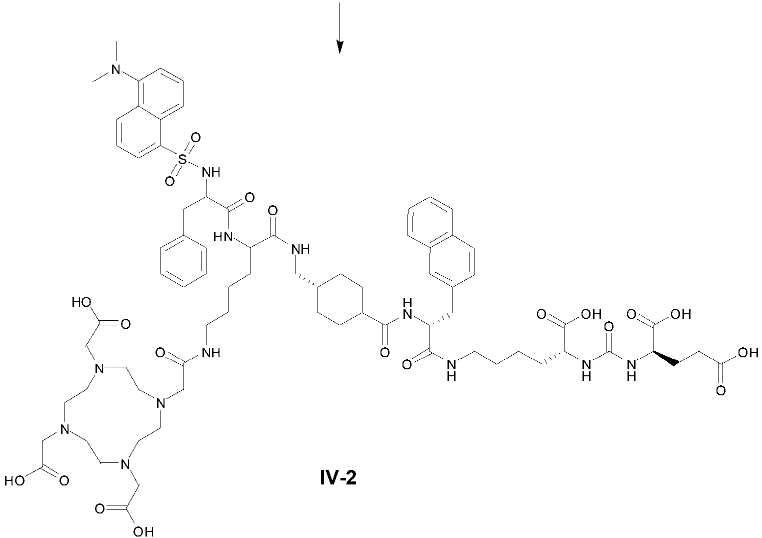

### Example 7. Preparation of a compound of formula IV-4

With reference to Example 2, (tert-butoxycarbonyl)-L-lysine, which was reacted with compound 3-3 in Example 2, was replaced with Nε-Boc-L-lysine to sequentially prepare compound 4-4, compound 5-4, and compound 6-4, and the compound of formula IV-4 was finally obtained.

The reaction route is as follows:

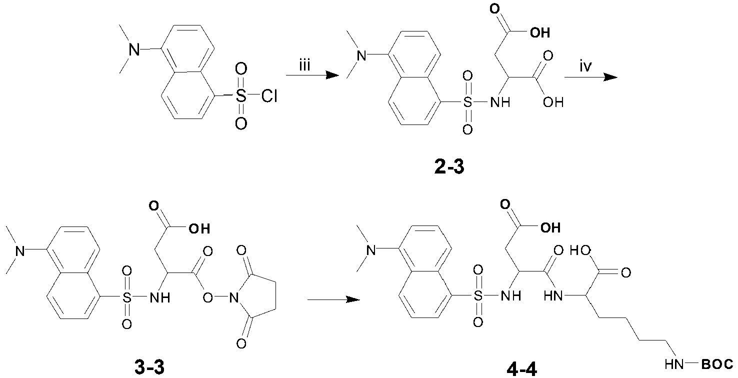

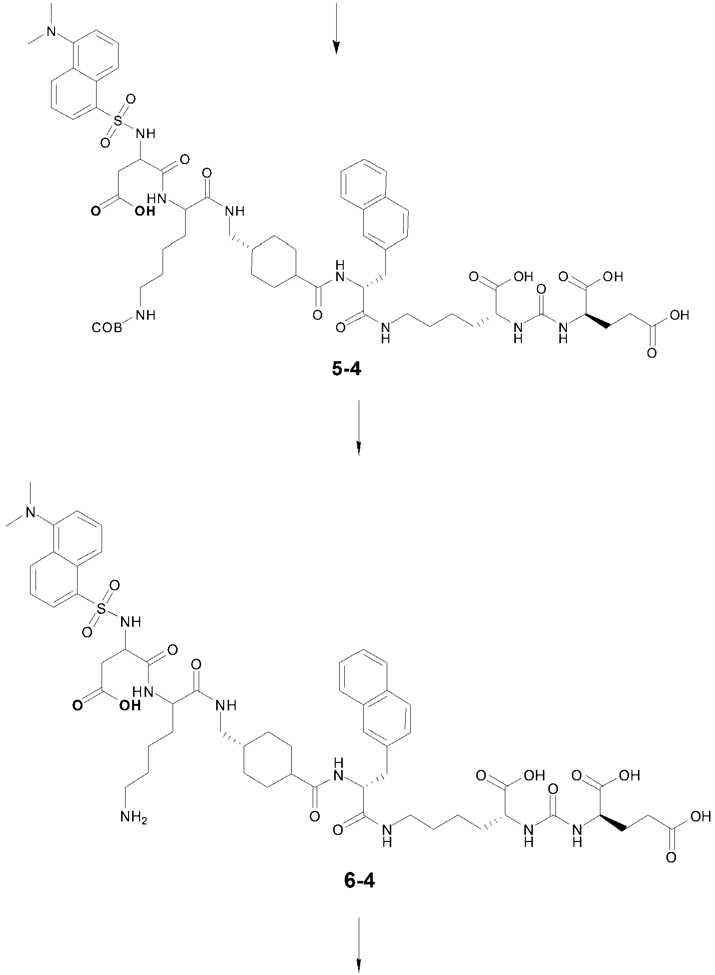

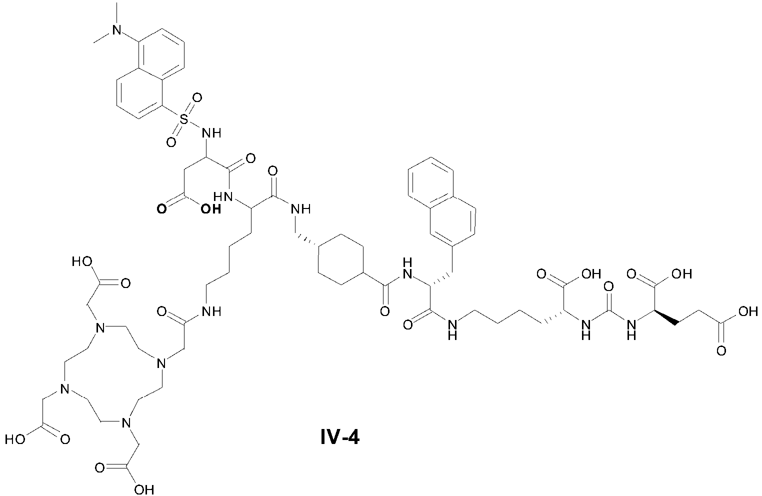

### Example 8. Preparation of a compound of formula IV-6

With reference to Example 3, (tert-butoxycarbonyl)-L-lysine, which was reacted with compound 3-5 in Example 3, was replaced with Nε-Boc-L-lysine to sequentially prepare compound 4-6, compound 5-6, and compound 6-6, and the compound of formula IV-6 was finally obtained.

The reaction route is as follows:

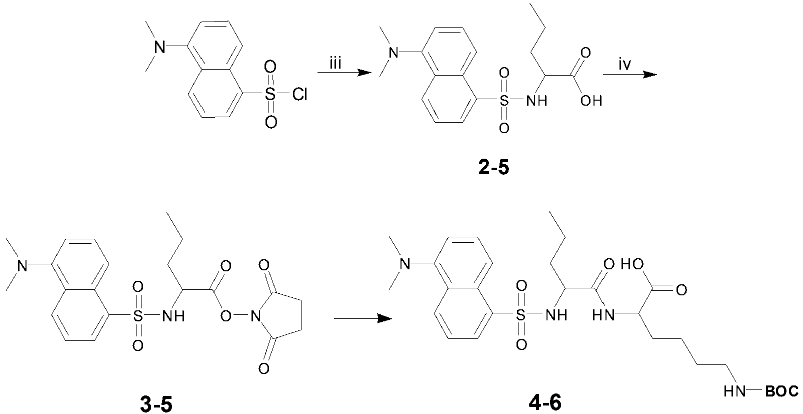

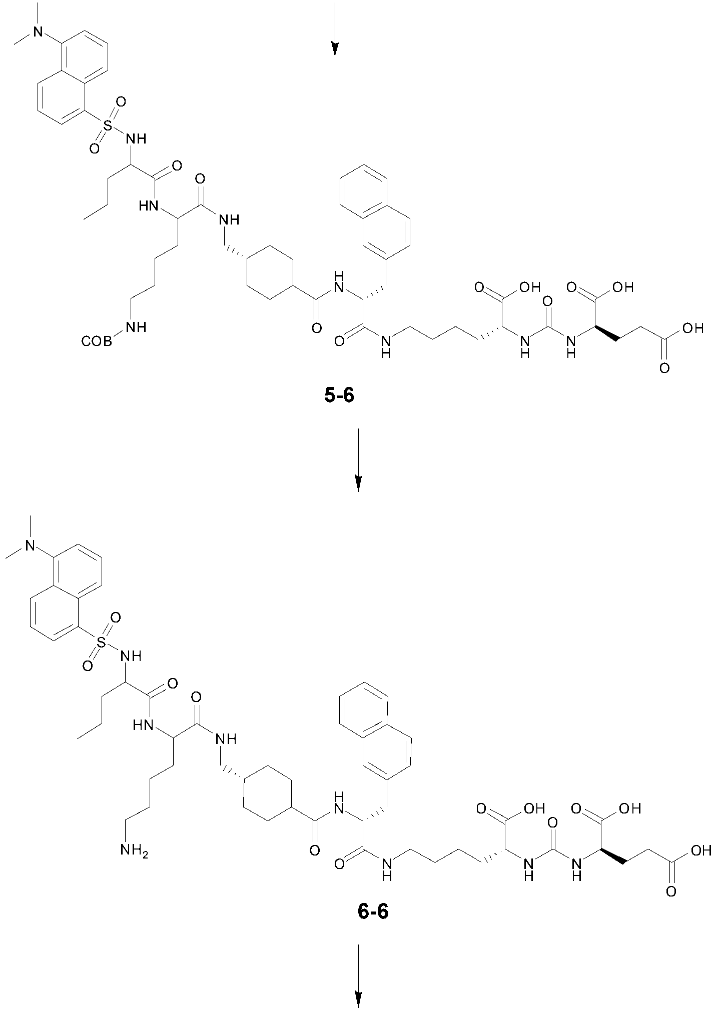

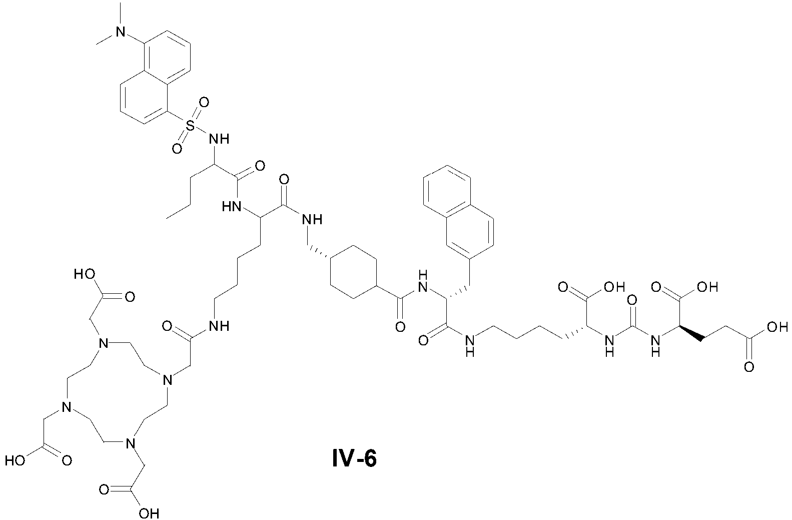

### Example 9. Preparation of a compound of formula IV-8

With reference to Example 4, (tert-butoxycarbonyl)-L-lysine, which was reacted with compound 3-7 in Example 4, was replaced with Nε-Boc-L-lysine to sequentially prepare compound 4-8, compound 5-8, and compound 6-8, and the compound of formula IV-8 was finally obtained.

The reaction route is as follows:

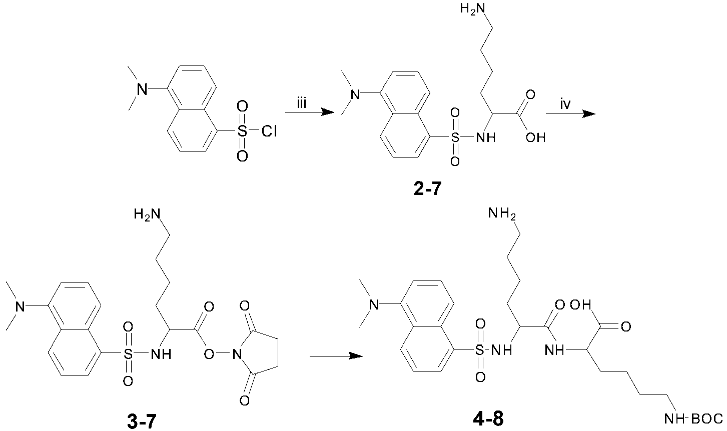

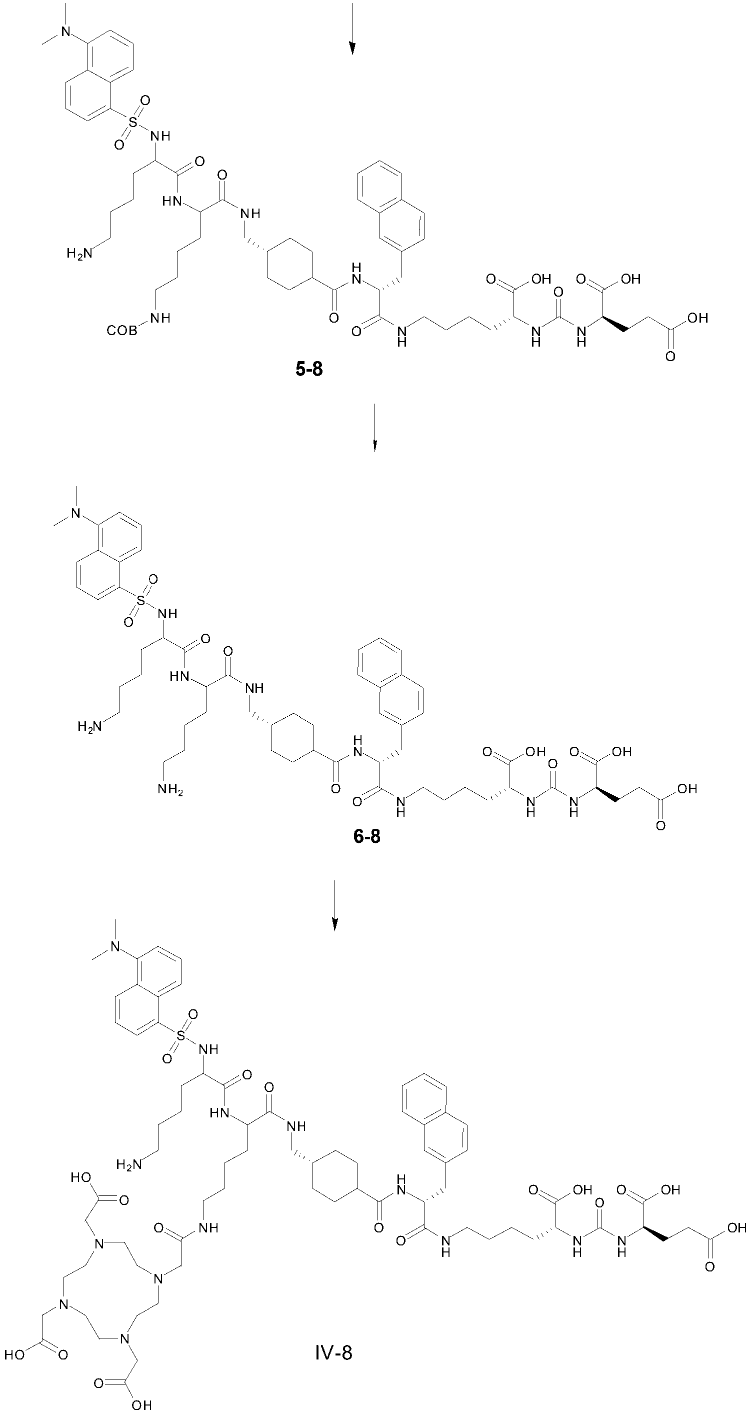

### Example 10. Preparation of a compound of formula IV-10

With reference to Example 5, (tert-butoxycarbonyl)-L-lysine, which was reacted with compound 3-9 in Example 5, was replaced with Nε-Boc-L-lysine to sequentially prepare compound 4-10, compound 5-10, and compound 6-10, and the compound of formula IV-10 was finally obtained.

The reaction route is as follows:

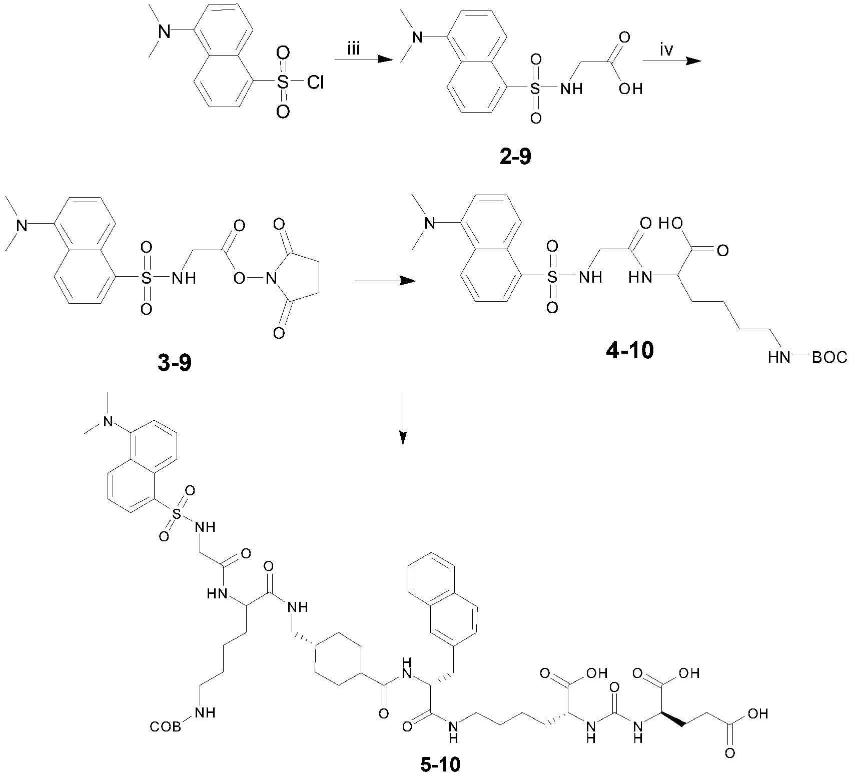

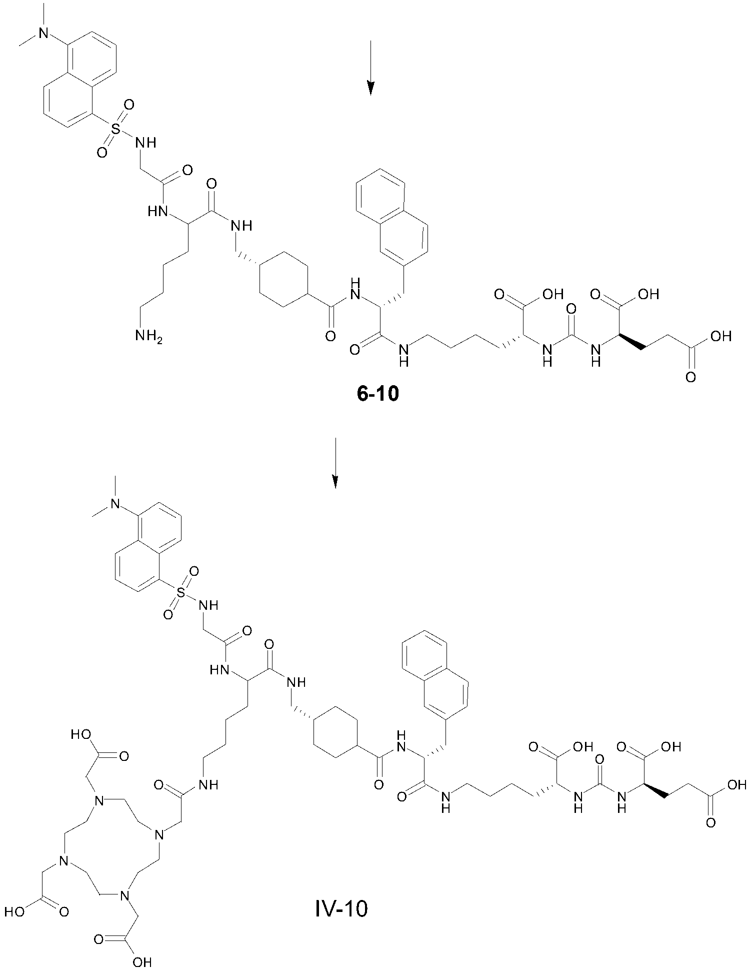

### Example 11-Example 12: Preparation of a compound of formula IV-11 and a compound of formula IV-12

Their preparation methods can be carried out with reference to Examples 1-10. For example, based on Example 1, Lys, which was reacted with compound **3-1,** was replaced with Lys-PEG₂-COOH or Lys-PEG₄-COOH, respectively, to obtain the corresponding structures as follows:

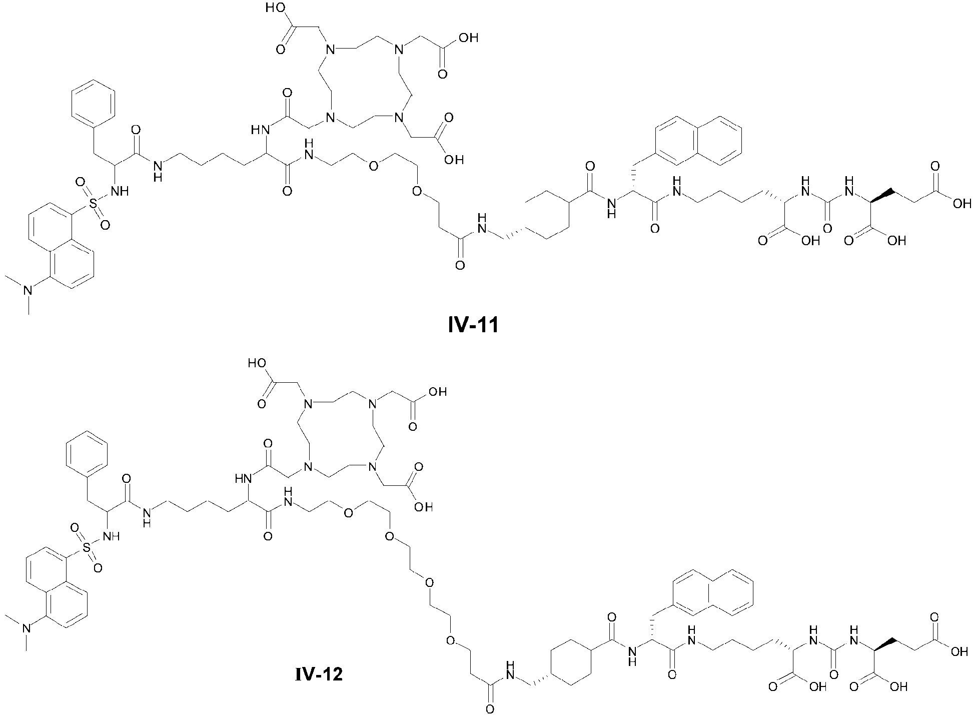

### Example 13. Preparation of a compound of formula IV-13

The synthetic route of this example is as follows:

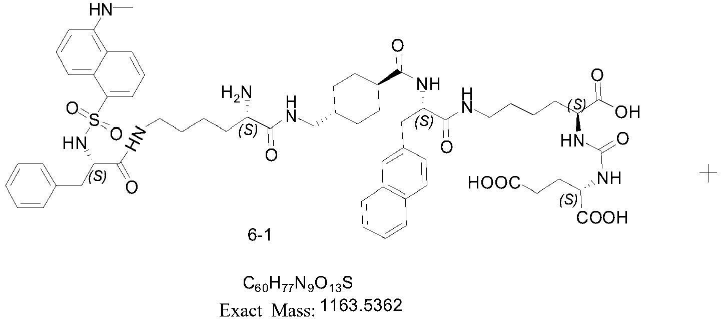

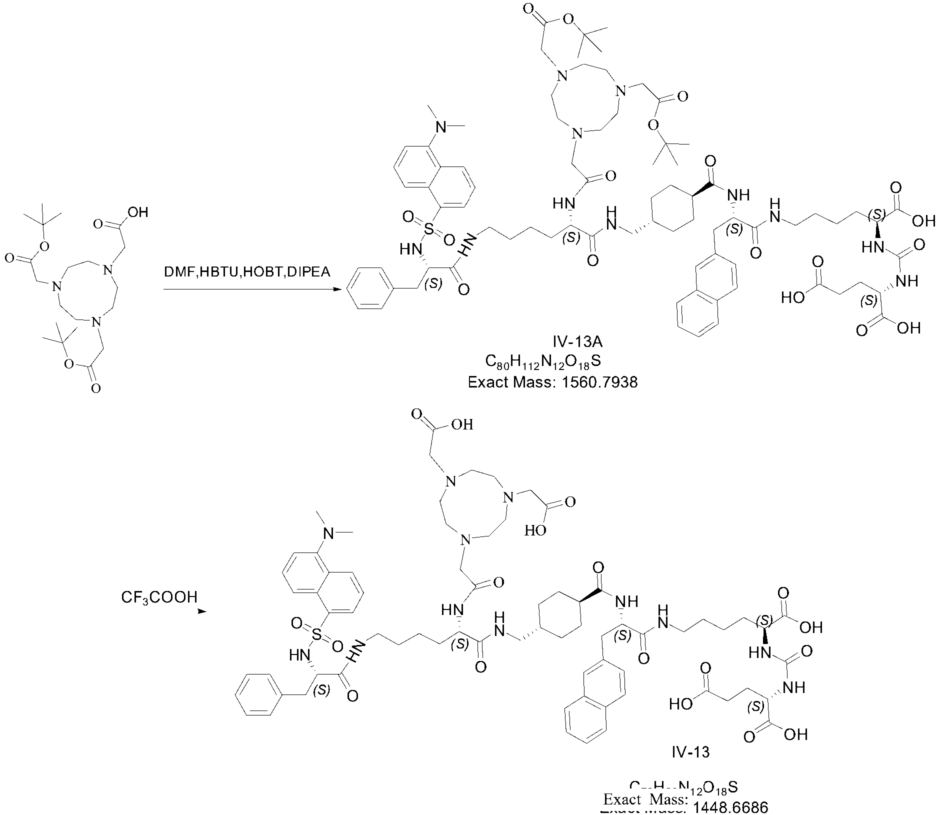

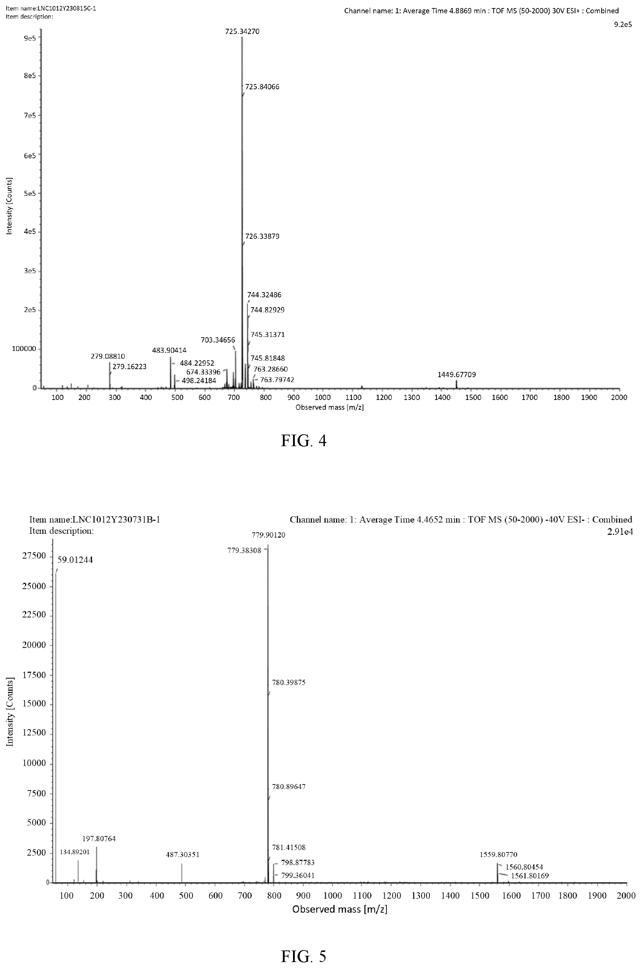

The specific steps are as follows:
NOTA-Bis(t-Bu Ester) was dissolved in DMF, DIPEA was added, and the temperature was lowered to 10°C. HBTU was then added, and the mixture was maintained at 10°C and reacted for 30 min. The compound of formula 6-1 prepared in Example 1 was taken and added to DIPEA to form a suspension, which was then added to the above reaction solution. After the mixture was stirred for reaction for 1 h, the reaction solution was added dropwise to MTBE to precipitate a solid. After standing, the supernatant was decanted. The resulting solid was purified by preparative high performance liquid chromatography to obtain compound IV-13A. Its structural characterization is shown in FIG. 4.

Compound IV-13A was dissolved in a solution of TFA:water = 100:5 and reacted at 30°C for 3 h. Then, MTBE was added dropwise to the reaction solution to precipitate a solid. After filtration, the resulting solid was purified by preparative high performance liquid chromatography and lyophilized to obtain the compound of formula IV-13. Its structural characterization is shown in FIG. 5.

Other compounds provided by the present invention, such as IV-14, IV-15, IV-16, IV-17, IV-18, IV-19, IV-20, IV-21, IV-22, IV-23, and IV-24, were prepared by referring to the methods provided in Examples 1-13.

### Example 14. Preparation of a radionuclide ⁶⁸Ga-labeled complex

The ⁶⁸Ga radionuclide labeling process is as follows:
Wet method: About 37-3700 MBq of ⁶⁸GaCl₃ hydrochloric acid solution (eluted from a germanium-gallium generator) was added to centrifuge tubes containing solutions of the compounds to be labeled (the solutions of the compounds to be labeled were 0.5-2 mL of acetic acid-acetate solutions containing 20-200 µg of each compound prepared in Example 1-Example 13). The mixture was placed at room temperature to 100°C and reacted for 20 min, then cooled to room temperature, diluted with physiological saline or water for injection, and subjected to sterile filtration to obtain a labeled compound injection, designated as "⁶⁸Ga-formula IV."

Lyophilization method: A certain amount of buffer solution and about 37-3700 MBq of ⁶⁸GaCl₃ hydrochloric acid solution (eluted from a germanium-gallium generator) were added to lyophilized kits containing 20-200 µg of the compounds to be labeled (the compounds to be labeled were each compound prepared in Example 1-Example 13). After mixing and dissolving, the mixture was placed at room temperature to 100°C and reacted for 20 minutes, then cooled to room temperature, diluted with physiological saline or water for injection, and subjected to sterile filtration to obtain a labeled compound injection, designated as "⁶⁸Ga-formula IV."

Taking the labeling of the compound of formula IV-1 prepared in Example 1 as an example, the processes of the above two labeling methods are represented as follows:

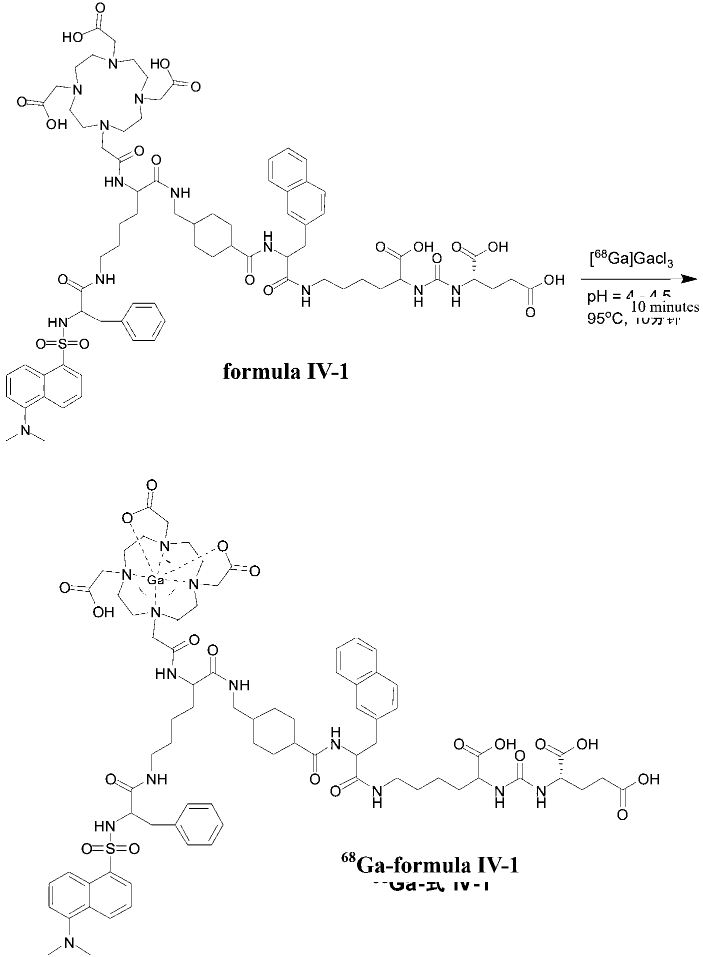

If the radiochemical purity is lower than 95%, purification is required. The purification steps are as follows: A Sep-Pak C18 cartridge was taken and activated by sequential elution with 10 mL of absolute ethanol and 10 mL of water. The labeling solution was diluted with 10 mL of water and then loaded onto the cartridge. The cartridge was rinsed with water to remove unreacted ⁶⁸Ga ions, and then eluted with an ethanol solution to obtain purified ⁶⁸Ga-IV. The organic solvent was removed by nitrogen blowing. Through dilution with physiological saline and then sterile filtration, the labeled compound injection was obtained.

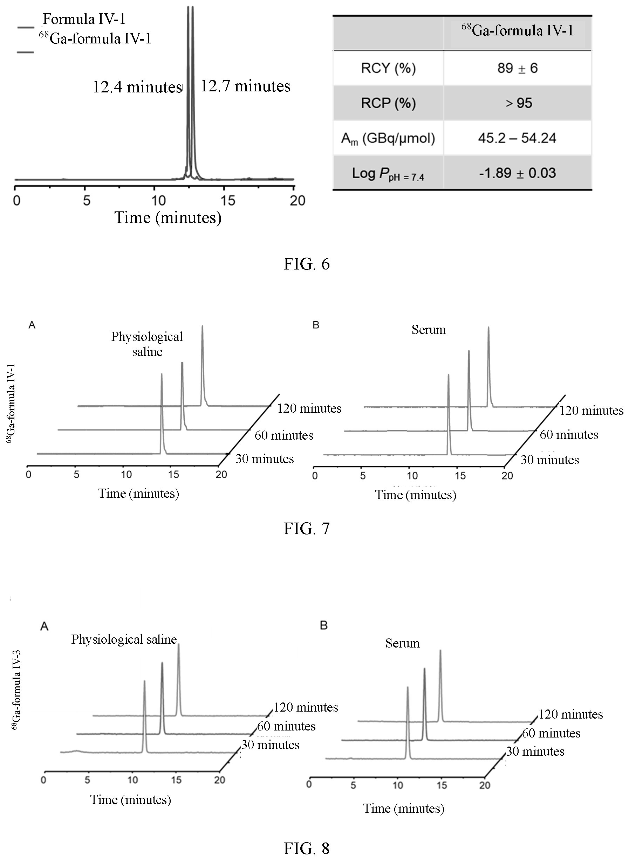

The compound of formula IV-1 of Example 1 was labeled according to the above method. The identification results are shown in FIG. 6. ⁶⁸Ga-formula IV-1 has a high labeling yield and specific activity. The radionuclide is easily available and the operation is simple, which is conducive to large-scale clinical application.

The present invention also employs ⁶⁸Ga to label other compounds, including but not limited to ⁶⁸Ga-formula IV-2, ⁶⁸Ga-formula IV-3, ⁶⁸Ga-formula IV-4, ⁶⁸Ga-formula IV-5, ⁶⁸Ga-formula IV-6, ⁶⁸Ga-formula IV-7, ⁶⁸Ga-formula IV-8, ⁶⁸Ga-formula IV-9, ⁶⁸Ga-formula IV-10, ⁶⁸Ga-formula IV-11, ⁶⁸Ga-formula IV-12, etc. Experiments demonstrate that the compounds provided by the present invention have high labeling yields and specific activities, and the radionuclide is easily available and the operation is simple, which is conducive to large-scale clinical application.

### Example 15. In vitro stability test

According to the labeling method of Example 14, the compounds IV-1, IV-3, and IV-9 prepared in Examples 1, 3, and 9 were labeled, and the resulting ⁶⁸Ga-labeled complexes were designated as "⁶⁸Ga-formula IV-1," "⁶⁸Ga-formula IV-3," and '⁶⁸Ga-formula IV-9," respectively. Solutions of ⁶⁸Ga-formula IV-1, ⁶⁸Ga-formula IV-3, and ⁶⁸Ga-formula IV-9 with a radiochemical purity greater than 95% were separately dissolved in physiological saline and allowed to stand at room temperature for different durations. Samples were taken and analyzed by HPLC. At the tested time points, each probe maintained a radiochemical purity of >95%, indicating that it is stable and not prone to decomposition in the specified solution. The HPLC identification results for the *in vitro* physiological saline stability of the solutions of ⁶⁸Ga-formula IV-1, ⁶⁸Ga-formula IV-3, and ⁶⁸Ga-formula IV-9 are shown in panel A of FIGS. 7-9, indicating that they maintain relatively high stability (>95%) in the physiological saline system for up to 2 h.

The above solutions of ⁶⁸Ga-formula IV-1, ⁶⁸Ga-formula IV-3, and ⁶⁸Ga-formula IV-9 with a radiochemical purity >95% were co-incubated with serum at room temperature for different durations. Acetonitrile was added to remove proteins, the mixture was centrifuged, and the supernatant solution was taken. Samples were taken and analyzed by HPLC. At the tested time points, each probe maintained a radiochemical purity of >95%, indicating that it is stable and not prone to decomposition in the specified solution. The HPLC identification results for the *in vitro* serum stability of ⁶⁸Ga-formula IV-1, ⁶⁸Ga-formula IV-3, and ⁶⁸Ga-formula IV-9 are shown in panel B of FIGS. 7-9, indicating that they maintain high stability (>95%) in both the physiological saline and serum systems for up to 2 h.

### Example 16. Preparation of a radionuclide ¹⁷⁷Lu-labeled complex

Wet method: About 18.5-1850 MBq of ¹⁷⁷LuCl₃ sodium acetate solution was added separately to centrifuge tubes containing the solutions of the compounds to be labeled (the solutions of the compounds to be labeled were 0.5-2 mL of acetic acid-acetate solutions containing 20-200 µg of each compound prepared in Example 1-Example 13). The mixture was placed at 90°C and reacted for 20 min, then cooled to room temperature, diluted with physiological saline or water for injection, and subjected to sterile filtration to obtain a labeled compound injection, designated as "¹⁷⁷Lu-formula IV."

Lyophilization method: A certain amount of buffer solution and about 37-3700 MBq of ¹⁷⁷LuCl₃ were added to lyophilized kits containing 20-200 µg of the compounds to be labeled (the compounds to be labeled were compounds provided by the present invention, including but not limited to each compound prepared in Example 1-Example 13). After mixing and dissolving, the mixture was placed at room temperature to 100°C and reacted for 20 minutes, then cooled to room temperature, diluted with physiological saline or water for injection, and subjected to sterile filtration to obtain a labeled compound injection, designated as "¹⁷⁷Lu-formula IV."

If the radiochemical purity is lower than 95%, purification is required. The purification steps are as follows: A Sep-Pak C18 cartridge was taken and activated by sequential elution with 10 mL of absolute ethanol and 10 mL of water. The labeling solution was diluted with 10 mL of water and then loaded onto the cartridge. The cartridge was rinsed with water to remove unreacted ¹⁷⁷Lu ions, and then eluted with an ethanol solution to obtain a purified ¹⁷⁷Lu-labeled complex. The organic solvent was removed by nitrogen blowing. Through dilution with physiological saline and then sterile filtration, the labeled compound injection was obtained.

The present invention also employs ¹⁷⁷Lu to label other compounds, including but not limited to ¹⁷⁷Lu-formula IV-1, ¹⁷⁷Lu-formula IV-2, ¹⁷⁷Lu-formula IV-3, ¹⁷⁷Lu-formula IV-4, ¹⁷⁷Lu-formula IV-5, ¹⁷⁷Lu-formula IV-6, ¹⁷⁷Lu-formula IV-7, ¹⁷⁷Lu-formula IV-8, ¹⁷⁷Lu-formula IV-9, ¹⁷⁷Lu-formula IV-10, ¹⁷⁷Lu-formula IV-11, ¹⁷⁷Lu-formula IV-12, etc.

The compounds provided by the present invention can also be labeled with reference to other labeling methods involved in the prior art, such as ¹⁸F-formula IV-13, ¹⁸F-formula IV-14, ¹⁸F-formula IV-15, ¹⁸F-formula IV-16, ¹⁸F-formula IV-17, ¹⁸F-formula IV-18, ¹⁸F-formula IV-19, ¹⁸F-formula IV-20, ¹⁸F-formula IV-21, ¹⁸F-formula IV-22, ¹⁸F-formula IV-23, ¹⁸F-formula IV-24, etc.

In addition, the radionuclide-labelable compounds provided by the present invention can be labeled with reference to other labeling methods provided by the prior art (including but not limited to the methods provided by the present invention). The radionuclide includes but is not limited to: ¹⁸F , ⁵¹Cr, ⁶⁴Cu, ⁶⁷Cu, ⁶⁷Ga , ⁶⁸Ga, ⁸⁹Zr, ¹¹¹In, ⁹⁹mTc, ¹⁸⁶Re, ¹⁸⁸Re, ¹³⁹La, ¹⁴⁰La, ¹⁷⁵Yb , ¹⁵³Sm, ¹⁶⁶Ho, ⁸⁶Y ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ¹⁰¹mRh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ¹⁹⁸Au, ²²⁵Ac, ²²⁷Th, or ¹⁹⁹Ag, etc.

### Example 17. Cellular uptake experiment

Tumor cellular uptake experiment: One day before the start of the experiment, 2×10⁵ PC3-PIP cells were added to each well of a 24-well plate and cultured overnight. The cells were divided into three groups: a total binding group, an internalization group, and an inhibition group, with four wells in each group. A 100-fold excess of PSMA617 was first added to the well plate of the inhibition group as an inhibitor. Then, 500 µL of a ⁶⁸Ga-formula IV-1 solution (18.5 kBq/well) with a radiochemical purity greater than 95%, prepared according to the method of Example 14 and diluted in serum-free medium, was added separately to all well plates. The plates were covered and incubated in a cell culture incubator at 37°C. For the total binding group and the internalization group, the cells were removed from the incubator at 10, 30, 60, and 120 min after incubation. The cells were rinsed 3 times with PBS, and the internalization group was washed once more with an eluent. The cells were then digested with 1 mL of digestion solution. For the inhibition group, after incubation at 37°C for 10, 30, 60, and 120 min, the cells were washed twice with ice-cold PBS (pH 7.4) and then digested with the digestion solution. All solutions and cells in the well plates were aspirated and placed into labeled small tubes. Three aliquots of 500 µL of the ⁶⁸Ga-formula IV-1 solution diluted in serum-free medium were taken. The total radioactive dose added to each well was calculated, and radioactivity counts were measured using a γ-counter to calculate the cell binding rate and internalization rate at each time point.

Cell receptor saturation competitive binding experiment: According to the results of the cellular uptake experiment, cells with a high cell binding rate were selected as cells for determining receptor affinity. These cells were used to screen the affinity and specificity of the labeled probe to guide and optimize probe design. In brief: Before the start of the experiment, 50 µL of serum-free medium containing PC3-PIP cells (2×10⁶/mL) was added to each well of a 96-well filter plate. Radionuclide-labeled compounds to be tested were added at increasing concentrations (18 kBq/well), and the final reaction volume in all wells was adjusted to 200 µL. The plate was placed on a shaker and shaken at room temperature for 4 h. The reaction solution was then filtered to dryness and air-dried. The filter membranes were collected, and radioactivity counts were measured using a γ-counter. K_{d} values were calculated using Graph-Pad Prism software.

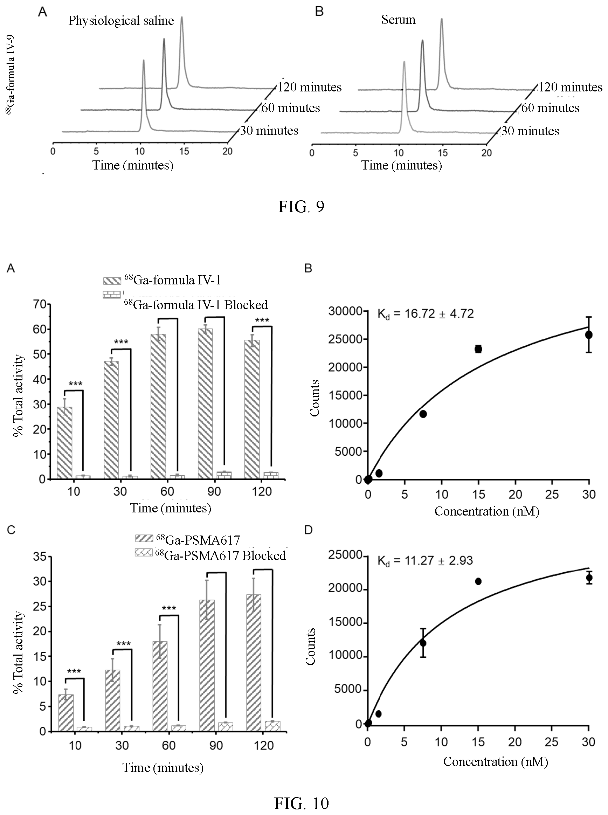

The experimental results are shown in FIG. 10. The K_{d} value of ⁶⁸Ga-formula IV-1 was comparable to that of ⁶⁸Ga-PSMA617 (see curves in panels B and D), but the cellular uptake of ⁶⁸Ga-formula IV-1 within 2 hours was significantly higher than that of ⁶⁸Ga-PSMA617 (see histograms in panels A and C).

### Example 18. MicroPET imaging of ⁶⁸Ga-formula IV-1 in tumor model mice

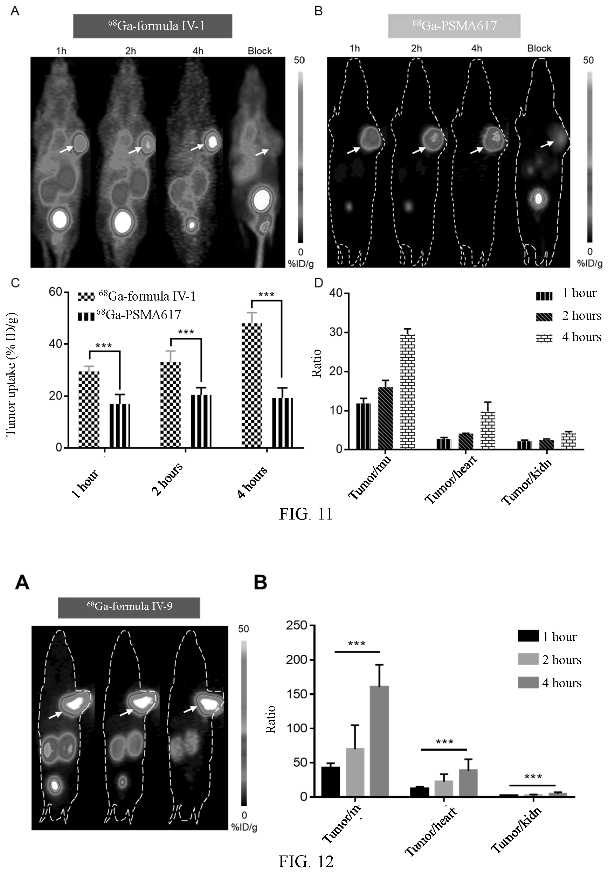

⁶⁸Ga-formula IV-1 with a radiochemical purity greater than 95% was prepared according to the labeling method of Example 14. PC3-PIP tumor-bearing mice were randomly divided into an experimental group and a control group. 0.2 mL (approximately 7.4 MBq) of ⁶⁸Ga-formula IV-1 solution was taken and injected into the mice of the experimental group via the tail vein, while mice in the control group were injected with ⁶⁸Ga-PSMA 617. MicroPET imaging was performed at time points of 1 h, 2 h, and 4 h after injection. The imaging results are shown in FIG. 11. The region indicated by the arrow in panel A is the tumor location. The tumor uptake of ⁶⁸Ga-PSMA 617 injected in the control group was approximately 20% ID/g at 4 h after injection (see the image in panel B and the vertical striped bar chart in panel C of FIG. 11), whereas the tumor uptake of ⁶⁸Ga-formula IV-1 of the present invention was approximately 50% ID/g at 4 h after injection, which was far higher than that of the control group. This indicates that the ⁶⁸Ga-formula IV-1 complex of the present invention has significantly enhanced tumor uptake and retention time (see the image in panel A and the grid-patterned bar chart in panel C of FIG. 11). At the same time, ⁶⁸Ga-formula IV-1 could obtain relatively high target-to-non-target ratios (tumor/muscle ratio, tumor/heart ratio, and tumor/kidney ratio) within 1 h, and the target-to-non-target ratios gradually increased with the extension of time (see the bar chart in panel D of FIG. 11).

In summary, on the one hand, ⁶⁸Ga-formula IV-1 of the present invention is helpful for longer-time imaging; under the guarantee of high imaging contrast, the diagnosis of primary micro-lesions will be more accurate, which is conducive to the delineation of the target volume; on the other hand, the high uptake and long retention of the nuclide-labeled probe at the tumor site will lay a foundation for nuclide-targeted therapy.

### Example 19. Use of a ⁶⁸Ga-formula IV-9 complex in MicroPET imaging of tumor model mice

A ⁶⁸Ga-formula IV-9 solution with a radiochemical purity greater than 95% was prepared according to the labeling method of Example 14. PC3-PIP tumor-bearing mice were randomly divided into an experimental group and a control group. 0.2 mL (approximately 7.4 MBq) of ⁶⁸Ga-formula IV-9 solution was taken and injected into the mice of the experimental group via the tail vein, while mice in the control group were injected with ⁶⁸Ga-PSMA 617. MicroPET imaging was performed at time points of 1 h, 2 h, and 4 h after injection. The imaging results are shown in FIG. 12. The region indicated by the arrow in panel A is the tumor location. The tumor uptake of ⁶⁸Ga-PSMA 617 injected in the control group was approximately 20% ID/g at 4 h after injection (see parts B and C of FIG. 11), whereas the tumor uptake of the ⁶⁸Ga-formula IV-9 complex of the present invention was approximately 50% ID/g at 4 h after injection, which was far higher than that of the control group. At the same time, ⁶⁸Ga-formula IV-9 could obtain relatively high target-to-non-target ratios (tumor/muscle ratio, tumor/heart ratio, and tumor/kidney ratio) within 1 h, and the target-to-non-target ratios gradually increased with the extension of time (see the bar chart in panel B of FIG. 12).

### Example 20. Biodistribution of a ¹⁷⁷Lu-labeled complex in normal mice

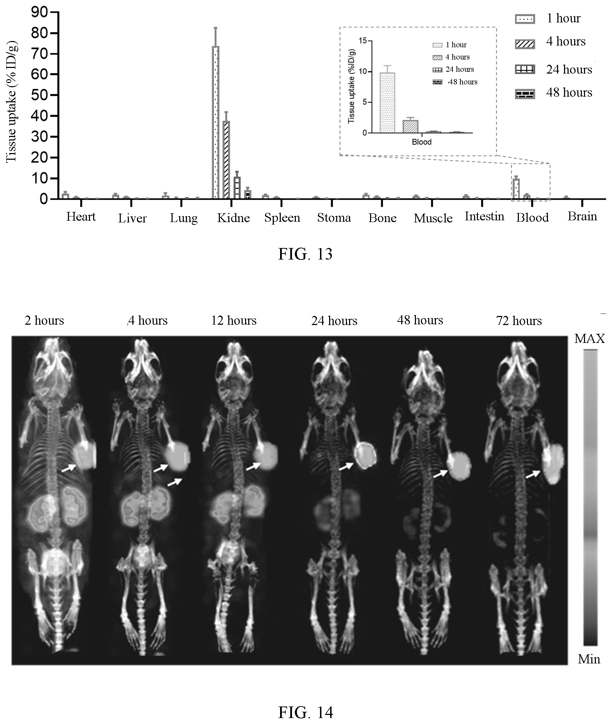

Compound IV-1 prepared in Example 1 was labeled according to the labeling method of Example 16 to obtain ¹⁷⁷Lu-formula IV-1 with a radiochemical purity greater than 95%. 0.2 mL (approximately 1.85 MBq) was taken and injected via the tail vein of normal mice. The mice were sacrificed at time points of 4 h, 12 h, 24 h, and 48 h after injection, and the organs of interest were then collected and measured through a γ-counter. As shown in FIG. 13, the blood uptake value of ¹⁷⁷Lu-formula IV-1 remained at a high level at 4 hours, and the radioactivity was gradually cleared from the blood with the extension of time. This indicates that the ¹⁷⁷Lu-labeled IV-1 of the present invention has the ability to moderately enhance blood circulation time, which is beneficial for enhancing tumor uptake while avoiding the problem of excessively long circulation of the drug in the body.

### Example 21. SPECT imaging of ¹⁷⁷Lu-formula IV-1 in tumor model mice

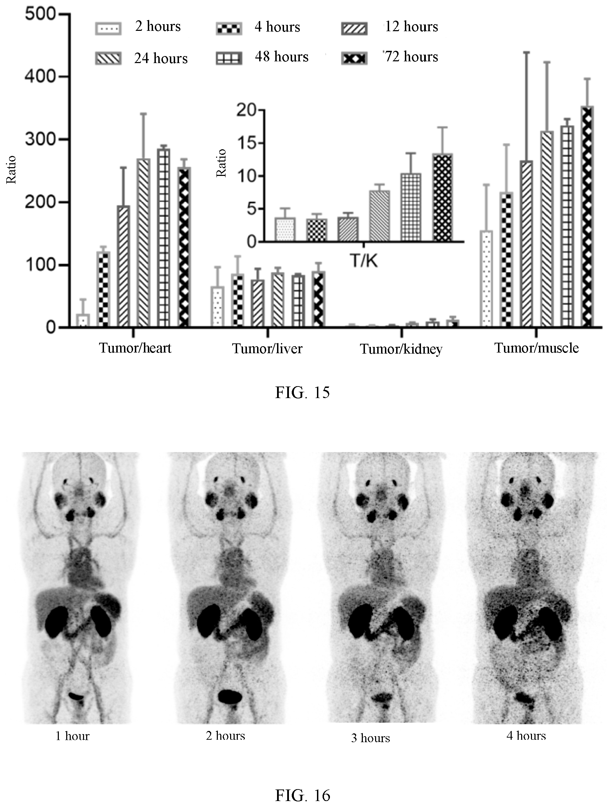

A ¹⁷⁷Lu-formula IV-1 solution with a radiochemical purity greater than 95% was prepared according to Example 16. 0.2 mL (approximately 37 MBq) was taken and injected via the tail vein of PC3-PIP tumor-bearing mice. SPECT imaging was performed at time points of 2, 4, 12, 24, 48, and 72 hours after injection. The imaging results are shown in FIG. 14. The position indicated by the arrow in FIG. 14 is the tumor location. The imaging results indicate that the ¹⁷⁷Lu-formula IV-1 of the present invention exhibits significantly enhanced tumor uptake and retention time at the tumor site. The high uptake and long retention of the nuclide-labeled probe at the tumor site will lay a foundation for nuclide-targeted therapy. The tissue uptake results after administration can be seen from FIG. 15. Relatively high target-to-non-target ratios (tumor/muscle ratio, tumor/heart ratio, and tumor/kidney ratio) could be obtained within 2 h, and the target-to-non-target ratios gradually increased with the extension of time.

### Example 22. Use of a ⁶⁸Ga-labeled complex in MicroPET imaging of prostate cancer patients

### Subject selection criteria:

1) Patients highly suspected of having prostate cancer according to clinical symptoms, laboratory tests, and other imaging examinations (CT, MRI);
2) Patients aged 18-80 years; and those with a pathological diagnosis obtainable via surgery or biopsy; and
3) Excluding patients with renal failure (serum Cr > 3 mg/dl); and excluding patients who are too seriously ill to undergo further diagnosis and treatment.

A ⁶⁸Ga-formula IV-1 solution and a ⁶⁸Ga-formula IV-9 solution with a radiochemical purity greater than 95% were prepared according to the labeling method of Example 14 and administered to the subjects via intravenous injection. The intravenous radiotracer dose was 1.85-2.22 MBq/kg. PET/CT imaging was required to be performed at 60 min, 120 min, 180 min, and 240 min after injection using an integrated PET/CT scanner (Polestar m660, Sino Union, China). Low-dose CT scans (120 kv, 35 mAs) from the skull vertex to the mid-thigh were used for attenuation correction and anatomical reference. Whole-body PET/CT coverage was performed over 5 to 6 bed positions (2 min/bed position). The acquired images were reconstructed using an ordered subset expectation maximization algorithm.

All images were transferred to MIM software (Version 7.1.4, MIM Software Inc., Cleveland, USA), and regions of interest (ROI) were drawn on the CT images using PET edges. Positive lesions were defined as areas with significantly increased tracer uptake compared with surrounding normal prostate tissue. The radioactive concentration was quantitatively evaluated using standard uptake values (SUV).

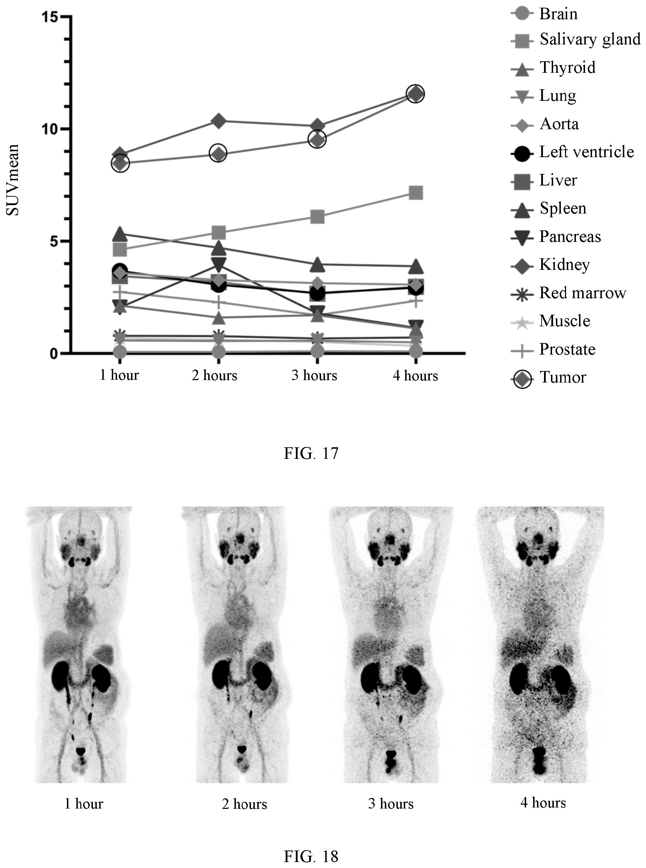

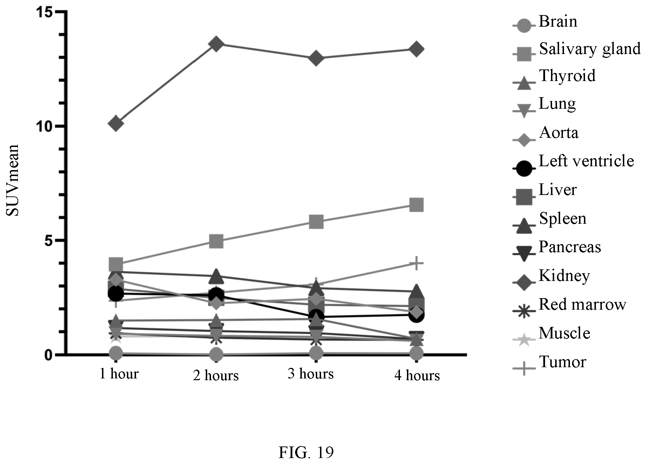

As shown in FIGS. 16-19, PET/CT images at 1 h, 2 h, 3 h, and 4 h after injection of the ⁶⁸Ga-formula IV-1 solution and the ⁶⁸Ga-formula IV-9 solution were analyzed, and the SUVmean of whole-body organs and SUVmax of tumors were measured. Imaging results for ⁶⁸Ga-formula IV-1 are shown in FIGS. 16 and 17, and imaging results for ⁶⁸Ga-formula IV-9 are shown in FIGS. 18-19. According to the imaging results, with the extension of time, the radioactive concentration of the two complexes of the present invention in the cardiac blood pool and blood-rich organs such as the liver and spleen gradually decreased, while gradually increasing in tumors, reaching the highest uptake value after 4 h with a high target-to-background ratio. This indicates that the ⁶⁸Ga-labeled complex of the present invention not only possesses high diagnostic efficacy but can also exert advantages in applications combining therapeutic nuclides with long half-lives.

## Claims

1. A dansylamide-modified PSMA-targeting compound, having a structure thereof as represented by formula A or formula I below: 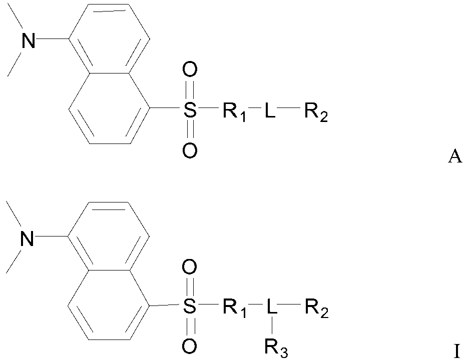 wherein:
L is -(X)ₙ-(CH₂)ₘ-(Y)_{q}-, wherein n is an integer from 0 to 10, X and Y are independently selected from lysine, glutamic acid, or a derivative structure comprising a lysine or glutamic acid structure, m is an integer from 0 to 20, and q is an integer from 0 to 6, and wherein each CH₂ may be individually replaced with -OCH₂-, -NH(CO)-, or -(CO)-NH-;
R₁ is of an amino acid analog structure having a structure of 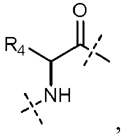 comprising an L-amino acid, a D-amino acid, or a derivative thereof, wherein R₄ is selected from: (CH₂)ᵣ-aryl, (CH₂)ᵣ-heteroaryl, (CH₂)ᵣ-COOH, (CH₂)ᵣ-NH₂, (CH₂)ᵣ-OH, (CH₂)ᵣ-SH, (CH₂)ᵣ-naphthylmethyl, (CH₂)ᵣ-indolyl, or (CH₂)ᵣ-benzothiazolyl, and wherein r is an integer from 0 to 10;
R₂ is of a structure targeting a prostate-specific membrane antigen; and
R₃ is a nuclide chelating group selected from any one of the following structures:

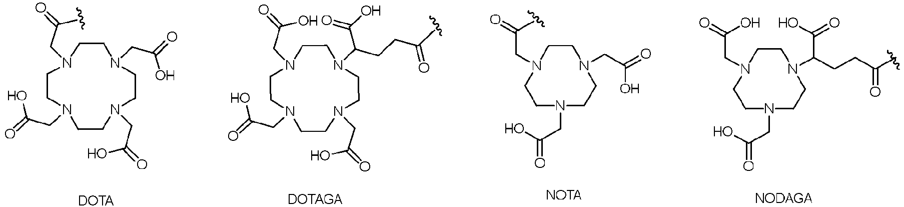

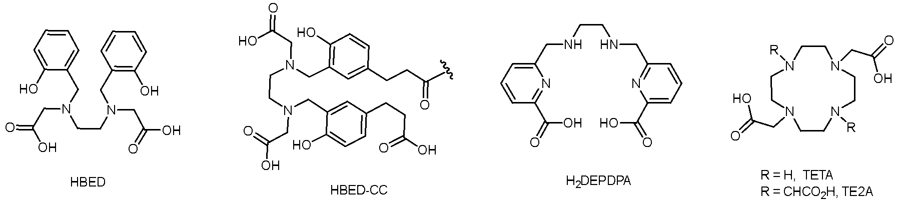

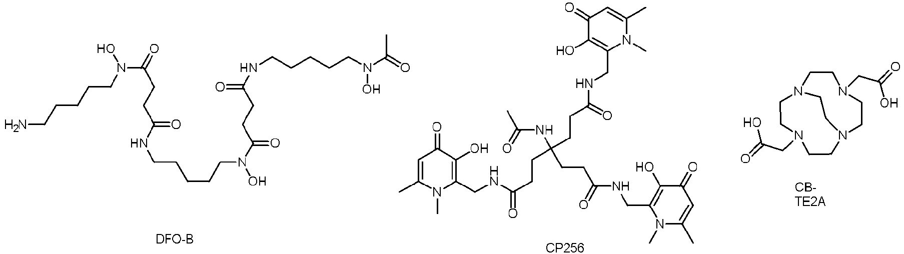

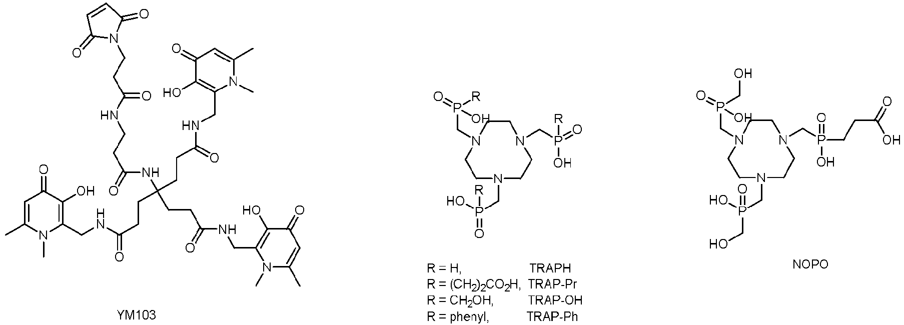

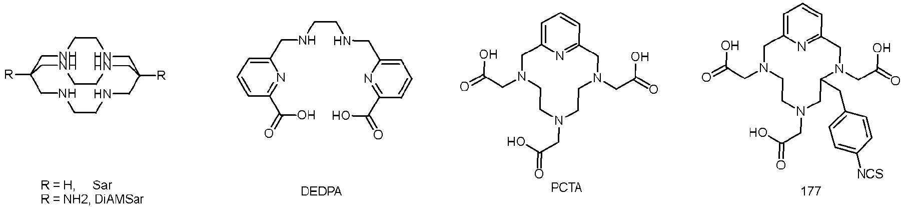

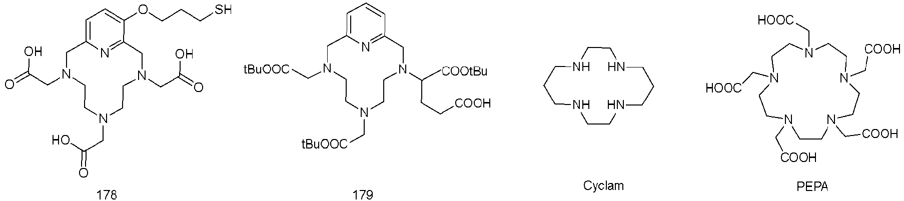

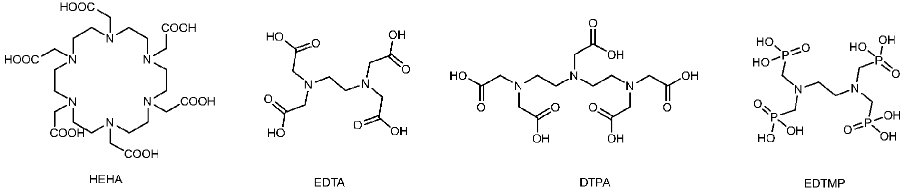

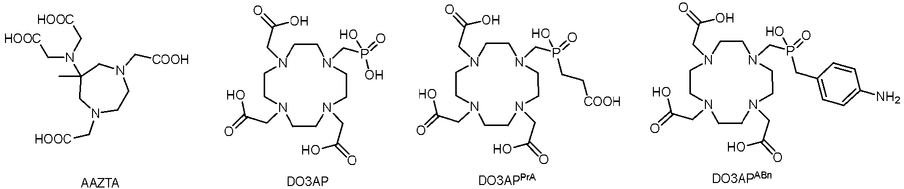

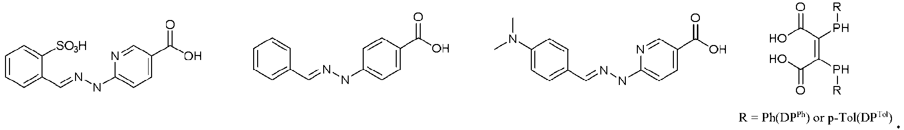

2. The dansylamide-modified PSMA-targeting compound according to claim 1, wherein R₁ is of the following structure: 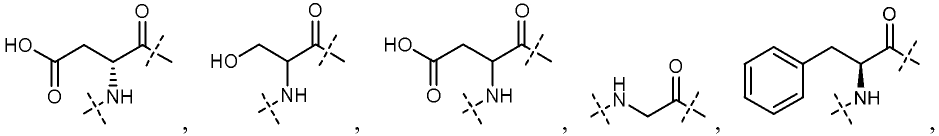 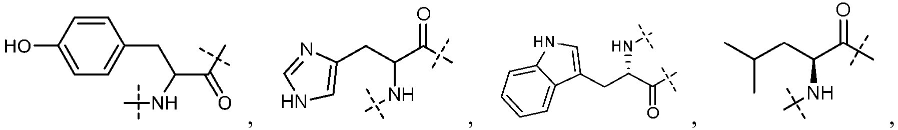 or

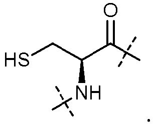

3. The dansylamide-modified PSMA-targeting compound according to claim 1, wherein R₂ in formula I is of a prostate-specific membrane antigen-targeting structure represented by formula II below: 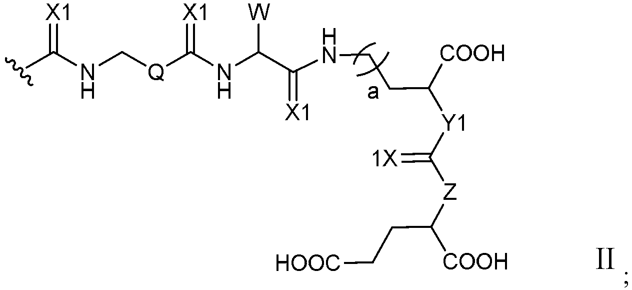 wherein:
X1 structures at different positions are the same or different and are independently selected from O, N, S, or P;
Y1 is O, S, or NH;
Z is O, S, or NH;
a is an integer from 0 to 20; Q is selected from substituted or unsubstituted alkyl, substituted or unsubstituted alkylaryl, or substituted or unsubstituted cycloalkyl; and
W is selected from -(CH₂)_{b}-aryl or -(CH₂)_{b}-heteroaryl, wherein b is an integer from 0 to 10.

4. The dansylamide-modified PSMA-targeting compound according to claim 3, wherein R₂ is any one of the following structures:

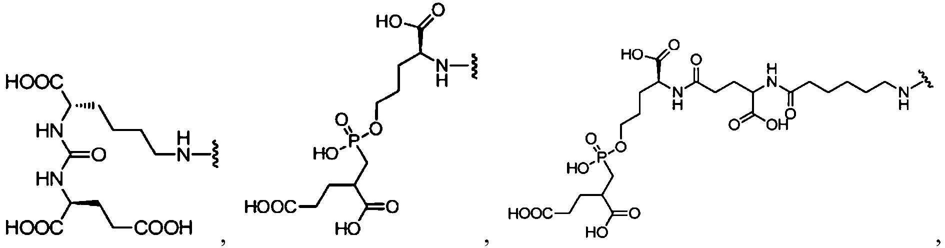

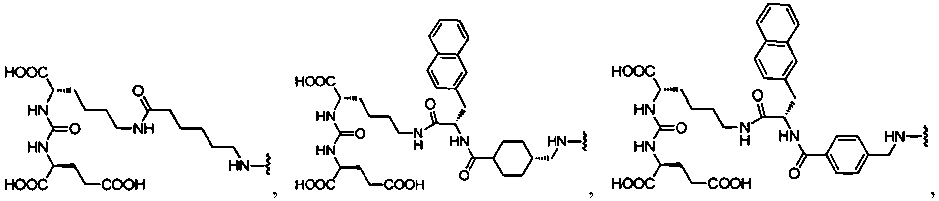

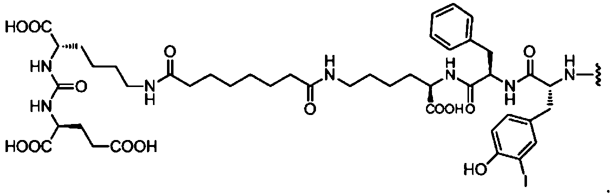

5. The dansylamide-modified PSMA-targeting compound according to claim 3, wherein Q in formula II is selected from substituted or unsubstituted C₅-C₁₄ aryl, substituted or unsubstituted C₅-C₁₄-alkylaryl, or substituted or unsubstituted C₅-C₁₄ cycloalkyl.

6. The dansylamide-modified PSMA-targeting compound according to any one of claims 1, 3, or 4, wherein:
R₂ is 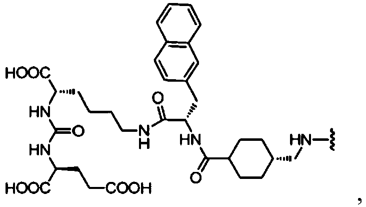 and R₃ is 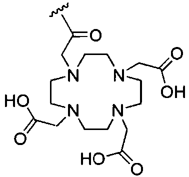 or 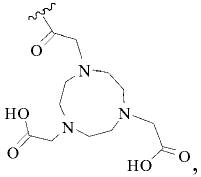
that is, a structure of a compound of formula A is as represented by formula B; and a compound of formula I is as represented by formula III or formula III-1: 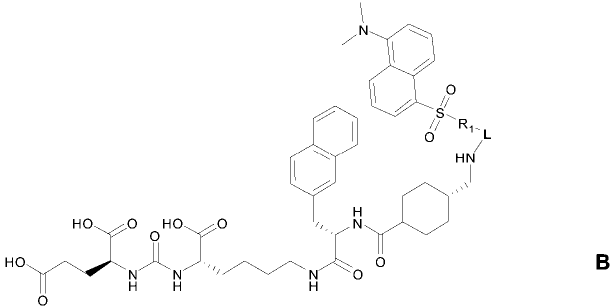 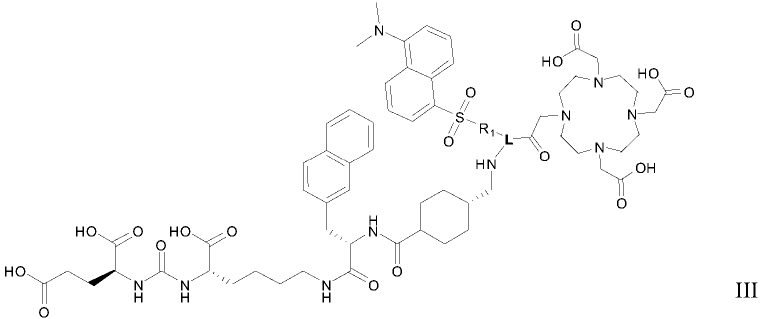 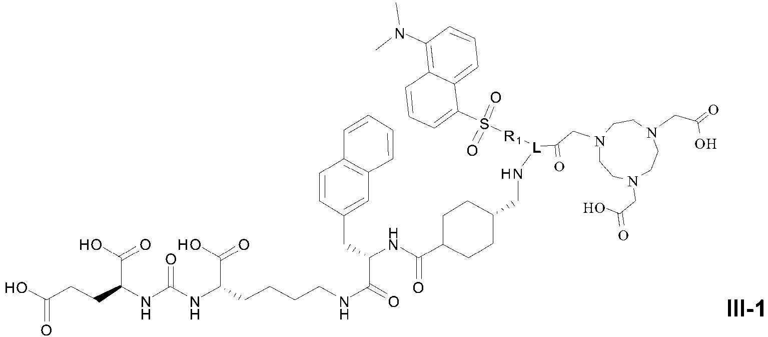
wherein L is selected from:
Lys, -Lys-(CO)-CH₂-(CO)-NH-CH₂-(CO), -Lys-(CO)-CH₂-(OCH₂CH₂)-O-CH₂(CO)-N H-CH₂-(CO), -Lys-(CO)-CH₂-(OCH₂CH₂)₃-O-CH₂(CO)-NH-CH₂-(CO), -(CO)-CH₂-(CO)-L ys-, -(CO)-(OCH₂CH₂)-O-CH₂(CO)-Lys-, or -(CO)-CH₂-(OCH2CH2)₃-O-CH₂(CO)-Lys-.

7. The dansylamide-modified PSMA-targeting compound according to any one of claims 1, 3, or 4, wherein:
R₂ is 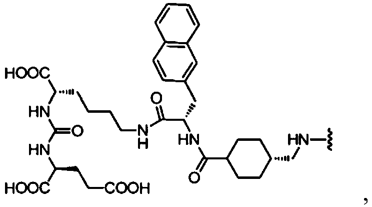 R₃ is 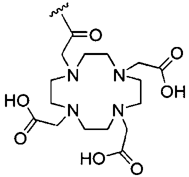 or 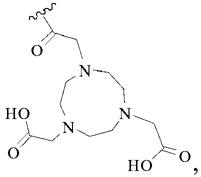 and L is of a Lys structure, that is, a structure of a compound of formula A is as represented by formula C, and a compound of formula I is as represented by formula IV or formula IV':

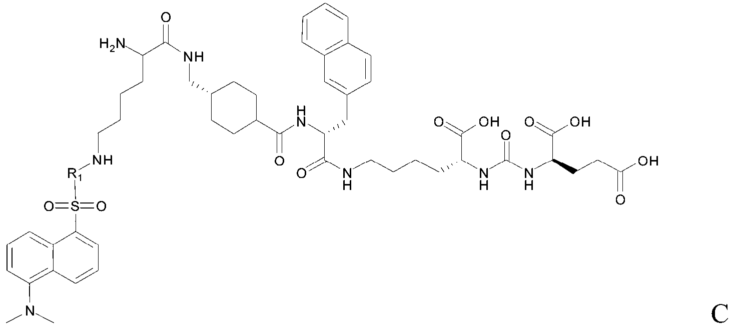

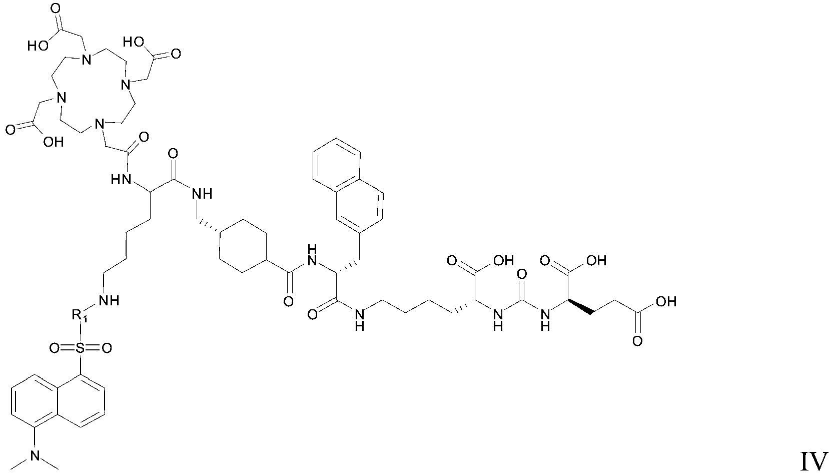

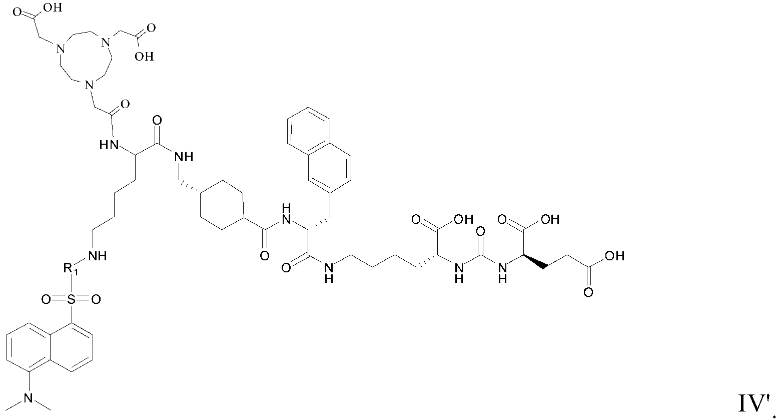

8. The dansylamide-modified PSMA-targeting compound according to claim 1, wherein a compound of formula A is any one of those represented by formulas A-1 to A-12 below: 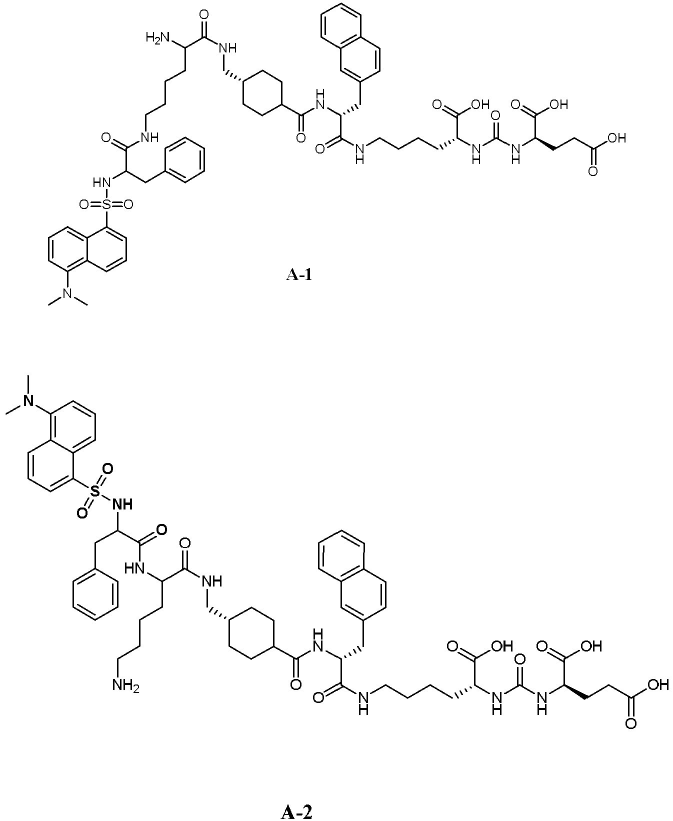 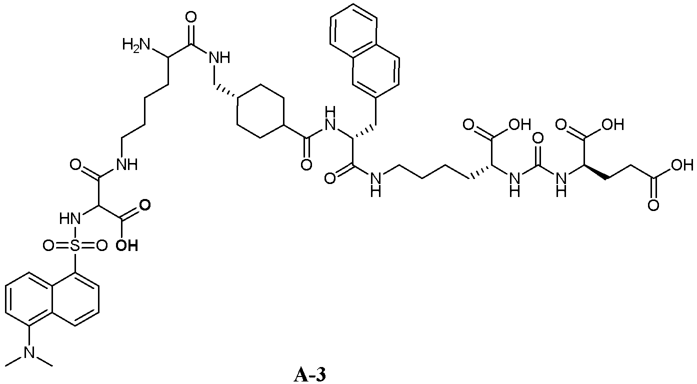 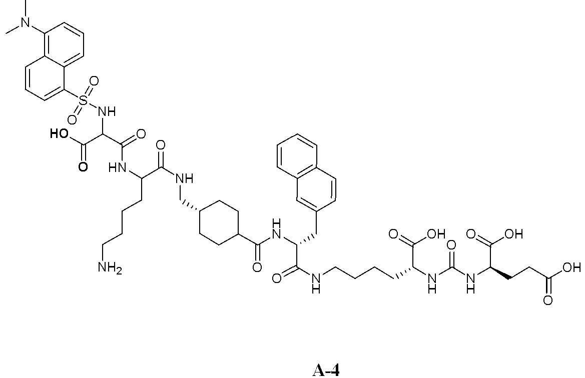 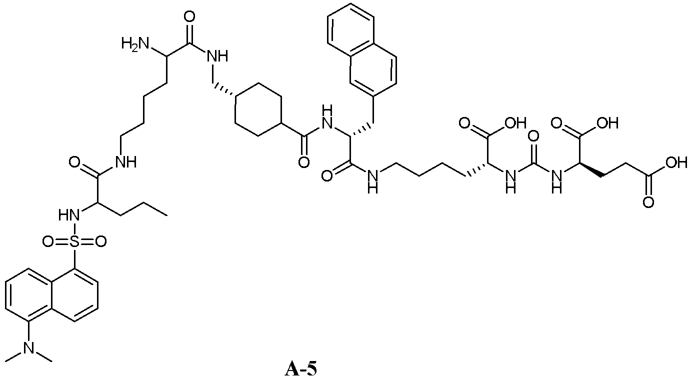 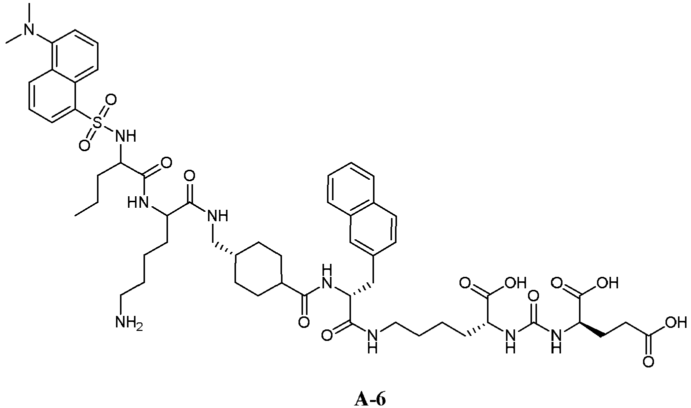 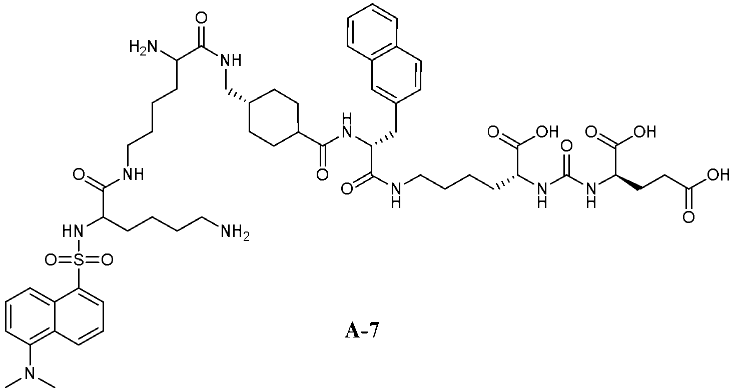 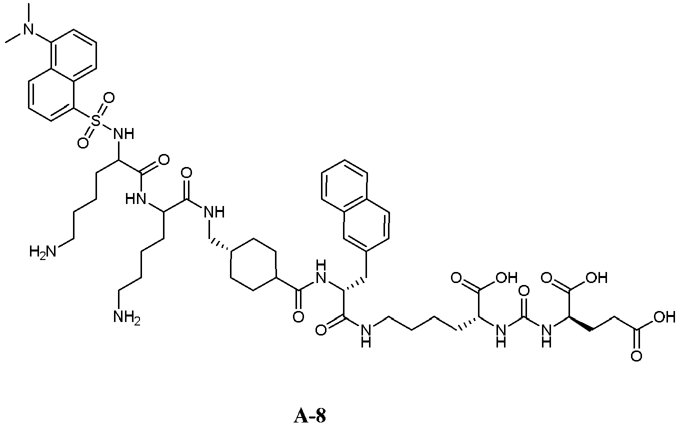 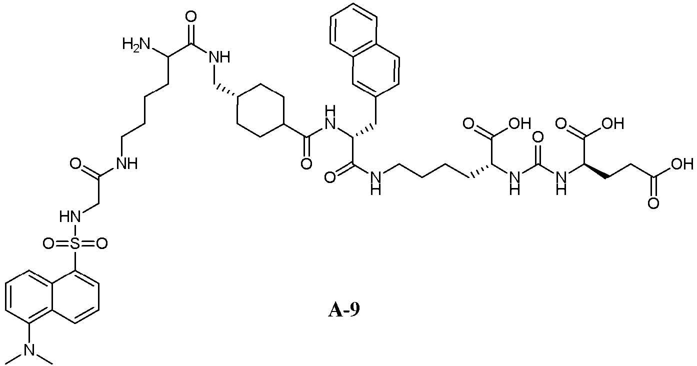 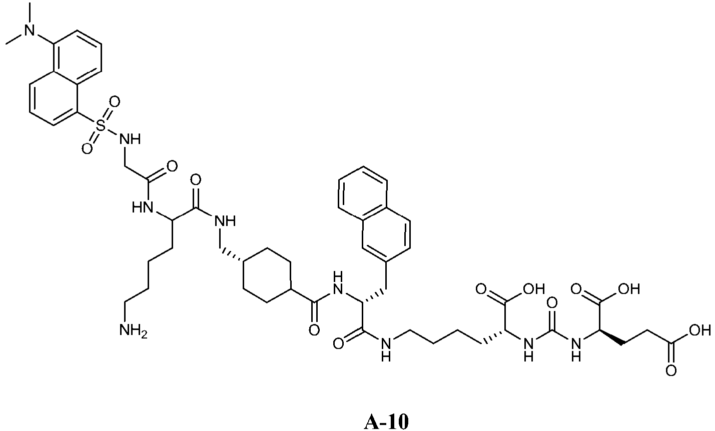 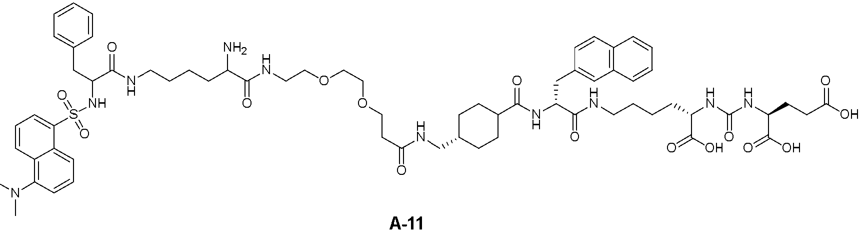 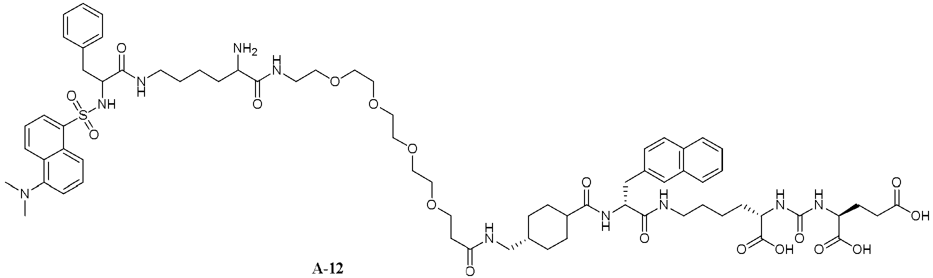 a compound of formula I is any one of those represented by formulas IV-1 to IV-24 below: 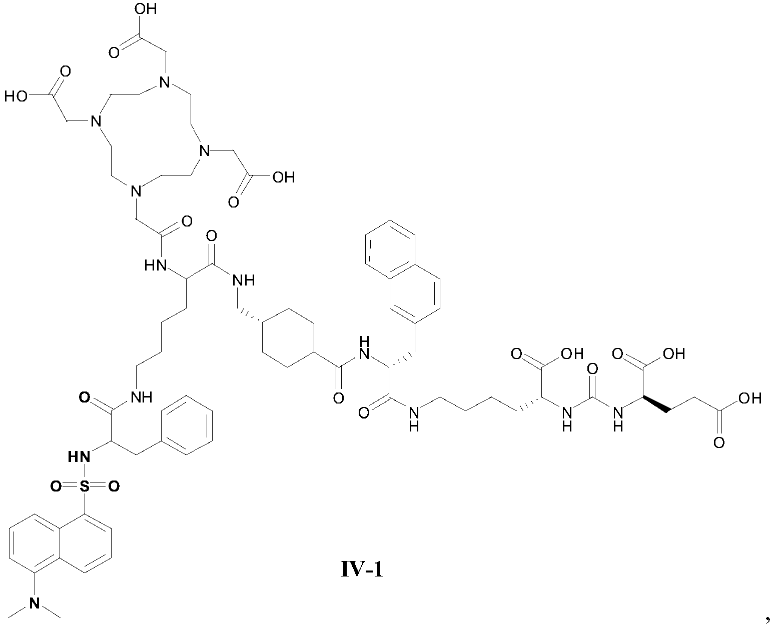 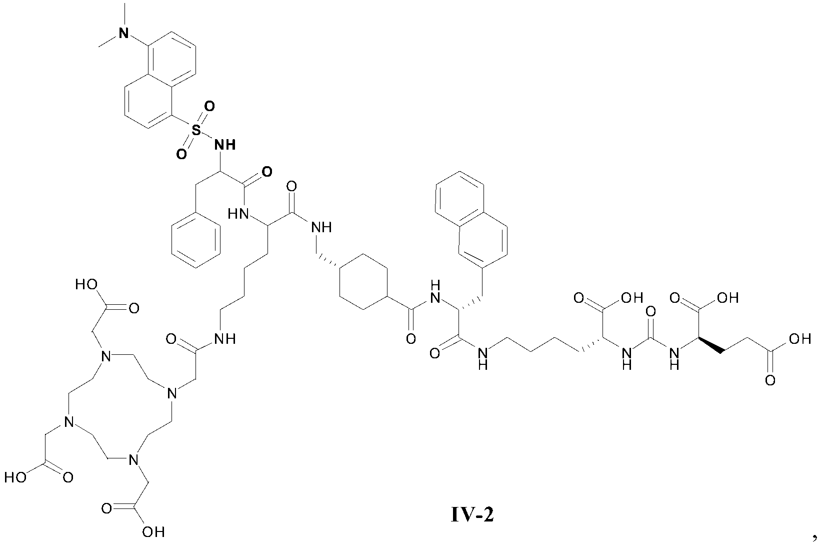 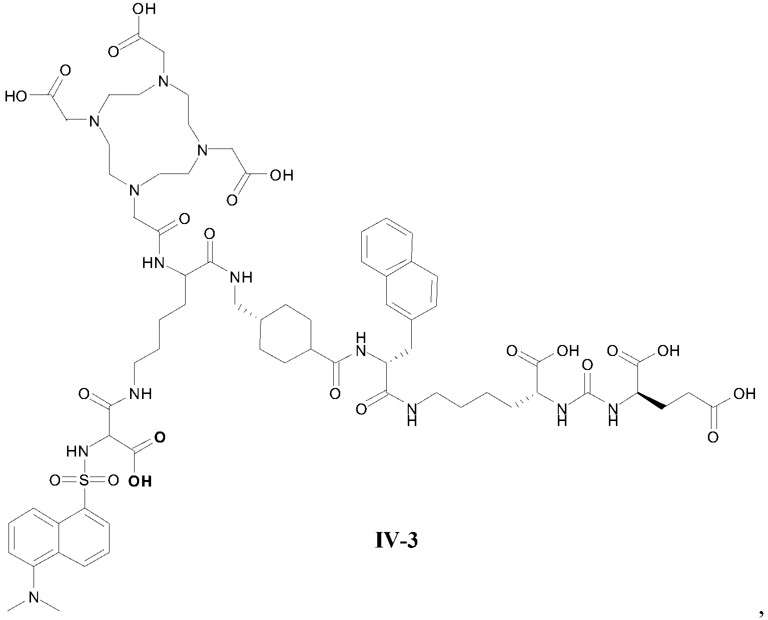 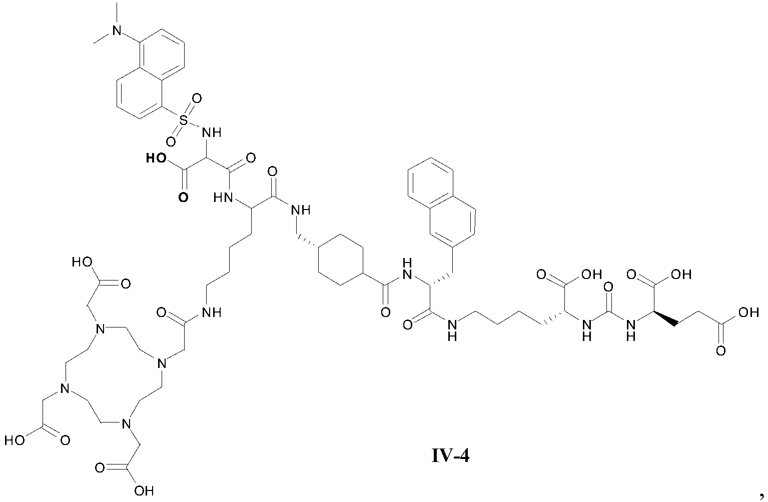 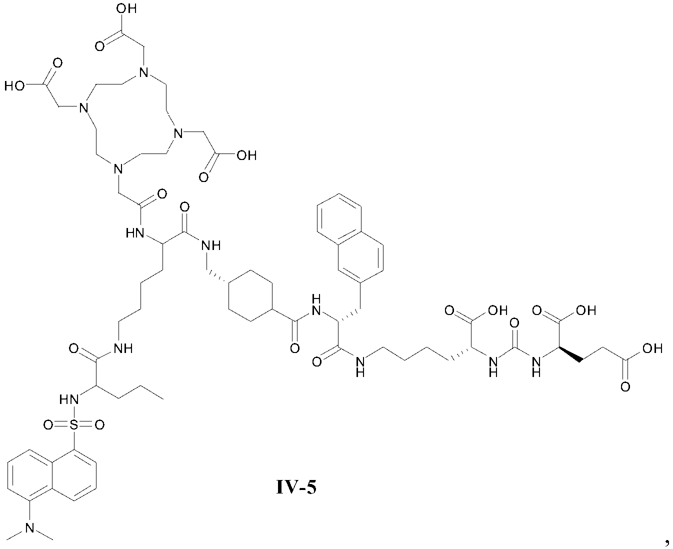 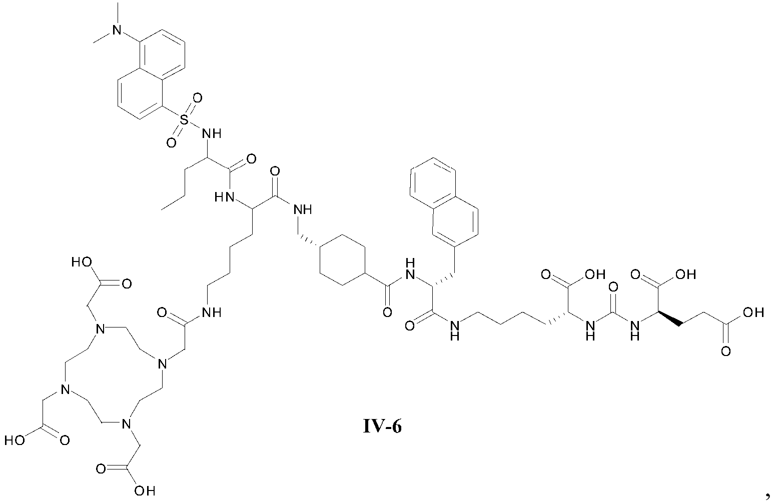 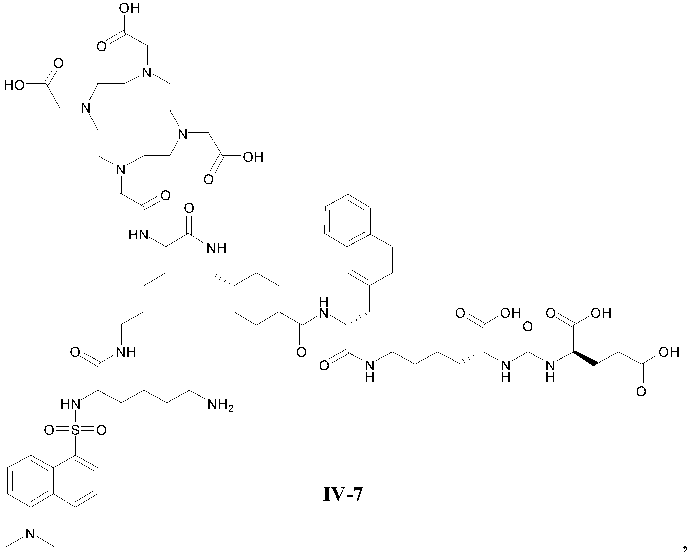 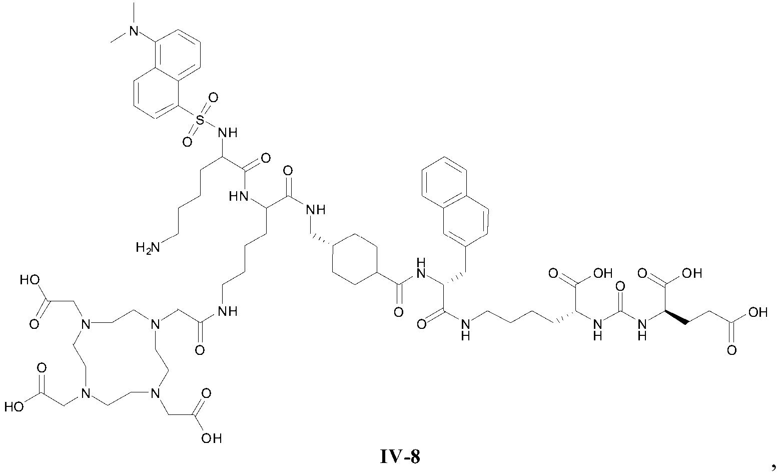 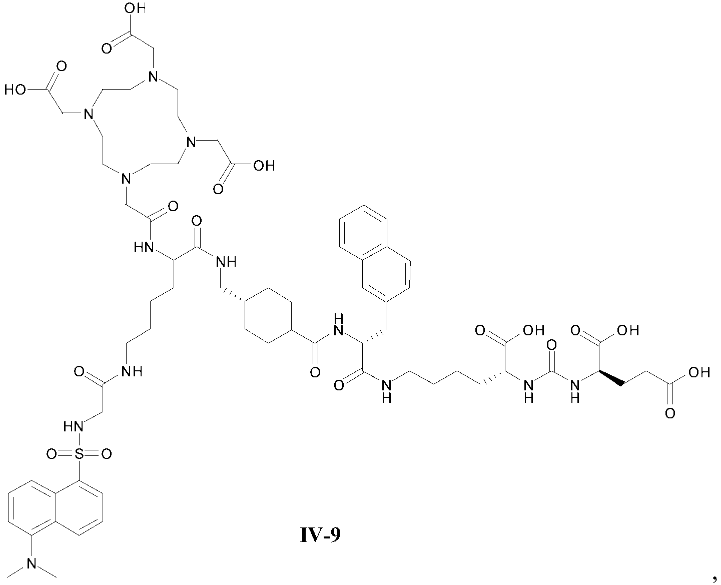 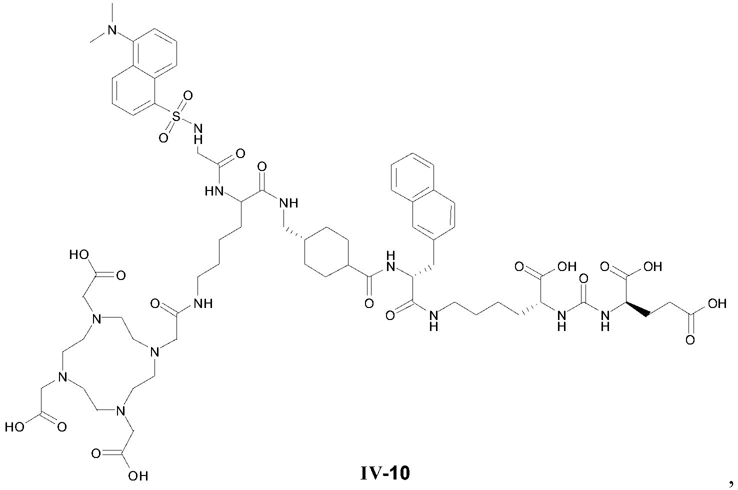 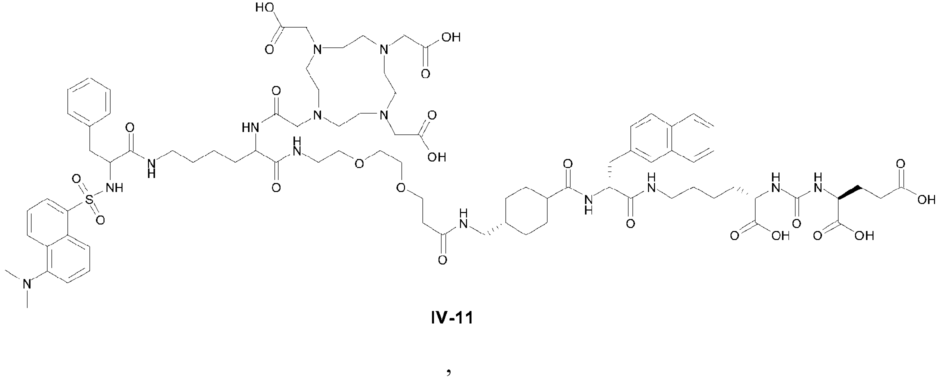 or

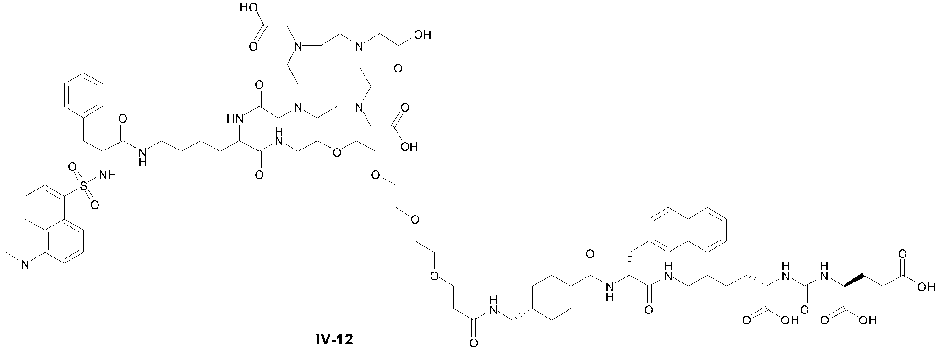

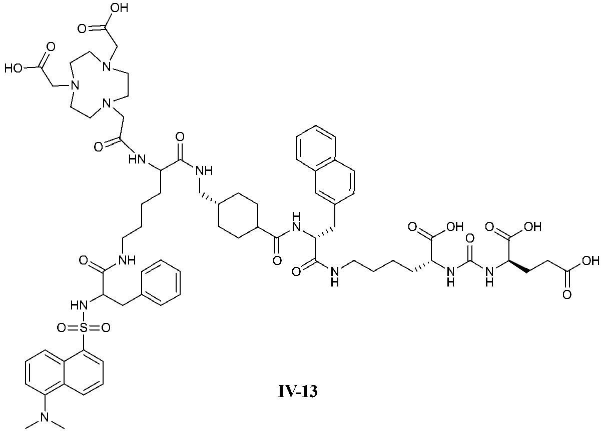

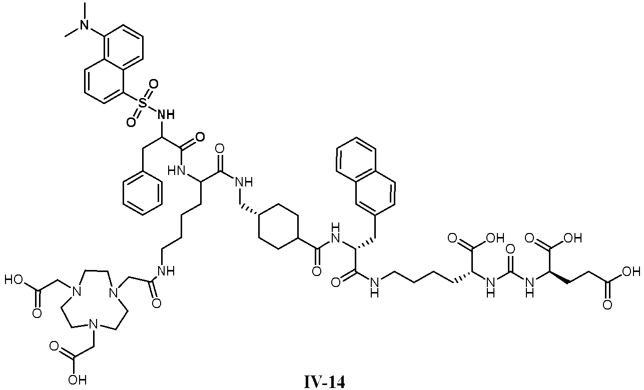

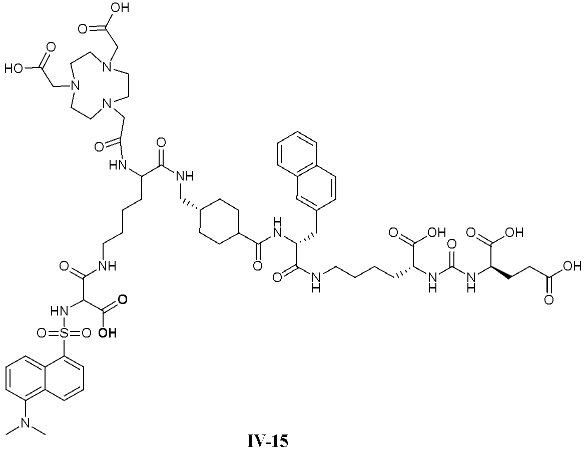

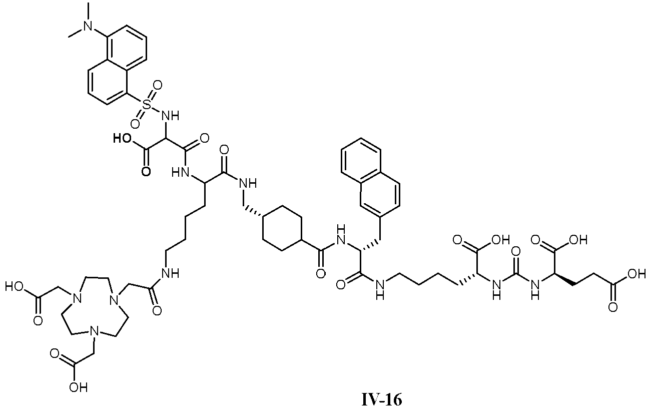

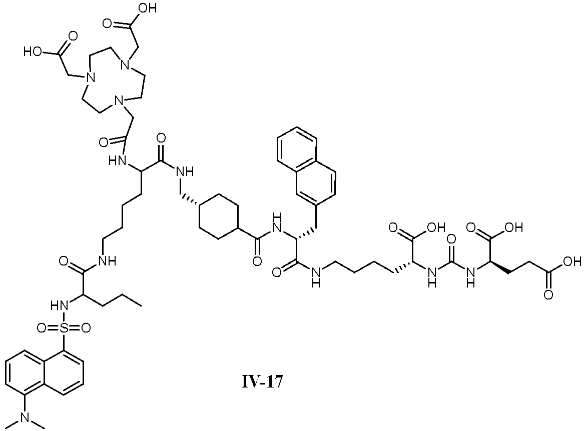

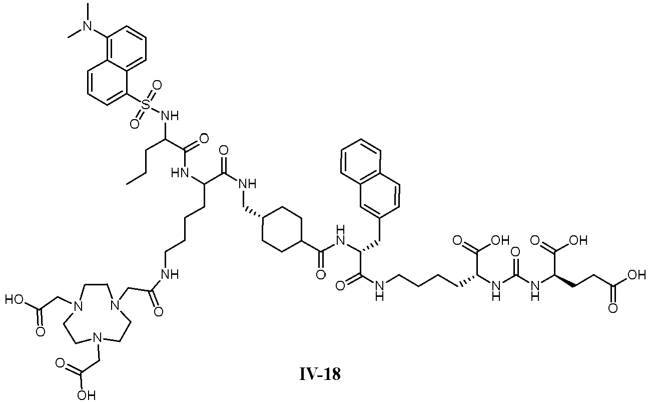

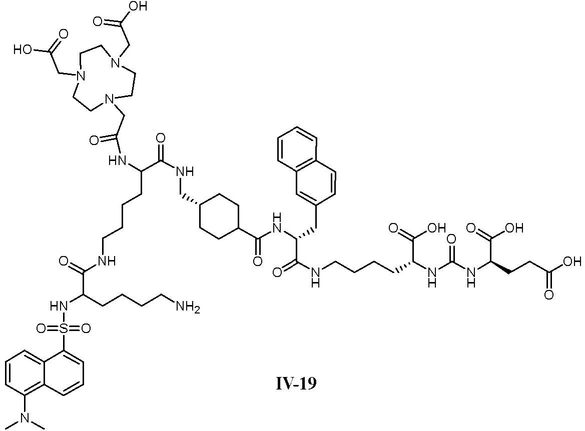

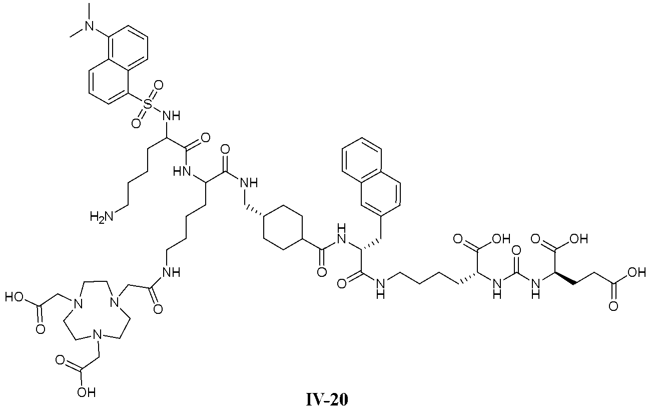

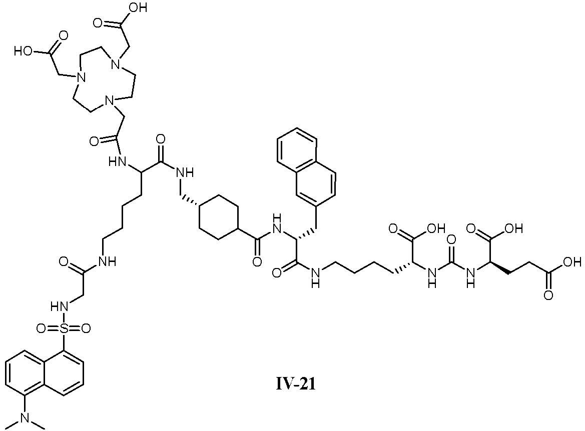

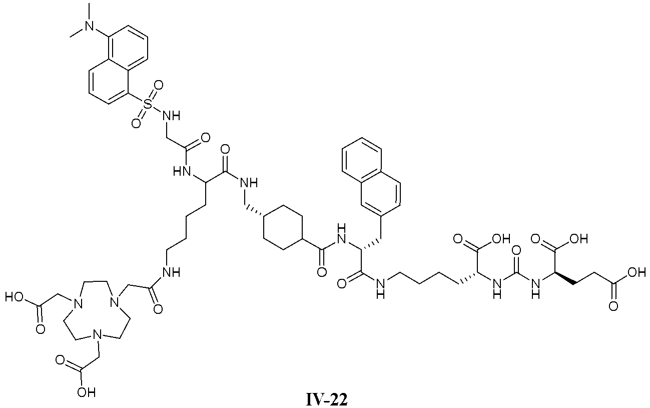

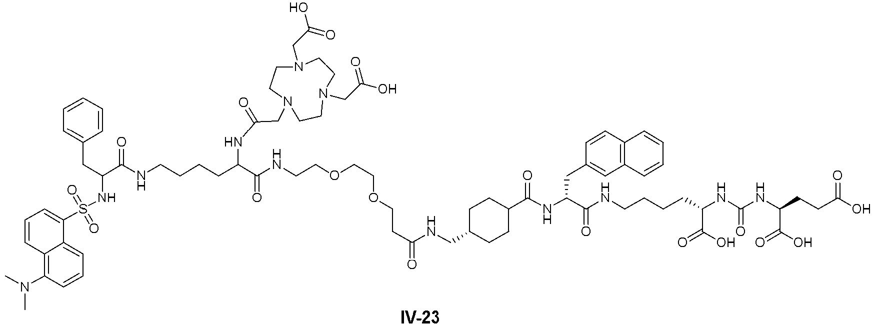

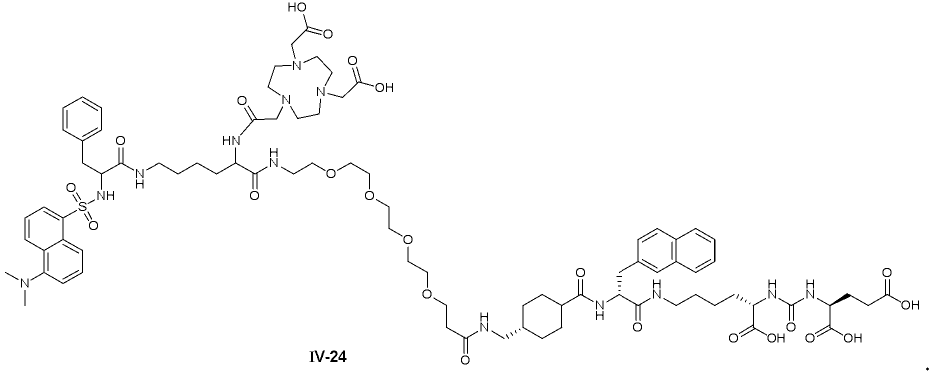

9. A radionuclide-labeled PSMA-targeting compound, which is a complex obtained by labeling a radionuclide using the dansylamide-modified PSMA-targeting compound having the structure represented by formula I according to any one of claims 1-8 as a ligand, wherein the radionuclide is selected from any one of ¹⁷⁷Lu, ¹⁸F, ⁴³Sc, ⁴⁴Sc, ⁴⁷Sc, ⁵⁵Co, ⁵⁷Co, ⁶⁸Ga, ⁶⁷Ga, ⁶²Cu, ⁶⁴Cu, ⁶⁷Cu, ⁸⁹Zr, ⁸⁶Y, ⁹⁰Y, ⁹⁹mTc, ⁹⁹Tc, ⁹⁰ln, ¹¹¹ln, ⁸⁹Sr, ¹⁵²Gd, ¹⁵³Gd, ¹⁵³Sm, ¹⁴⁹Tb, ¹⁵¹Tb, ¹⁶¹Tb, ¹⁶⁶Ho, ¹⁶⁶Dy, ¹⁸⁶Re, ¹⁸⁸Re, ²¹²Pb, ²¹²Bi, ²¹³Bi, ²²³Ra, ²²⁵Ac, ²²⁶Th, ²²⁷Th, or ²¹¹At.

10. A pharmaceutical composition, comprising the dansylamide-modified PSMA-targeting compound according to any one of claims 1-8 or the radionuclide-labeled PSMA-targeting compound according to claim 9, and a pharmaceutically acceptable carrier.

11. The pharmaceutical composition according to claim 10, wherein the pharmaceutically acceptable carrier is selected from a binder, a buffer, a colorant, a diluent, a disintegrant, an emulsifier, a flavoring agent, a glidant, a lubricant, a preservative, a stabilizer, a surfactant, a tableting agent, or a wetting agent, or a combination thereof.

12. Use of the radionuclide-labeled PSMA-targeting compound according to claim 9 in preparation of a medicament for treatment or diagnosis of a PSMA-highly expressing cancer in a mammal, wherein the cancer is prostate cancer, lung cancer, liver cancer, gastric cancer, breast cancer, colorectal cancer, renal cell carcinoma, bladder cancer, a brain tumor, thyroid cancer, or adenoid cystic carcinoma; in the use, a diagnostically or therapeutically effective amount of the radionuclide-labeled PSMA-targeting compound according to claim 9 for the mammal is combined with one or more other active ingredients.

13. Use of the pharmaceutical composition according to claim 10 in preparation of a medicament for treatment or diagnosis of a PSMA-highly expressing cancer in a mammal, wherein the cancer is prostate cancer, lung cancer, liver cancer, gastric cancer, breast cancer, colorectal cancer, renal cell carcinoma, bladder cancer, a brain tumor, thyroid cancer, or adenoid cystic carcinoma; in the use, a diagnostically or therapeutically effective amount of the pharmaceutical composition according to claim 10 for the mammal is combined with one or more other active ingredients.

14. The use according to any one of claims 12-13, wherein the one or more other active ingredients are selected from anticancer therapeutic compounds.

15. The use according to claim 14, wherein the anticancer therapeutic compound is selected from any one or a combination of two or more of doxorubicin, paclitaxel, docetaxel, cisplatin, camptothecin, temozolomide, Avastin, Herceptin, or Erbitux.
